(19) **Europäisches Patentamt**
**European Patent Office**
**Office européen des brevets**

(11) **EP 3 768 296 B1**

(12) **EUROPEAN PATENT SPECIFICATION**

(45) Date of publication and mention
of the grant of the patent:
**06.09.2023 Bulletin 2023/36**

(21) Application number: **19714061.9**

(22) Date of filing: **15.03.2019**

(51) International Patent Classification (IPC):
*A61K 38/10* (2006.01)    *A61K 38/08* (2019.01)
*A61K 9/00* (2006.01)    *G02B 1/04* (2006.01)
*A61P 27/02* (2006.01)    *A61P 27/06* (2006.01)

(52) Cooperative Patent Classification (CPC):
**A61K 38/10; A61K 9/0048; A61K 38/08;**
**A61P 27/02; A61P 27/06;** A61K 9/0019;
G02B 1/043

(86) International application number:
**PCT/US2019/022542**

(87) International publication number:
**WO 2019/182905 (26.09.2019 Gazette 2019/39)**

(54) **SAP AND PEPTIDOMIMETICS FOR TREATMENT OF EYE DISEASE**

SAFT- UND PEPTIDOMIMETIKA ZUR BEHANDLUNG VON AUGENKRANKHEITEN

SAP ET PEPTIDOMIMÉTIQUES POUR LE TRAITEMENT D'UNE MALADIE OCULAIRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **23.03.2018 US 201862647184 P**

(43) Date of publication of application:
**27.01.2021 Bulletin 2021/04**

(73) Proprietor: **Arch Biosurgery, Inc.**
**Framingham MA 01702 (US)**

(72) Inventors:
• **NORCHI, Terrence W.**
**Natick, Massachusetts 01760 (US)**
• **ELLIS-BEHNKE, Rutledge**
**Myrtle Beach, South Carolina 29575 (US)**

(74) Representative: **Potter Clarkson**
**Chapel Quarter**
**Mount Street**
**Nottingham NG1 6HQ (GB)**

(56) References cited:
WO-A1-2006/116524    WO-A1-2015/027203
WO-A2-2008/113030    US-A1- 2010 272 803

• **CHRISTINA KARAVASILI ET AL:**
**"Self-Assembling Peptide Nanofiber Hydrogels**
**for Controlled Ocular Delivery of Timolol**
**Maleate", ACS BIOMATERIALS SCIENCE &**
**ENGINEERING, vol. 3, no. 12, 16 November 2017**
**(2017-11-16), pages 3386-3394, XP055593886, US**
**ISSN: 2373-9878, DOI:**
**10.1021/acsbiomaterials.7b00706**
• **Fabrizio Gelain ET AL: "Chapter 4: Designer**
**Self-Assembling Peptide Scaffolds for3D Tissue**
**Cell Cultures" In: "WOSUID:**
**WOS:000278836000004", 1 January 2010**
**(2010-01-01), Researchcher ID, XP055594081,**
**pages 59-81, the whole document**

Remarks:
The complete document including Reference
Table(s) and the Sequence Listing(s) can be
downloaded from the EPO website

**Description**

**CROSS-REFERENCE TO RELATED APPLICATIONS**

This application claims the benefit of and priority to U.S.S.N. 62/647,184 filed March 23, 2018, and which is incorporated by reference in its entirety.

**REFERENCE TO SEQUENCE LISTING**

The Sequence Listing submitted March 15, 2019 as a text file named "CNS_109_ST25.txt," created on March 14, 2019, and having a size of 114,130 bytes is hereby incorporated by reference pursuant to 37 C.F.R. § 1.52(e)(5).

**FIELD OF THE INVENTION**

This invention is in the field of therapeutic reagents, particularly compositions of SAPs, which are useful for the treatment, prevention or alleviation of the symptoms of diseases, disorders, injuries and related symptoms that affect the eye.

**BACKGROUND OF THE INVENTION**

The outer covering of the eyeball consists of a relatively tough, white layer called the sclera (or white of the eye). Near the front of the eye, in the area protected by the eyelids, the sclera is covered by a thin, transparent membrane (conjunctiva), which runs to the edge of the cornea. The conjunctiva also covers the moist back surface of the eyelids and eyeballs.

Light enters the eye through the cornea, the clear, curved layer in front of the iris and pupil. The cornea serves as a protective covering for the front of the eye and also helps focus light on the retina at the back of the eye. After passing through the cornea, light travels through the pupil (the black dot in the middle of the eye). The iris, the circular, colored area of the eye that surrounds the pupil, controls the amount of light that enters the eye. The iris allows more light into the eye, (enlarging or dilating the pupil, when the environment is dark and allows less light into the eye, (shrinking or constricting the pupil, when the environment is bright. The size of the pupil is controlled by the action of the pupillary sphincter muscle and dilator muscle.

Behind the iris sits the lens. By changing its shape, the lens focuses light onto the retina. Through the action of small muscles called the ciliary muscles, the lens becomes thicker to focus on nearby objects and thinner to focus on distant objects. The retina contains the cells that sense light (photoreceptors) and the blood vessels that nourish them. The most sensitive part of the retina is a small area called the macula, which has millions of tightly packed photoreceptors (the type called cones). The high density of cones in the macula makes the visual image detailed, just as a high-resolution digital camera has more megapixels. Each photoreceptor is linked to a nerve fiber. The nerve fibers from the photoreceptors are bundled together to form the optic nerve. The optic disk, the first part of the optic nerve, is at the back of the eye. The photoreceptors in the retina convert the image into electrical signals, which are carried to the brain by the optic nerve. There are two main types of photoreceptors: cones and rods.
Cones are responsible for sharp, detailed central vision and color vision and are clustered mainly in the macula. Rods are responsible for night and peripheral vision. Rods are more numerous than cones and much more sensitive to light, but they do not register color or contribute to detailed central vision as the cones do. Rods are grouped mainly in the peripheral areas of the retina.

The eyeball is divided into two sections, each of which is filled with fluid. The pressure generated by these fluids fills out the eyeball and helps maintain its shape. The front section (anterior segment) extends from the inside of the cornea to the front surface of the lens and is filled with aqueous humor, which nourishes the internal structures. The anterior segment is divided into two chambers. The anterior chamber extends from the cornea to the iris. The posterior) chamber extends from the iris to the lens. Normally, the aqueous humor is produced in the posterior chamber, flows slowly through the pupil into the anterior chamber, and then drains out of the eyeball through outflow channels located where the iris meets the cornea. The posterior segment extends from the back surface of the lens to the retina. It contains a jelly like fluid called the vitreous humor.

The cornea is the dome-shaped, transparent outer surface of the front of the eye that exists directly beneath the pre-corneal tear film. The tear film is a thin layer of tears that covers the exposed area of the globe and contains an outer oily layer, a middle watery layer, and an inner mucus layer. Light passes to the lens through the cornea, which provides approximately two-thirds of the eye's total optical power. The protective, outermost layer of the cornea contains regenerative epithelium that acts as a water permeable physical barrier.

Diseases, disorders, injuries of the eye, including the ocular surface, cornea, and other structures, can negatively impact eyesight and quality of life and are often inadequately treated due to the need for more effective treatment options. Patients can experience a range of problems and sequelae, including loss of visual acuity, blurry vision, photophobia, pain, discomfort, redness, itching, and blindness due to the cornea, retina, and other structures. Chronic eye diseases and disorders represent a significant healthcare burden. For example, the annual cost of managing DED in the United States has been estimated to be 3.8 billion USD (Yu, et al., Cornea, 30(4): pp. 379-87 (2011); Waduthantril, et al., PLoS ONE, Vol 7(6), e37711 (2012)).

Dry Eye Disease (DED), also known as keratoconjunctivitis sicca (Lemp, et al., Report of the International Dry Eye Workshop (DEWS) Ocul Surf 5: 65-204 (2007)), is a multifactorial disease of the tears and ocular surface that may cause discomfort, visual disturbance, tear film instability and damage to the ocular surface. It is typically accompanied by increased osmolarity of the tear film and inflammation at the ocular surface. Treatment, which typically includes frequent applications per day of artificial tears and non-prescription eye drops to mitigate irritation and lubricate the eyes, usually provides only minimal and limited relief without modifying the disease course.

Recurrent corneal erosion syndrome (RCES) is a common clinical disorder characterized by a disturbance at the level of the corneal epithelial basement membrane resulting in defective adhesions and recurrent breakdown of the epithelium. It may arise spontaneously or from anterior basement membrane dystrophy (e.g., Cogan dystrophy or map dot fingerprint dystrophy) or from other problems, such as adhesions between the palpebral conjunctiva of the eyelids and the corneal epithelium. Management of RCES is usually aimed at regenerating or repairing the epithelial basement membrane to restore the adhesion between the epithelium and the anterior stroma. However, current limited options for effectively controlling the development and progression of RCES typically include application of lubricating ointment to prevent surface aggravation and antimicrobials to prevent and reduce infection of the damaged mucosal tissue. Treatment can be prolonged and arduous, often leading to poor patient compliance poor therapeutic outcomes.

Although often unsuccessful, RCES treatment may include punctal occlusion, in which a plug is inserted into the tear duct, or direct application of a bandage soft contact lens. Patients with refractory RCES may undergo surgical interventions, such as Anterior Stromal Micropuncture (ASM), phototherapeutic keratectomy and debridement of the corneal epithelium, however, the attendant risks include scarring, glare, and blurred vision, and they often fail.

In many instances, eye disease prevalence and severity are affected by underlying medical conditions. For instance, patients with diabetes are twice as likely to suffer symptoms of DED and/or require frequent use of artificial tear products. Elevated blood glucose levels can increase tear osmolarity and impair lacrimal gland tear secretion, resulting in DED. Also, increased glucose in the meibomian glands may disrupt the normal flow of oil and lead to increased evaporation of tears and increased the risk of microbial overgrowth.

Hyperglycemia also adversely affects adherence of corneal epithelial cells to underlying tissue, which can result in chronic sloughing of the corneal surface characteristic of RCES and increased risk of infection. Current treatment regimens involving contact lenses and corneal refractive surgery are intended for patients who maintain good glycemic control (Working Together to Manage Diabetes: A guide for pharmacy, podiatry, Optometry, and dentistry, What Eye Care Professionals Would Like Team Members to Know About Eye Health and Diabetes, pp. 59-67, 2016). In addition, prolonged periods of hyperglycemia can lead to retraction of neuronal processes in the cornea and corneal neuropathy (Yagihashi, et al., Journal of Diabetes Investigation, V.2 (1), pp. 18-32 (2011)), which in turn can delay intervention, diagnosis and treatment due to reduced sensitivity at the ocular surface, further exacerbating the eye diseases and their effects.

Treatment options for diseases, disorders, injuries and related symptoms of the corneal surface, such as DED and RCES, may also include prescription eye drops containing immune-suppressants, such as topical preparations of cyclosporine (CsA; RESTASIS®), or corticosteroids (Lemp, Am J Manag Care:, 14(3 Suppl):S88-101(2008)). However, corticosteroids are not ideal for long-term use due to possible side effects, and this treatment regimen is often contraindicated in patients.

WO 2006/116524, WO 2015/027203, WO 2008/113030 and US 2010/272803 all disclose self-assembling peptides but not for treating ocular or intraocular inflammation.

There remains a need for therapies that can be used to treat the symptoms of eye disease, such as DED and RCES, while providing a barrier to protect the corneal epithelium from abrasion, contaminants, and infective agents that contribute to disease progression and severity.

It is therefore an object of the present invention to provide compositions for treatment and prevention of one or more diseases, disorders, injuries and related symptoms that affect the eye.

It is also an object of the present invention to provide methods and compositions for alleviating the symptoms of chronic DED.

It is also an object of the present invention to provide methods and compositions for alleviating the symptoms of corneal erosions and RCES.

It is also an object of the present invention to provide methods and compositions for reducing and preventing infection and enhancing repair of the diseased or damaged cornea.

It is still a further object of the present invention to provide methods and compositions for preventing and treating diseases, disorders, injuries and related symptoms of the interior compartments of the eye.

## SUMMARY OF THE INVENTION

Compositions of SAP and/or self-assembling peptidomimetics (referred to herein as "SAP", unless designated otherwise), and methods of use thereof to control or treat diseases, disorders, injuries and related symptoms that affect the eye by application directly onto or into the eye, are described. The SAP may be assembled prior to application, or be applied as non-assembled precursor peptides and/or peptidomimetics, which assemble during or following application. Assembly can be initiated upon contact with bodily fluids (*e.g.*, tears at the surface of the eye) or an ionic solution.

The SAP have a sequence of amino acid residues conforming to one or more of the following formulas:

$$((Xaa^{neu}\text{-}Xaa^{+})_{x}(Xaa^{neu}\text{-}Xaa^{-})_{y})_{n}; \quad (I)$$

$$((Xaa^{neu}\text{-}Xaa^{-})_{x}(Xaa^{neu}\text{-}Xaa^{+})_{y})_{n}; \quad (II)$$

$$((Xaa^{+}\text{-}Xaa^{neu})_{x}(Xaa^{-}\text{-}Xaa^{neu})_{y})_{n}; \quad (III)$$

and

$$((Xaa^{-}\text{-}Xaa^{neu})_{x}(Xaa^{+}\text{-}Xaa^{neu})_{y})_{n}, \quad (IV)$$

where each $Xaa^{neu}$ represents an amino acid residue having a neutral charge, $Xaa^{+}$ represents an amino acid residue having a positive charge, $Xaa^{-}$ represents an amino acid residue having a negative charge, x and y are integers having a value of 1, 2, 3, or 4, independently, and n is an integer having a value of 1-5.

At least 70% and up to 100% of the SAP in the composition are of the same size and have the same amino acid sequence, for example, 75% or more, such as 80%, 85%, 90% or 95%, or 99% of the SAP are of the same size and have the same amino acid sequence. The compositions can include two or more different SAP having different sizes and/or sequences. The compositions can also include polymers and can be partly biodegradable, fully biodegradable, or non-biodegradable. Compositions can optionally include a separate scaffold or support material.

The SAP may contain a tissue specific targeting or binding sequence or ligand. This may be mucoadhesive or targeted to specific cell types, such as epithelial or endothelial cells.

Compositions including one or more SAP can control, prevent or treat inflammatory diseases, disorders, injuries and related symptoms that affect the eye, provide an optically clear barrier to infection in and contamination of the cornea and other eye structures, and prevent or mitigate inflammation of the corneal stroma, retina and other eye structures.

Compositions including one or more SAP may contain one or more therapeutic, prophylactic, diagnostic agents, and/or cells. Examples include anti-inflammatories, anesthetics, antimicrobials, angiogenesis inhibitors, immunosuppressants, chemotherapeutics, ocular anti-hypertensive agents, and combinations thereof.

In preferred embodiments, the SAP compositions are formulated for topical administration to the eye or for injection. In some embodiments, the SAP penetrates and transports the bound agent across the corneal surface. The compositions are suitable for administration into one or more of the internal compartments of the eye or onto the surface of the eye by, for example, instillation or injection. Systems for the topical or intratissue application of compositions including SAP into or onto the surface of the eye have been developed. Examples include eye-dropper, sprays, and syringes. The amount and concentration of the SAP applied to the eye is typically sufficient to form an SAP structure at the surface of the eye, which may act as a barrier to the passage of fluid. The SAP structure is optically clear, which can act as a barrier to prevent contamination and/or infection of the eye. Contact lenses, lacrimal inserts, and lens replacements can be coated on one or more surface with SAP and optionally one or more therapeutic, prophylactic or diagnostic agents. In some embodiments, the contact lenses include a backing material or support scaffold, which may be non-peptide.

Methods of making compositions that contain SAP for administration to the eye can include injection molding, stamping, templating onto a surface having a desired shape, coating of a solid substrate, electro-spinning, or combinations of these. The SAP can be assembled by contacting the composition with a solution of cations. Self-assembly of the peptides can occur at the time of manufacture of the composition, or immediately prior to, during or after application of the composition to the eye.

In some embodiments, the SAP are applied as eye drops to the eye of a subject to treat or prevent one or more diseases of the eye such as diabetic retinopathy, retinitis pigmentosa, uveitis, inflammatory eye diseases, autoimmune eye diseases, dry eye syndrome (DED), Reiter's syndrome, psoriasis, rheumatoid arthritis, Sjogren's Syndrome, recurrent corneal erosion syndrome (RCES), optic neuritis, and sarcoidosis. In certain embodiments, the methods prevent the development or progression of DED or RCES. The patient may suffer from a primary, secondary, or acquired metabolic disorder, such as diabetes. In some embodiments, the administration of compositions of SAP enhances the healing of one or more damaged or diseased structures at the surface of the eye. For example, in some embodiments, the methods decrease the amount of time required for the various layers of the cornea to heal by at least about 10%, at least about 30%, or at least about 50%, relative to an untreated control.

In some embodiments, methods for the formation of an SAP structure at the surface of the eye include the successive applications of one or more SAP to the eye, to produce a multi-layered structure at the ocular surface. When multiple layers of SAP structures are provided, each layer can include a specific peptide sequence or mixture of peptide sequences, having the same or different properties, as desired. For example, in some embodiments, two or more different SAP layers are consecutively deposited onto the surface of the eye. In some embodiments, each application forms an SAP structure having different properties relative to the other one or more layers. Exemplary properties include thickness, flexibility, the ability to bind or adhere to tissue, presence or hydrophobic or hydrophilic moieties, and the presence of one or more additional agents.

## BRIEF DESCRIPTION OF THE DRAWINGS

Figure 1A is a schematic depicting a cross-section view of the human eye. Figure 1B is a schematic depicting a cross-section view of the layers of the tear film at the corneal surface, including the external lipid, middle aqueous, and inner mucus layers as well as the epithelial layer and corneal stroma. Figure 1C is a schematic depicting a cross-section view of the cornea and tear ducts.

Figure 2 is a histogram depicting the JEB score (-20 to 200) for each of Normal, Saline Day 3, contralateral eye LPS Day 3, 1 Day LPS, 3 Day LPS, 0.5% RADA + LPS Day 1, 0.5% RADA + LPS Day 3, and 0.5% RADA + LPS Day 7, respectively.

Figure 3 is a histogram depicting the total retinal area weight-averaged density (-200 to 1,200) for each of Normal, Saline Day 3, contralateral eye LPS Day 3, 1 Day LPS, 3 Day LPS, 0.5% RADA + LPS Day 1, 0.5% RADA + LPS Day 3, and 0.5% RADA + LPS Day 7, respectively.

Figure 4 is a graph showing quantification of activated retinal microglial cells as an indicator of inflammation. The data is presented as pixel intensity and is normalized to remove variation across sections. The pixel intensity from each of the layers of the eye across the various tested conditions are presented. LPS alone (Ctrl L), or LPS in combination with $(RADA)_2$ at a concentration of 0.1% (S1-1), 1.0% (S1-10), and 10.0% (S1-100). S+A+R are the retina, sclera, and pigment epithelium.

Figure 5 is a graph showing quantification of inflammation. The y-axis indicates pixel density normalized to area as an indicator of activated retinal microglial cells. S2: $H_2N(EARA)COOH$ (SEQ ID NO: 92); S3: $H_2N(RARA)CONH_2$ (SEQ ID NO: 413); S5: $H_2N(EARA)_2CONH_2$ (SEQ ID NO: 414).

## DETAILED DESCRIPTION OF THE INVENTION

### I. Definitions

The term "about" is intended to describe values either above or below the stated value in a range of approximately +/- 10%; in other embodiments the values may range in value either above or below the stated value in a range of approximately +/- 5%; in other embodiments the values may range in value either above or below the stated value in a range of approximately +/- 2%; in other embodiments the values may range in value either above or below the stated value in a range of approximately +/- 1%. The preceding ranges are intended to be made clear by context, and no further limitation is implied.

"Biocompatible" refers to compatibility with living tissue or a living system by not being toxic, injurious, or physiologically reactive and not causing immunological rejection. Biocompatible materials, along with any metabolites or degradation products thereof, are generally non-toxic and do not cause significant adverse effects to the recipient. Biocompatible materials generally do not elicit a significant or problematic inflammatory or immune response when administered.

"Biodegradable" generally refers to a material that under physiologic conditions degrades or erodes to smaller units or chemical species that are capable of being metabolized, eliminated, or excreted by the subject. The degradation time is a function of composition and morphology and can last, for instance, hours, weeks or months. Degradation can include disassembly of SAP structures. Therefore, in some embodiments, degradation can include disassembly of SAP structures.

"Complementary" means having the capability of forming ionic or hydrogen bonding interactions between hydrophilic residues in adjacent peptides in a structure. Hydrophilic residues in a peptide contain either hydrogen bonds or ionically pairs with a hydrophilic residue on an adjacent peptide, or is exposed to solvent. In most cases the peptides assemble hydrophobic to hydrophobic and hydrophilic to hydrophilic, although hydrophobic to hydrophilic can occur. The structure of the molecule will change during assembly over time. Alignment can and does change during packing. In the case of an SAP such as RADA (SEQ ID NO: 57), the hydrophobic face will assemble while the hydrophilic face will assemble in aqueous solvent; in the case of an oil solvent, the peptide will assemble hydrophilic - hydrophilic, with the hydrophobic face oriented into the oil. Pairing may also involve van der Waals forces.

"Effective amount refers to the amount necessary to elicit a desired response, which may vary depending on such factors as the desired outcome, the agent being delivered, the nature of the target site, the nature of the conditions under which the agent is administered, *etc.* For example, the effective amount of a composition for treatment of a disease or disorder may be an amount sufficient to promote recovery to a greater extent than would occur in the absence of the composition.

"Preventing" refers to reducing the risk that a condition, state, disease, or symptom, or the manifestation or worsening thereof, will occur.

The terms "treat", "treatment" and "treating" refer to the reduction or amelioration of the progression, severity and/or duration of one or more symptoms of an injury, disease or disorder, delay of the onset of a disease or disorder, or the amelioration of one or more consequences, indications or symptoms (preferably, one or more discernible symptoms) of an injury, disease or disorder, resulting from the administration of one or more therapies (*e.g.*, one or more therapeutic agents such as a compound as described).

"Increase," "enhance," "stimulate," "induce" and/or like terms generally refer to the act of directly or indirectly improving or increasing a function or behavior relative to its natural, expected, or average or relative to current conditions.

The term "self-assembling" refers to the spontaneous or induced assembly of molecules into defined, stable, non-covalently bonded structures that are held together by intermolecular and/or intramolecular forces.

The term "topical administration" means non-invasive administration to a tissue, organ, or orifice. Topical administrations can be administered locally and can provide a local effect in the region of application while limiting systemic exposure. Topical formulations can provide systemic effect via adsorption into the blood stream of the individual. Topical administration can include, but is not limited to, ophthalmic, cutaneous, transdermal, intravesicular, or mucosal (rectal, buccal, intranasal, or intravaginal, and rectal) administration.

"Small Molecule" refers to a molecule having a relatively low molecular weight, such as less than about 1000 or 1,500 g/mol. Typically, small molecules are not peptides or nucleic acids.

The term "carrier" or "excipient" refers to an organic or inorganic, natural or synthetic inactive ingredient in a formulation, with which one or more additional ingredients are combined. Typically a carrier or an excipient is an inert substance added to a pharmaceutical composition to further facilitate its administration, does not interfere with its activity or properties, and/or does not cause significant irritation to the recipient.

## II. Compositions

### A. SAPs

Compositions for the treatment and prevention of eye disease include SAP, amino acid residues or peptidomimetics that are capable of self-assembly, or combinations thereof. In some embodiments, SAP compositions include mixtures of self-assembling peptides and self-assembling peptidomimetics. In other embodiments, SAP include combinations of standard amino acids and non-standard amino acids.

### 1. SAP

The term "peptide" includes "polypeptide," "oligopeptide," and "protein," and refers to a chain of at least two $\alpha$-amino acid residues linked together by covalent bonds (peptide bonds). "Peptide" may refer to an individual peptide or to a collection of peptides having the same or different sequences, any of which may contain naturally occurring $\alpha$-amino acid residues, non-naturally occurring $\alpha$-amino acid residues, and combinations thereof. In particular, the D-enantiomer ("D-$\alpha$-amino acid") of residues may be used. When D-$\alpha$-amino acid residues (Xaa) are included within a sequence, they are annotated as "Xaa$^D$". $\alpha$-Amino acid analogs are also known in the art and may be employed. Non-naturally occurring amino acids are not found or have not been found in nature, but they can by synthesized and incorporated into a peptide chain. Suitable non-naturally occurring amino acids include, but are not limited to, D-alloisoleucine(2R,3S)-2-amino-3-methylpentanoic acid, L-cyclopentyl glycine (S)-2-amino-2-cyclopentyl acetic acid.

Peptides can be represented as amino acid residue sequences. Those sequences are written left to right in the direction from the amino ("N-") to the carboxyl ("-C") terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V). A "variant" of a peptide refers to a polypeptide that differs from a reference polypeptide but retains essential properties. A variant and reference polypeptide may differ in amino acid sequence by one or more modifications (*e.g.*, substitutions, additions, and/or deletions of one or more residues) relative to the reference peptide.

Modifications and changes (*e.g.*, "conservative amino acid substitutions") can be made in the structure of the polypeptides without substantially affecting the self-assembly characteristics of the polypeptide. For example, certain amino acids can be substituted for other amino acids in a sequence without appreciable variation in activity. In making such changes, the hydropathic index of amino acids can be considered. It is known that certain amino acids can be substituted for other amino acids having a similar hydropathic index or score and still result in a polypeptide with similar functional activity. It is known in the art that an amino acid can be substituted by another amino acid having a similar hydropathic index and still obtain a functionally equivalent polypeptide.

Substitution of like amino acids can also be made on the basis of charge. In certain embodiments, the substitution of amino acids having an equivalent charge under physiological conditions can be made in the structure of the polypeptides of the disclosure without substantially affecting the self-assembly characteristics of the polypeptide. Charge states negative ("-ve"), positive ("+ve"), and non-charged or neutral ("neu") can be assigned to amino acid residues under physiological conditions as follows: aspartate (-ve); glutamate (-ve); arginine (+ve); lysine (+ve); histidine (neu or +ve); serine (neu); asparagine (neu); glutamine (neu); glycine (neu); proline (neu); threonine (neu); alanine (neu); cysteine (neu); methionine (neu); valine (neu); leucine (neu); isoleucine (neu); tyrosine (neu); phenylalanine (neu); tryptophan (neu).

Useful peptides can vary in length as long as they retain the ability to self-assemble to an extent useful for one or more of the purposes. The number of amino acid residues in the peptide may range from as few as four $\alpha$-amino acid residues to as many as 100 residues. Typically, peptides which self-assemble have from about 6 to about 64 residues, more preferably from about 8 to about 36 residues, most preferably from about 8 to about 16 residues. In preferred embodiments, the peptide has from about 8 to about 12 residues or about 12 to about 16 residues, or about 16 to about 20 residues. In yet another embodiment, the peptide has from about 16 to about 24 residues, or from about 16 to about 28 residues, or from about 16 to about 32 residues.

One or more amino acid residues in an SAP can be altered or derivatized by the addition of one or more chemical entities including, but not limited to, acyl groups, carbohydrate groups, carbohydrate chains, phosphate groups, farnesyl groups, isofarnesyl groups, fatty acid groups, or a linker which allows for conjugation or functionalization of the peptide. For example, either or both ends of a given peptide can be modified. The carboxyl and/or amino groups of the carboxyl- and amino-terminal residues, respectively, can be protected or not protected. The charge at a terminus can also be modified. For example, a group or radical such as an acyl group (RCO-, where R is an organic group (*e.g.*, an acetyl group ($CH_3CO-$)) can be present at the N-terminus of a peptide to neutralize an "extra" positive charge that may otherwise be present (*e.g.*, a charge not resulting from the side chain of the N-terminal amino acid). Similarly, a group such as an amine group (RNH-, where R is an organic group (*e.g.*, an amino group -$NH_2$)) can be used to neutralize an "extra" negative charge that may otherwise be present at the C-terminus (*e.g.*, a charge not resulting from the side chain of the C-terminal amino acid residue). Where an amine is used, the C-terminus bears an amide (-CONHR). The neutralization of charges on a terminus may facilitate self-assembly. One of ordinary skill in the art will be able to select other suitable groups.

Useful peptides can also be branched, in which case they will contain at least two peptide "branches", each of which includes at least three amino acid residues joined by peptide bonds. The two peptide branches may be linked by a bond other than a peptide bond.

The peptides can have an amphiphilic nature (*e.g.*, the peptides can contain approximately equal numbers of hydrophobic and hydrophilic amino acid residues), which can be complementary and structurally compatible. Complementary peptides have the ability to form ionic or hydrogen bonds with residues on adjacent peptides in a structure. For example, one or more hydrophilic residues in a peptide can either hydrogen bond or ionically pair with one or more hydrophilic residues on an adjacent peptide. Hydrophilic residues typically contain a polar functional group or a functional group that is charged at physiological conditions. Exemplary functional groups include, but are not limited to, carboxylic acid groups, amino groups, sulfate groups, hydroxyl groups, halogen groups, nitro groups, phosphate groups, *etc.* Hydrophobic residues are those residues that contain non-polar functional groups. Exemplary functional groups include, but are not limited to, alkyl groups, alkene groups, alkyne groups, and phenyl groups.

In one embodiment, the hydrophilic residue has the formula -NH-CH(X)-COO-, wherein X has the formula $(CH_2)_yZ$, wherein y = 0-8, preferably 1-6, more preferably 1-4 and most preferably 1-3, and Z is a polar or charged functional group including, but not limited to, a carboxylic acid group, an amino group, a sulfate group, a hydroxyl group, a halogen group, a nitro group, a phosphate group, or a functional group containing a quaternary amine. The alkyl chain can be in a linear, branched, or cyclic arrangement. X may also contain one or more heteroatoms within the alkyl chain and/or X may be substituted with one or more additional substituents. In a preferred embodiment, Z is a carboxylic acid group or an amino group. In one embodiment, the hydrophobic residue has the formula -NH-CH(X)-COO-, wherein X has the formula $(CH_2)_yZ$, wherein $y = 0-8$, preferably 1-6, more preferably 1-4, and more preferably 1-3, and Z is a non-polar functional group including, but not limited to, an alkyl group, an alkene group, an alkyne group, or a phenyl group. The alkyl, alkene, or alkyne chain can be in a linear, branched, or cyclic arrangement. X may also contain one or more heteroatoms within the alkyl chain and/or X may be substituted with one or more additional substituents. In a preferred embodiment, X is an alkyl group, such as a methyl group.

The SAP includes peptides having a sequence of amino acid residues conforming to one or more of Formulas I-IV:

$$((Xaa^{neu}\text{-}Xaa^{+})_x(Xaa^{neu}\text{-}Xaa^{-})_y)_n \qquad (I)$$

$$((Xaa^{neu}\text{-}Xaa^{-})_x(Xaa^{neu}\text{-}Xaa^{+})_y)_n \qquad (II)$$

$$((Xaa^{+}\text{-}Xaa^{neu})_x(Xaa^{-}\text{-}Xaa^{neu})_y)_n \qquad (III)$$

$$((Xaa^{-}\text{-}Xaa^{neu})_x(Xaa^{+}\text{-}Xaa^{neu})_y)_n \qquad (IV)$$

wherein each $Xaa^{neu}$ represents an amino acid residue having a neutral charge; $Xaa^{+}$ represents an amino acid residue having a positive charge; $Xaa^{-}$ represents an amino acid residue having a negative charge; x and y are integers having a value of 1, 2, 3, or 4, independently; and n is an integer having a value of 1-5.

Useful peptides can also include one or more amino acid residue having a neutral charge between one or more sets of residues conforming to any one of Formulas I-IV. For example, in some embodiments, peptides include Formulas III and IV, linked with a single amino acid residue having a neutral charge, or linked with two amino acid residues having a neutral charge, or linked with three amino acid residues having a neutral charge.

Peptides with modulus I (*i.e.*, peptides having alternate positively and negatively charged R groups on one side (*e.g.*, the polar face of the β-sheet) are described by each of Formulas I-IV, where x and y are 1. Examples of peptides of modulus I include, but are not limited to, RADA (SEQ ID NO. 57) and RADARADARADARADA (SEQ ID NO. 1). Examples of peptides of modulus II (*i.e.,* peptides having two residues bearing one type of charge (*e.g.,* a positive charge) followed by two residues bearing another type of charge (*e.g.,* a neutral charge)) are described by the same formulas where both x and y are 2. Examples of peptides of modulus III (*i.e.,* peptides having three residues bearing one type of charge (*e.g.,* a positive charge) followed by three residues bearing another type of charge (*e.g.,* a negative charge)) include, but are not limited to, RARARADADADA (SEQ ID NO. 415). Examples of peptides of modulus IV (*i.e.,* peptides having four residues bearing one type of charge (*e.g.,* a positive charge) followed by four residues bearing another type of charge (*e.g.,* a negative charge)) include, but are not limited to, RARARARADADADADA (SEQ ID NO. 416).

In some embodiments, the SAP comprises peptides having a sequence of amino acid residues comprising one or more of Formulas V-XII:

$$Xaa^{neu} ((Xaa^{neu}\text{-}Xaa^{+})_x(Xaa^{neu}\text{-}Xaa^{-})_y)_n; \qquad (V)$$

$$Xaa^{neu} ((Xaa^{neu}\text{-}Xaa^{-})_x(Xaa^{neu}\text{-}Xaa^{+})_y)_n; \qquad (VI)$$

$$((Xaa^{+}\text{-}Xaa^{neu})_x(Xaa^{-}\text{-}Xaa^{neu})_y)_n Xaa^{neu}; \qquad (VII)$$

$$((Xaa^{-}\text{-}Xaa^{neu})_x(Xaa^{+}\text{-}Xaa^{neu})_y)_n Xaa^{neu}; \qquad (VIII)$$

$$((Xaa^{neu}\text{-}Xaa^+)_x(Xaa^{neu}\text{-}Xaa^-)_y)_n\ Xaa^{neu};\qquad(IX)$$

$$((Xaa^{neu}\text{-}Xaa^-)_x(Xaa^{neu}\text{-}Xaa^+)_y)_n\ Xaa^{neu};\qquad(X)$$

$$Xaa^{neu}\ ((Xaa^+\text{-}Xaa^{neu})_x(Xaa^-\text{-}Xaa^{neu})_y)_n;\qquad(XI)$$

$$Xaa^{neu}\ ((Xaa^-\text{-}Xaa^{neu})_x(Xaa^+\text{-}Xaa^{neu})_y)_n;\qquad(XII)$$

Wherein each $Xaa^{neu}$ represents an amino acid residue having a neutral charge; $Xaa^+$ represents an amino acid residue having a positive charge; $Xaa^-$ represents an amino acid residue having a negative charge; x and y and z are integers having a value of 1, 2, 3, or 4, independently; and n is an integer having a value of 1-5.

Where SAP are used, it is thought that their side chains (or R groups) partition into two faces, a polar face with positively and/or negatively charged ionic side chains (*e.g.,* side chains containing -OH, -NH, $-CO_2H$, or - SH groups), and a nonpolar face with side chains that are considered neutral or uncharged at physiological pH (*e.g.,* the side chain of an alanine residue or residues having other hydrophobic groups). The positively charged and negatively charged amino acid residues on the polar face of one peptide can form complementary ionic pairs with oppositely charged residues of another peptide. These peptides may therefore be called ionic, self-complementary peptides. If the ionic residues alternate with one positively and one negatively charged residue on the polar face (- + - + - + +), the peptides may be described as "modulus I;" if the ionic residues alternate with two positively and two negatively charged residues (- - + + - - + +) on the polar face, the peptides are described as "modulus II;" if the ionic residues alternate with three positively and three negatively charged residues (+ + + - - - + + + - - -) on the polar face, the peptides are describe as "modulus III;" if the ionic residues alternate with four positively and four negatively charged residues (+ + + + - - - - + + + + - - - -) on the polar face, they are described as "modulus IV." A peptide having four repeating units of the sequence EAKA (SEQ ID NO: 77) may be designated EAKA16-I (SEQ ID NO: 76), and peptides having other sequences may be described by the same convention.

Other hydrophilic residues that form hydrogen bonds including, but not limited to, asparagine and glutamine, may be incorporated into the peptides. If the alanine residues in the peptides are changed to more hydrophobic residues, such as leucine, isoleucine, phenylalanine or tyrosine, the resulting peptides have a greater tendency to self-assemble and form peptide matrices with enhanced strength. Some peptides that have similar amino acid sequences and lengths as the peptides described form alpha-helices and random-coils, rather than beta-sheets, without forming macroscopic structures. In addition to self-complementarity, other factors likely to be important for the formation of macroscopic structures include the peptide length, the degree of intermolecular interaction, and the ability to form staggered arrays.

Unpaired residues can interact (*e.g.,* form hydrogen bonds, *etc.,*) with the solvent. Peptide-peptide interactions may also involve van der Waals forces and/or forces that do not constitute covalent bonds. The peptides are structurally compatible when they are capable of maintaining a sufficiently constant intrapeptide distance to allow self-assembly and structure formation. The intrapeptide distance can vary. The term "intrapeptide distance" refers to the average of a representative number of distances between adjacent amino acid residues. In one embodiment, the intrapeptide distance is less than about 4 angstroms, preferably less than about 3, more preferably less than about 2 angstroms, and most preferably less than about 1 angstrom. The intrapeptide distance may be larger than this, however. These distances can be calculated based on molecular modeling or based on a simplified procedure described in U.S. Patent Number No. 5,670,483 to Zhang, et al.

The compositions including SAP can be formed through self-assembly of the peptides described in U.S. Patent Nos. 5,670,483; 5,955,343; 6,548,630; 6,800,481; 7,098,028; 9,327,010; and 9,364,513 to Zhang, et al.*;* U.S. Patent Nos. 9,162,005; 9,415,084; and 9,339,476 to Ellis-Behnke, et al.; Holmes, et al., Proc. Natl. Acad. Sci. USA, 97:6728-6733 (2000); Zhang, et al., Proc. Natl. Acad. Sci. USA, 90:3334-3338 (1993); Zhang, et al., Biomaterials, 16:1385-1393 (1995); Caplan et al., Biomaterials, 23:219-227 (2002); Leon, et al., J. Biomater. Sci. Polym. Ed., 9:297-312 (1998); and Caplan, et al., Biomacromolecules, 1:627-631 (2000). See also WO 2007/142757.

In some embodiments, an SAP can contain a segment of residues that have either a positive or negative charge under physiological conditions. For example, representative amino acid sequences for positively charged SAP include, but are not limited to, KKKK (SEQ ID NO: 419), RRRR (SEQ ID NO: 420), or HHHH (SEQ ID NO: 421). Representative amino acid sequences for negatively charged SAP include, but are not limited to, DDDD (SEQ ID NO: 422) or EEEE (SEQ ID NO: 423). When combined, a string of positively charged amino acid residues align parallel and opposite with a string of negatively charged amino acid residues. In certain embodiments, strings of positively and negatively charged amino acids will alternate and form a multilayered structure.

In some embodiments, an SAP can contain sequences in which at least one hydrophobic residue alternates with at least one hydrophilic residue (under physiological conditions). For example, the sequence of a representative SAP can be GQGQ (SEQ ID NO: 424), GGQQGG (SEQ ID NO: 425), GQQGQQG (SEQ ID NO: 426), GGQGGQGG (SEQ ID NO: 427), *etc.*

The partitioning of the SAP into a non-polar or polar environment can be controlled by altering hydrophobic to hydrophilic amino acid residue ratio, wherein a ratio greater than 1: 1 indicates that the peptide partitions more in hydrophobic conditions, while a ratio of less than 1: 1 indicates that the peptide partitions more in hydrophilic conditions

The compositions, regardless of the precise form (*e.g.*, whether in a liquid form or molded) and regardless of the overall compositions (*e.g.*, whether combined with another agent, contained within a device, or packaged in a kit), can include a mixture of one or more peptides. Peptide-based structures can be formed of heterogeneous mixtures of peptides (i.e., mixtures containing more than one type of peptide conforming to a given formula or to two or more of the formulas). In some embodiments, each of the types of peptides in the mixture can self-assemble with the same type of peptide. In other embodiments, one or more of each type of peptide would not self-assemble alone, but the combination of heterogeneous peptides may self-assemble (*i.e.*, peptides in the mixture are complementary and structurally compatible with each other). Thus, either a homogeneous mixture of self-complementary and self-compatible peptides of the same sequence or containing the same repeating subunit, or a heterogeneous mixture of different peptides, which are complementary and structurally compatible to each other, can be used.

In some embodiments, mixtures of one or more peptide sequences produce structures having combined properties of the different sequences used. The physical properties of self-assembled peptide structures vary according to the ratio of the different SAP from which they are formed.

In some embodiments, SAP structures include two or more layers of structurally distinct SAP structures, for example, formed by consecutive administration and assembly of each peptide onto the surface of the other. Therefore, in some embodiments, the structural and biochemical properties of each surface of a multi-layered SAP structure are different, according to the different properties of the SAP from which they are formed, respectively.

One or more short amino acid sequences that assist in self-assembly (referred to as assembly assist sequences) can be added to a homogeneous or heterogeneous mixture of amino acid sequences that alone do not self-assemble. The assembly assist sequences contain amino acids that are complementary with the amino acids in the sequences in the mixture. The assembly assist sequences may contain any number of amino acids. Preferably, the assembly assist sequences contain at least four amino acids. The assembly assist sequences may contain a flexible linker between the amino acids that assists in self-assembly. For example, the assembly assist sequence may contain a pair, a triad, or a quartet of assembly assisting amino acids at the termini connected via a flexible linker. Suitable assembly assist sequences include, but are not limited to, RADA (SEQ ID NO: 57) and EAKA (SEQ ID NO: 77).

Suitable linkers include, but are not limited to, ether based tethers such as polyethylene glycol (PEG), *N*-succinimidyl 3-(2-pyridyldithio)propionate (SPDP, 3- and 7-atom spacer), long-chain- SPDP (12-atom spacer), (succinimidyloxycarbonyl-α-methyl-2-(2-pyridyldithio) toluene) (SMPT, 8-atom spacer), succinimidyl-4-(*N*-maleimidomethyl)cyclohexane-1-carboxylate) (SMCC, 11-atom spacer) and sulfosuccinimidyl-4-(*N*-maleimidomethyl)cyclohexane-1-carboxylate, (sulfo-SMCC, 11-atom spacer), *m*-maleimidobenzoyl-*N*-hydroxysuccinimide ester (MBS, 9-atom spacer), *N*-(γ-maleimidobutyryloxy) succinimide ester (GMBS, 8-atom spacer), *N*-(γ-maleimidobutyryloxy) sulfosuccinimide ester (sulfo-GMBS, 8-atom spacer), succinimidyl 6-((iodoacetyl) amino) hexanoate (SIAX, 9-atom spacer), succinimidyl 6-(6-(((4-iodoacetyl)amino)hexanoyl)amino)hexanoate (SIAXX, 16-atom spacer), and p-nitrophenyl iodoacetate (NPIA, 2-atom spacer). One of ordinary skill in the art also will recognize that a number of other linkers with varying numbers of atoms may be used.

SAP structures can be formed that have varying degrees of stiffness or elasticity. The structures typically have a low elastic modulus (*e.g.,* a modulus in the range of between about 0.01 and about 1,000 kPa, preferably between about 1 and about 100 kPa, more preferably between about 1 and about 10 kPa as measured by standard methods, such as in a standard cone-plate rheometer). Low values may be preferable, as they permit structure deformation as a result of movement, in response to pressure, for instance in the event of cell contraction. Stiffness can be controlled in a variety of ways, including by changing the length, sequence, and/or concentration of the precursor molecules (*e.g.,* SAP). Other methods for increasing stiffness can also be employed. For example, one can attach to the precursors either biotin or other molecules that can be subsequently cross-linked or otherwise bonded to one another. The molecules (*e.g.,* biotin) can be included at an *N* or C-terminus of a peptide/peptidomimetic or attached to one or more residues between the termini. Where biotin is used, cross-linking can be achieved by subsequent addition of avidin. Other cross-linkable molecules can be used, for example, amino acid residues with polymerizable groups such as vinyl groups may be incorporated and cross-linked by exposure to UV light. The extent of crosslinking can be precisely controlled by applying the radiation for a predetermined length of time. The extent of crosslinking can be determined by light scattering, gel filtration, scanning electron microscopy, or other methods well known in the art. Crosslinking can be assessed by HPLC or mass spectrometry analysis of the structure after digestion with a protease, such as a matrix metalloprotease. Material strength may be determined before and/or after cross-linking. Regardless of whether cross-linking is achieved by a chemical agent or light energy, the molecules may be cross-linked in the course of creating a mold or when peptide-containing solutions are applied to the eye.

SAP chains can be cross-linked (e.g., to form a spider web-type pattern) to reinforce the material *in vivo*. The crosslinks can serve to reinforece the material to provide increased rigidity and strength. For example, an SAP functionalized with a polymerizable group at the periphery can be applied to the surface of the eye. Upon crosslinking, the peripheral material becomes more rigid, anchoring the material to the surface of the eye or other ocular tissue while the interior material remains flexible to move with the body tissue.

Factors influencing the physical properties of self-assembled peptide structures at the surface of the eye include, but are not limited to, peptide sequence, peptide length, presence of bound agents, presence of tissue-specific or tissue-binding motifs, for example, a tissue-specific peptide sequence, as well as peptide amount (*e.g.*, concentration, mass and volume), peptide form (*e.g.,* powder or solution) and assembly-state at application time.

The half-life (*e.g.*, the *in vivo* half-life) of the structures formed by SAP can also be modulated by incorporating protease or peptidase cleavage sites into the precursors that subsequently form a given structure. Proteases or peptidases that occur naturally *in vivo* or that are administered can promote degradation by cleaving their cognate substrates.

Combinations of any of the modifications here can be made. For example, SAP that include a protease cleavage site and a cysteine residue and/or a cross-linking agent, kits and devices containing them, and methods of using them, can be utilized.

The peptide structures formed from any SAP made by any process can be characterized using various biophysical and optical techniques, such as circular dichroism (CD), dynamic light scattering, Fourier transform infrared (FTIR), atomic foRCES (tension) microscopy (ATM), scanning electron microscopy (SEM), and transmission electron microscopy (TEM). For example, biophysical methods can be used to determine the degree of beta-sheet secondary structure in the peptide structure. Filament and pore size, fiber diameter, length, elasticity, and volume fraction can be determined using quantitative image analysis of scanning and/or transmission electron micrographs. The structures can also be examined using several standard mechanical testing techniques to measure the extent of swelling, the effect of pH and ion concentration on structure formation, the level of hydration under various conditions, the tensile strength, as well as the manner in which various characteristics change over the period of time required for the structures to form and degrade. Typically, the SAP are biocompatible, non-toxic, fully or partially biodegradable, and do not cause local or systemic inflammation. Preferably, break down products of the SAP do not cause secondary toxicity and are preferably suitable for growth and repair of the surrounding tissues.

## 2. Peptidomimetics

Another class of materials that can self-assemble is peptidomimetics. The term "peptidomimetics" refers to non-natural peptide molecules which mimic peptide structure. Peptidomimetics typically retain the ability to produce the same biological effect as a parent peptide and can interact with the biological target of the parent peptide. Peptidomimetics may be used to circumvent some of the problems associated with a natural peptide: e.g. stability against proteolysis and poor bioavailability (Vagner J., et al. Curr. Opin. Chem. Biol., 12(3): 292-296 (2008)). Self-assembling eptidomimetics are molecules that are structurally similar to peptides, having a segment of residues having a positive charge under physiological conditions joined to a segment of residues having a negative charge under physiological conditions.

Peptidomimetics have general features analogous to peptides, such as amphiphilicity. Examples of peptidomimetics are described in Moore et al., Chem. Rev. 101(12), 3893-4012 (2001), and in WO 2007/142757

The peptidomimetics can be classified into four categories: α-peptides, β-peptides, γ-peptides, and δ-peptides. Peptides including combinations of more than one of α-amino acids, β-amino acids, γ-amino acids, and δ-amino acids can also be used. For example, SAP includes alpha-amino and beta-amino acid residues (*i.e.,* alpha-beta peptides), alpha-amino and delta-amino acid residues (*i.e.,* alpha-delta peptides), and alpha-amino and gamma- amino acid residues *(i.*e., alpha-gamma peptides).

The alpha amino acids can be classical or non-classical alpha amino acids (*i.e.,* L-form or D-form, or combinations thereof). Examples of α-peptide peptidomimetics that can be used include *N,N'*-linked oligoureas, oligopyrrolinones, oxazolidin-2-ones, azatides and azapeptides.

Examples of β-peptides include β-peptide foldamers, β-aminoxy acids, sulfur-containing β-peptide analogues, and hydrazino peptides.

Examples of γ-peptides include γ-peptide foldamers, oligoureas, oligocarbamates, and phosphodiesters.

Examples of δ-peptides include alkene-based δ-amino acids and carbopeptoids, such as pyranose-based carbopeptoids and furanose-based carbopeptoids.

SAP can be generated, for example, which differ from those exemplified by a single amino acid residue or by multiple amino acid residues (*e.g.,* by inclusion or exclusion of a repeating quartet). For example, one or more cysteine residues may be incorporated into the peptides, and these residues may bond with one another through the formation of disulfide bonds. Structures bonded in this manner may have increased mechanical strength relative to structures made with comparable peptides that do not include cysteine residues and thus are unable to form disulfide bonds.

**3. Exemplary SAP**

In an exemplary embodiment, a self-assembling peptidomimetic includes both alpha amino acids (annotated as Xaa) and beta amino acids (annotated as Xaa$^B$). Exemplary self-assembling peptidomimetic sequences include EA$^B$KA$^B$EA$^B$KA$^B$EA$^B$KA$^B$EA$^B$KA$^B$ (SEQ ID NO: 428); EA$^B$KA$^B$EA$^B$KA$^B$ (SEQ ID NO: 429); RA$^B$DA$^B$RA$^B$DA$^B$RA$^B$DA$^B$-RA$^B$DA$^B$ (SEQ ID NO: 430); and RA$^B$DA$^B$RA$^B$DA$^B$ (SEQ ID NO: 431).

Examples of representative hydrophobic and hydrophilic SAP sequences are listed in Table 1.

**Table 1.** Representative SAP

| Sequence (N → C) | SEQ ID NO: |
| --- | --- |
| RADARADARADARADA | 1 |
| SGSGSGSGSGSGSGSG | 2 |
| SASASASASASASASA | 3 |
| SVSVSVSVSVSVSVSV | 4 |
| SLSLSLSLSLSLSLSL | 5 |
| SISISISISISISISI | 6 |
| SMSMSMSMSMSMSMSM | 7 |
| SFSFSFSFSFSFSFSF | 8 |
| SWSWSWSWSWSWSWSW | 9 |
| SPSPSPSPSPSPSPSP | 10 |
| TGTGTGTGTGTGTGTG | 11 |
| TATATATATATATATA | 12 |
| TVTVTVTVTVTVTVTV | 13 |
| TLTLTLTLTLTLTLTL | 14 |
| TITITITITITITITI | 15 |
| TMTMTMTMTMTMTMTM | 16 |
| TFTFTFTFTFTFTFTF | 17 |
| TWTWTWTWTWTWTWTW | 18 |
| TPTPTPTPTPTPTPTP | 19 |
| CGCGCGCGCGCGCGCG | 20 |
| CACACACACACACACA | 21 |
| CVCVCVCVCVCVCVCV | 22 |
| CLCLCLCLCLCLCLCL | 23 |
| CICICICICICICICI | 24 |
| CMCMCMCMCMCMCMCM | 25 |
| CFCFCFCFCFCFCFCF | 26 |
| CWCWCWCWCWCWCWC | 27 |
| CPCPCPCPCPCPCPCP | 28 |
| YGYGYGYGYGYGYGYG | 29 |
| YAYAYAYAYAYAYAYA | 30 |
| YVYVYVYVYVYVYVYV | 31 |
| YLYLYLYLYLYLYLYL | 32 |
| YIYIYIYIYIYIYIYI | 33 |
| YMYMYMYMYMYMYMYM | 34 |
| YFYFYFYFYFYFYFYF | 35 |
| YWYWYWYWYWYWYWYW | 36 |
| YPYPYPYPYPYPYPYP | 37 |
| NGNGNGNGNGNGNGNG | 38 |
| NANANANANANANANA | 39 |
| NVNVNVNVNVNVNVNV | 40 |
| NLNLNLNLNLNLNLNL | 41 |

(continued)

| Sequence (N → C) | SEQ ID NO: |
|---|---|
| NININININININININI | 42 |
| NMNMNMNMNMNMNMNM | 43 |
| NFNFNFNFNFNFNFNF | 44 |
| NWNWNWNWNWNWNWNW | 45 |
| NPNPNPNPNPNPNPNP | 46 |
| QGQGQGQGQGQGQGQG | 47 |
| QAQAQAQAQAQAQAQA | 48 |
| QVQVQVQVQVQVQVQV | 49 |
| QLQLQLQLQLQLQLQL | 50 |
| QIQIQIQIQIQIQIQI | 51 |
| QMQMQMQMQMQMQMQM | 52 |
| QFQFQFQFQFQFQFQF | 53 |
| QWQWQWQWQWQWQWQW | 54 |
| QPQPQPQPQPQPQPQP | 55 |
| AEAKAEAKAEAKAEAK | 56 |
| RADA | 57 |
| RAEARAEARAEARAEA | 58 |
| KADAKADAKADAKADA | 59 |
| ARADARADARADA | 60 |
| RADARADARADARADARADA | 61 |
| ARADARADARADARADARADA | 62 |
| ARADARADARADARADA | 63 |
| RLDLRLDLRLDLRLDL | 64 |
| RLDL | 65 |
| RLDLRL | 66 |
| RADARA | 67 |
| LRLDLR | 68 |
| IEIKIEIKIEIKI | 69 |
| IEIKIEIKIEIKIEIK | 70 |
| IEIKIEIKIEIKIEIKI | 71 |
| IEIKIEIKIEIKIEIKIEIK | 72 |
| IEIKIEIKIEIKIEIKIEIKI | 73 |
| IEIKIEIKIEIK | 74 |
| EIKIEIKIEIKIEIKI | 75 |
| EAKAEAKAEAKAEAKA | 76 |
| EAKA | 77 |
| EAKAEAKAEA | 78 |
| EAKAEAKAEAKAEAKAEAKA | 79 |
| AEAKAEAKAEAKAEAKA | 80 |
| AEAKAEAKAEAKA | 81 |
| RADARADARADARADADLRA-c | 82 |
| R$^D$AD$^D$DA$^D$R$^D$AD$^D$DA$^D$R$^D$AD$^D$DA$^D$R$^D$AD$^D$DA$^D$ | 83 |
| R$^D$AD$^D$DA$^D$R$^D$AD$^D$DA$^D$R$^D$AD$^D$DA$^D$ | 84 |
| E$^D$AD$^D$KD$^D$AD$^D$E$^D$AD$^D$KD$^D$AD$^D$E$^D$AD$^D$KD$^D$AD$^D$ | 85 |
| E$^D$AD$^D$KD$^D$AD$^D$ | 86 |
| R$^D$AD$^D$DD$^D$AD$^D$ | 87 |
| RADARADA | 88 |
| EARAEARAEARAEARA | 89 |
| EARAEARAEARA | 90 |
| EARAEARAE | 91 |

(continued)

| Sequence (N → C) | SEQ ID NO: |
|---|---|
| EARA | 92 |

## B. Tissue-Specific Components

The SAP may contain a tissue-specific component ("TSC"), which can be peptides, polysaccharides, or glycoproteins that are present within the eye, or that are specific to the tissue surrounding or in contact with the eye. Specificity can vary in degree. For example, a TSC may bind to a single cell type or to cells found in one type of tissue, in connective tissue, to epithelial cells, or by species (human) cells, or specific organs or organelles.

TSC bind to tissues within or adjacent to the ocular compartment including tissues at or near the surface of the eye, tissues within the interior cavities of the eye, and tissue surrounding the eye. Tissues at or near the surface of the eye include the tissues of the corneal epithelium, the Bowman's membrane layer, the corneal stroma, the Descemet's membrane of the cornea, the corneal endothelium, the fornix conjunctiva, the scleral conjunctiva, the bulbar conjunctiva, the marginal conjunctiva, the tarsal conjunctiva, the orbital conjunctiva and the limbal conjunctiva. Exemplary tissues within the interior of the eye include tissues of the anterior cavity, the scleral venous sinus, the lens, the suspensory ligament of the lens, the vitreous chamber, the retina, the ciliary cavity, the retina, the retinal arteries and veins, the optic disc and the choroid. Exemplary tissues surrounding the eye include the palpebral conjunctiva, the tear ducts, tarsal glands, the lacus lacrimalis, the lacrimal punctum, and the epithelial tissues lining the interior of the ocular cavity.

The TSC can target cell specific surface carbohydrates. Cell type-specific carbohydrates are involved in cell-cell interactions. In some embodiments, the TSC is a sequence of amino acids that recognizes and interacts with one or more components of injured or diseased tissue. In some embodiments, TSC interact with a ligand or component that is common to many tissues, and that is also expressed in the eye. In other embodiments, the TSC interacts with a sequence that is not present or exposed in healthy tissue. In certain embodiments, the TSC interacts with one or more of the components of the extracellular matrix (ECM). The SAP can be modified such that they can anchor or interact with the structural ECM at the edges of blood vessels and/or tissues. ECM is any material part of a tissue that is not part of any cell, and it is the defining feature of connective tissue. The ECM's main components are various glycoproteins, proteoglycans and hyaluronic acid. In most animals, the most abundant glycoproteins in the ECM are collagens. ECM also contains many other components: proteins such as fibrin, elastin, fibronectins, laminins, and nidogens, and minerals such as hydroxyapatite, or fluids such as blood plasma or serum with secreted free flowing antigens. In addition, the ECM sequesters a wide range of cellular growth factors, and acts as a local depot for them. Changes in physiological conditions can trigger protease activities that cause local release of such depots. This allows the rapid and local activation of cellular functions, without de novo synthesis. Given this diversity, ECM can serve many functions, such as providing support and anchorage for cells, providing a way of separating the tissues, and regulating intercellular communication.

### 1. TSC sequences

Peptides or proteins can be used in combination with or alternating with the SAP. Representative TSC are provided in Table 2.

**Table 2. Tissue Specific Components**

| Sequence (N → C) | SEQ ID NO: |
|---|---|
| Pmp(Y(Me)ITNCP-Orn-Y)NH$_2$ | 409 |
| Mpr(YFQNCPR) | 410 |
| (CYFQNCPRG)NH$_2$ | 411 |
| CYFQNCPR | 412 |
| (CYIQNCPRG)NH$_2$ | 93 |
| (YFQN(Asu)PRG)NH$_2$ | 94 |
| (YIQN(Asu)PRG)NH$_2$ | 95 |
| Mpr-D-PyridylAnine(FQNCPRG)NH$_2$ | 96 |
| (Deamino-Pen-YFVNCPDRG)NH$_2$ | 97 |
| Mpr(YFQNCPRG)NH$_2$ | 98 |
| Mpr(YFQNCPDRG)NH$_2$ | 99 |
| Mpr(YFQNCPK) | 100 |

(continued)

| Sequence (N → C) | SEQ ID NO: |
|---|---|
| (CYFQNCPKG)NH$_2$ | 101 |
| CYFQNCPK | 102 |
| Mpr(YFVNCPDRG)NH$_2$ | 103 |
| (CFIQNCP-Orn-G)NH$_2$ | 104 |
| Pmp(DY(OEt)FVNCP-Cit-G)NH$_2$ | 105 |
| Pmp(Y(OEt)FVNCPRG)NH$_2$ | 106 |
| Pmp(Y(Me)FQNCPRG)NH$_2$ | 107 |
| Pmp(Y(Me)IQNCP-Orn-G)NH$_2$ | 108 |
| GDRGDSP | 109 |
| GDRGDSPASSK | 110 |
| G-Pen-GRGDSPCA | 111 |
| GRADSP | 112 |
| GRGDDSP | 113 |
| GRGDNP | 114 |
| GRGDS | 115 |
| GRGDSP | 116 |
| GRGDSPC | 117 |
| GRGDSPK | 118 |
| GRGDTP | 119 |
| GRGES | 120 |
| GRGESP | 121 |
| GRGETP | 122 |
| KGDS | 123 |
| GAVSTA | 124 |
| WTVPTA | 125 |
| TDVNGDGRHDL | 126 |
| REDV | 127 |
| RGDC | 128 |
| RODS | 129 |
| RGDSPASSKP | 130 |
| RGDT | 131 |
| RGDV | 132 |
| RGES | 133 |
| SDGR | 134 |
| SDGRG | 135 |
| YRGDS | 136 |
| EGVNDNEEGFFSAR | 137 |
| YADSGEGDFLAEGGGVR | 138 |
| G(Glp)VNDNEEGFFSARY | 139 |
| GPR | N/A |
| MSCRAMM | 141 |

Pmp = pyridoxamine phosphate
Mpr = 3-mercaptopropionyl
Deamino-Pen = deamino penicillamine
Pen = penicillamine
Asu = amino succinyl
OEt = ethoxy
Me = methyl
Cit = citrulline

## C. Hydrophobic Peptide Sequences

Hydrophobic or hydrophilic tails can be added to the SAP. The tails can interact with cell membranes, thus anchoring the SAP on to the cell surface. Table 3 shows a list of peptides with hydrophobic tails.

**Table 3. SAP including Hydrophobic Tails**

| Sequence (N → C) | SEQ ID NO: |
| --- | --- |
| GGGGGDGDGDGDGDGD | 142 |
| GGGGGEGEGEGEGEGE | 143 |
| GGGGGKGKGKGKGKGK | 144 |
| GGGGGRGRGRGRGRGR | 145 |
| GGGGGHGHGHGHGHGH | 146 |
| AAAAADADADADADAD | 147 |
| AAAAAEAEAEAEAEAE | 148 |
| AAAAAKAKAKAKAKAK | 149 |
| AAAAARARARARARAR | 150 |
| AAAAAHAHAHAHAHAH | 151 |
| VVVVVDVDVDVDVDVD | 152 |
| VVVVVEVEVEVEVEVE | 153 |
| VVVVVKVKVKVKVKVK | 154 |
| VVVVVRVRVRVRVRVR | 155 |
| VVVVVHVHVHVHVHVH | 156 |
| LLLLLDLDLDLDLDLD | 157 |
| LLLLLELELELELELE | 158 |
| LLLLLKLKLKLKLKLK | 159 |
| LLLLLRLRLRLRLRLR | 160 |
| LLLLLHLHLHLHLHLH | 161 |
| IIIIIDIDIDIDIDID | 162 |
| IIIIIEIEIEIEIEIE | 163 |
| IIIIIKIKIKIKIKIK | 164 |
| IIIIIRIRIRIRIRIR | 165 |
| IIIIIHIHIHIHIHIH | 166 |
| MMMMMDMDMDMDMDMD | 167 |
| MMMMMEMEMEMEMEME | 168 |
| MMMMMKMKMKMKMKMK | 169 |
| MMMMMRMRMRMRMRMR | 170 |
| MMMMMHMHMHMHMHMH | 171 |
| FFFFFDFDFDFDFDFD | 172 |
| FFFFFEFEFEFEFEFE | 173 |
| FFFFFKFKFKFKFKFK | 174 |
| FFFFFRFRFRFRFRFR | 175 |
| FFFFFHFHFHFHFHFH | 176 |
| WWWWWDWDWDWDWDWD | 177 |
| WWWWWEWEWEWEWEWE | 178 |
| WWWWWKWKWKWKWKWK | 179 |
| WWWWWRWRWRWRWRWR | 180 |
| WWWWWHWHWHWHWHWH | 181 |
| PPPPPDPDPDPDPDPD | 182 |
| PPPPPEPEPEPEPEPE | 183 |
| PPPPPKPKPKPKPKPK | 184 |
| PPPPPRPRPRPRPRPR | 185 |
| PPPPPHPHPHPHPHPH | 186 |
| AAAAARADARADARAD | 187 |

(continued)

| Sequence (N → C) | SEQ ID NO: |
| --- | --- |
| AAAAARARADADARAR | 188 |
| AAAAAEAKAEAKAEAK | 189 |
| AAAAAEAEAKAKAEAE | 190 |
| AAAAARAEARAEARAE | 191 |
| AAAAARARAEAEARAE | 192 |
| AAAAAKADAKADAKAD | 193 |
| AAAAAEAHAEAHAEAH | 194 |
| AAAAAEAEAHAHAEAE | 195 |
| AAAAARARARARADAD | 196 |
| AAAAARARARADADAD | 197 |
| AAAAAHADAHADAHAD | 198 |
| AAAAAHADADAHADAD | 199 |
| AAAAAHAEAEAHAEAE | 200 |
| GGGGGRGDGRGDGRGD | 201 |
| GGGGGRGRGDGDGRGR | 202 |
| GGGGGEGKGEGKGEGK | 203 |
| GGGGGEGEGKGKGEGE | 204 |
| GGGGGRGEGRGEGRGE | 205 |
| GGGGGRGRGEGEGRGE | 206 |
| GGGGGKGDGKGDGKGD | 207 |
| GGGGGEGHGEGHGEGH | 208 |
| GGGGGEGEGHGHGEGE | 209 |
| GGGGGRGRGRGRGDGD | 210 |
| GGGGGRGRGRGDGDGD | 211 |
| GGGGGHGDGHGDGHGD | 212 |
| GGGGGHGDGDGHGDGD | 213 |
| GGGGGHGEGEGHGEGE | 214 |
| VVVVVRVDVRVDVRVD | 215 |
| VVVVVRVRVDVDVRVR | 216 |
| VVVVVEVKVEVKVEVK | 217 |
| VVVVVEVEVKVKVEVE | 218 |
| VVVVVRVEVRVEVRVE | 219 |
| VVVVVRVRVEVEVRVE | 220 |
| VVVVVKVDVKVDVKVD | 221 |
| VVVVVEVHVEVHVEVH | 222 |
| VVVVVEVEVHVHVEVE | 223 |
| VVVVVRVRVRVRVDVD | 224 |
| VVVVVRVRVRVDVDVD | 225 |
| VVVVVHVDVHVDVHVD | 226 |
| VVVVVHVDVDVHVDVD | 227 |
| VVVVVHVEVEVHVEVE | 228 |
| LLLLLRLDLRLDLRLD | 229 |
| LLLLLRLRLDLDLRLR | 230 |
| LLLLLELKLELKLELK | 231 |
| LLLLLELELKLKLELE | 232 |
| LLLLLRLELRLELRLE | 233 |
| LLLLLRLRLRLELRLE | 234 |
| LLLLLKLDLKLDLKLD | 235 |
| LLLLLELHLELHLELH | 236 |
| LLLLLELELHLHLELE | 237 |

(continued)

| Sequence (N → C) | SEQ ID NO: |
|---|---|
| LLLLLRLRLRLRLDLD | 238 |
| LLLLLRLRLRLDLDLD | 239 |
| LLLLLHLDLHLDLHLD | 240 |
| LLLLLHLDLDLHLDLD | 241 |
| LLLLLHLELELHLELE | 242 |
| IIIIIRIDIRIDIRID | 243 |
| IIIIIRIRIDIDIRIR | 244 |
| IIIIIEIKIEIKIEIK | 245 |
| IIIIIEIEIKIKIEIE | 246 |
| IIIIIRIEIRIEIRIE | 247 |
| IIIIIRIRIEIEIRIE | 248 |
| IIIIIKIDIKIDIKID | 249 |
| IIIIIEIHIEIHIEIH | 250 |
| IIIIIEIEIHIHIEIE | 251 |
| IIIIIRIRIRIRIDID | 252 |
| IIIIIRIRIRIDIDID | 253 |
| IIIIIHIDIHIDIHID | 254 |
| IIIIIHIDIDIHIDID | 255 |
| IIIIIHIEIEIHIEIE | 256 |
| MMMMMRMDMRMDMRMD | 257 |
| MMMMMRMRMDMDMRMR | 258 |
| MMMMMEMKMEMKMEMK | 259 |
| MMMMMEMEMKMKMEME | 260 |
| MMMMMRMEMRMEMRME | 261 |
| MMMMMRMRMEMEMRME | 262 |
| MMMMMKMDMKMDMKMD | 263 |
| MMMMMEMHMEMHMEMH | 264 |
| MMMMMEMEMHMHMEME | 265 |
| MMMMMRMRMRMRMDMD | 266 |
| MMMMMRMRMRMDMDMD | 267 |
| MMMMMHMDMHMDMHMD | 268 |
| MMMMMHMDMDMHMDMD | 269 |
| MMMMMHMEMEMHMEME | 270 |
| FFFFFRFDFRFDFRFD | 271 |
| FFFFFRFRFDFDFRFR | 272 |
| FFFFFEFKFEFKFEFK | 273 |
| FFFFFEFLFKFKFEFE | 274 |
| FFFFFRFEFRFEFRFE | 275 |
| FFFFFRFRFEFEFRFE | 276 |
| FFFFFKFDFKFDFKFD | 277 |
| FFFFFEFHFEFHFEFH | 278 |
| FFFFFEFEFHFHFEFE | 279 |
| FFFFFRFRFRFRFDFD | 280 |
| FFFFFRFRFRFDFDFD | 281 |
| FFFFFHFDFHFDFHFD | 282 |
| FFFFFHFDFDFHFDFD | 283 |
| FFFFFHFEFEFHFEFE | 284 |
| WWWWWRWDWRWDWRWD | 285 |
| WWWWWRWRWDWDWRWR | 286 |
| WWWWWEWKWEWKWEWK | 287 |

(continued)

| Sequence (N → C) | SEQ ID NO: |
|---|---|
| WWWWWEWEWKWKWEWE | 288 |
| WWWWWRWEWRWEWRWE | 289 |
| WWWWWRWRWEWEWRWE | 290 |
| WWWWWKWDWKWDWKWD | 291 |
| WWWWWEWHWEWHWEWH | 292 |
| WWWWWEWEWHWHWEWE | 293 |
| WWWWWRWRWRWRWDWD | 294 |
| WWWWWRWRWRWDWDWD | 295 |
| WWWWWHWDWHWDWHWD | 296 |
| WWWWWHWDWDWHWDWD | 297 |
| WWWWWHWEWEWHWEWE | 298 |
| PPPPPRPDPRPDPRPD | 299 |
| PPPPPRPRPDPDPRPR | 300 |
| PPPPPEPKPEPKPEPK | 301 |
| PPPPPEPEPKPKPEPE | 302 |
| PPPPPRPEPRPEPRPE | 303 |
| PPPPPRPRPEPEPRPE | 304 |
| PPPPPKPDPKPDPKPD | 305 |
| PPPPPEPHPEPHPEPH | 306 |
| PPPPPEPEPHPHPEPE | 307 |
| PPPPPRPRPRPRPDPD | 308 |
| PPPPPRPRPRPDPDPD | 309 |
| PPPPPHPDPHPDPHPD | 310 |
| PPPPPHPDPDPHPDPD | 311 |
| PPPPPHPEPEPHPEPE | 312 |
| SSSSSRSDSRSDSRSD | 313 |
| SSSSSRSRSDSDSRSR | 314 |
| SSSSSESKSESKSESK | 315 |
| SSSSSESESKSKSESE | 316 |
| SSSSSRSESRSESRSE | 317 |
| SSSSSRSRSESESRSE | 318 |
| SSSSSKSDSKSDSKSD | 319 |
| SSSSSESHSESHSESH | 320 |
| SSSSSESESHSHSESE | 321 |
| SSSSSRSRSRSRSRSR | 322 |
| SSSSSRSRSRSRSDSD | 323 |
| SSSSSRSRSRSDSDSD | 324 |
| SSSSSHSDSHSDSHSD | 325 |
| SSSSSHSHSHSHSHSH | 326 |
| SSSSSHSDSDSHSDSD | 327 |
| SSSSSHSESESHSESE | 328 |
| TTTTTRTDTRTDTRTD | 329 |
| TTTTTRTRTDTDTRTR | 330 |
| TTTTTETKTETKTETK | 331 |
| TTTTTETETKTKTETE | 332 |
| TTTTTRTETRTETRTE | 333 |
| TTTTTRTRTETETRTE | 334 |
| TTTTTKTDTKTDTKTD | 335 |
| TTTTTETHTETHTETH | 336 |
| TTTTTETETHTHTETE | 337 |

(continued)

| Sequence (N → C) | SEQ ID NO: |
|---|---|
| TTTTTRTRTRTRTRTR | 338 |
| TTTTTRTRTRTRTDTD | 339 |
| TTTTTRTRTRTDTDTD | 340 |
| TTTTTHTDTHTDTHTD | 341 |
| TTTTTHTHTHTHTHTH | 342 |
| TTTTTHTDTDTHTDTD | 343 |
| TTTTTHTETETHTETE | 344 |
| CCCCCRCDCRCDCRCD | 345 |
| CCCCCRCRCDCDCRCR | 346 |
| CCCCCECKCECKCECK | 347 |
| CCCCCECECKCKCECE | 348 |
| CCCCCRCECRCECRCES | 349 |
| CCCCCRCRCECECRCES | 350 |
| CCCCCKCDCKCDCKCD | 351 |
| CCCCCECHCECHCECH | 352 |
| CCCCCECECHCHCECE | 353 |
| CCCCCRCRCRCRCRCR | 354 |
| CCCCCRCRCRCRCDCD | 355 |
| CCCCCRCRCRCDCDCD | 356 |
| CCCCCHCDCHCDCHCD | 357 |
| CCCCCHCHCHCHCHCH | 358 |
| CCCCCHCDCDCHCDCD | 359 |
| CCCCCHCECECHCECE | 360 |
| YYYYYRYDYRYDYRYD | 361 |
| YYYYYRYRYDYDYRYR | 362 |
| YYYYYEYKYEYKYEYK | 363 |
| YYYYYEYEYKYKYEYE | 364 |
| YYYYYRYEYRYEYRYE | 365 |
| YYYYYRYRYEYEYRYE | 366 |
| YYYYYKYDYKYDYKYD | 367 |
| YYYYYEYHYEYHYEYH | 368 |
| YYYYYEYEYHYHYEYE | 369 |
| YYYYYRYRYRYRYRYR | 370 |
| YYYYYRYRYRYRYDYD | 371 |
| YYYYYRYRYRYDYDYD | 372 |
| YYYYYHYDYHYDYHYD | 373 |
| YYYYYHYHYHYHYHYH | 374 |
| YYYYYHYDYDYHYDYD | 375 |
| YYYYYHYEYEYHYEYE | 376 |
| NNNNNRNDNRNDNRND | 377 |
| NNNNNRNRNDNDNRNR | 378 |
| NNNNNENKNENKNENK | 379 |
| NNNNNENENKNKNENE | 380 |
| NNNNNRNENRNENRNE | 381 |
| NNNNNRNRNENENRNE | 382 |
| NNNNNKNDNKNDNKND | 383 |
| NNNNNENHNENHNENH | 384 |
| NNNNNENENHNHNENE | 385 |
| NNNNNRNRNRNRNRNR | 386 |
| NNNNNRNRNRNRNDND | 387 |

(continued)

| Sequence (N → C) | SEQ ID NO: |
|---|---|
| NNNNNRNRNRNDNDND | 388 |
| NNNNNHNDNHNDNHND | 389 |
| NNNNNHNHNHNHNHNH | 390 |
| NNNNNHNDNDNHNDND | 391 |
| NNNNNHNENENHNENE | 392 |
| QQQQQRQDQRQDQRQD | 393 |
| QQQQQRQRQDQDQRQR | 394 |
| QQQQQEQKQEQKQEQK | 395 |
| QQQQQEQEQKQKQEQE | 396 |
| QQQQQRQEQRQEQRQE | 397 |
| QQQQQRQRQEQEQRQE | 398 |
| QQQQQKQDQKQDQKQD | 399 |
| QQQQQEQHQEQHQEQH | 400 |
| QQQQQEQEQHQHQEQE | 401 |
| QQQQQRQRQRQRQRQR | 402 |
| QQQQQRQRQRQRQDQD | 403 |
| QQQQQRQRQRQDQDQD | 404 |
| QQQQQHQDQHQDQHQD | 405 |
| QQQQQHQHQHQHQHQH | 406 |
| QQQQQHQDQDQHQDQD | 407 |
| QQQQQHQEQEQHQEQE | 408 |

Hydrophilic tails can be added to the SAP, alone or in addition to hydrophobic tails, to facilitate interaction with the ECM of different vessels or tissues, such as the bladder.

## D. Formulations of SAPs

The compositions can be used to prevent or limit movement of a bodily fluid, to stabilize tissue, components thereof or cells, or to prevent contamination when administered to a site in need thereof. The compositions can be in the form of a dry powder, a wafer, a disk, a tablet, a capsule, a liquid, a gel, a cream, a foam, an ointment, an emulsion, a coating on a medical device or implant, incorporated into a microparticle, a polymeric matrix, a hydrogel, a fabric, a bandage, a suture, a contact lens, an eye drop, an eye patch, or a sponge.

The concentration of the SAP in any given formulation can vary and can be between approximately 0.1% and 99%, inclusive, preferably between 0.1% and 10%. In one embodiment, the concentration of the SAP in a liquid formulation is between approximately 0.1 and 10.0% (1-100 mg/m1). The concentration of SAP can be higher in stock solutions and in solid (*e.g.,* powdered) formulations. Solid preparations may have a concentration of SAP approaching 100% (*e.g.,* the concentration of SAP can be 75, 80, 85, 90, 95, 96, 97, 98, 99% or more (*e.g.,* 99.99%) of the composition).

In some embodiments, compositions of SAP are formulated for application to the eye as a dry powder. Dry powder formulation may contain at least 75% weight/weight (w/w) SAP, at least 80% w/w, at least 85% w/w, at least 90% w/w, at least 95% w/w, or more than 95% w/w.

In other embodiments, the SAP are formulated for application to the eye as a solution. Based on studies using RADA (SEQ ID NO: 57) and EAKA (SEQ ID NO: 77), SAP can be can be present in an aqueous solution that contains from about 0.25% weight/volume (w/v), to at least 7.5% w/v, preferably from about 1% w/v, to about 6% w/v, inclusive, for example, at least 0.1%, such as 0.1%-1%, 0.5%-5%, 1%-4%, 1%-5%, 1%-6%, 2%, 3%, 4% or 5% w/v. In some embodiments, at least 75%, at least 80%, at least 85%, at least 90%, at least 95%, or more than 95% of the SAP have the same size and sequence. In particular embodiments the SAP include two or more repeating units of the sequence RADA (SEQ ID NO: 57), two or more repeating units of the sequence EAKA (SEQ ID NO: 77), or combinations thereof.

In some forms, compositions of SAP formulated for application onto the surface of the eye are preferably of low viscosity, and flow across the entire surface of the eye to form a continuous SAP structure (*e.g.,* one "layer") across the exposed surface of the cornea. The concentration of SAP in the formulations for application to the surface of the eye typically are between about 0.05% and about 0.5% w/v, for example, 0.1%-0.3 % of the solution, in an amount sufficient to coat the entire exposed surface of the cornea. An exemplary volume for application to the surface of the eye is between about 10 μl and about 1,000 μl.

The viscosity of the SAP formulation may be adjusted to mimic that of a bodily fluid, for example, the vitreous humor. In other embodiments, the SAP forms a barrier to movement of fluids and bodily substances, for example, to prevent or reduce cell-cell interactions and cell-cell signaling within or at the surface of the eye. In these embodiments, the concentration of the SAP within the solution is between about 1% and about 4% w/v, for example, 2.5% w/v. Further, the density of the material may be adjusted or sufficiently raised such that it maintains enhanced resting contact with a healing retina, thereby allowing the patient greater freedom of head movement of the head.

Assembly of the SAP can be initiated or enhanced by the addition of an ionic solute or diluent to a solution of the SAP or by a change in pH. For example, NaCl at a concentration of at least 5 mM can induce the assembly of macroscopic structures within a short period of time (*e.g.*, within a few seconds to minutes). Lower concentrations of NaCl may also induce assembly but at a slower rate. Alternatively, self-assembly may be initiated or enhanced by introducing the SAP (whether dry, in a semi-solid gel, or dissolved in a liquid solution that is substantially free of ions) into a fluid *(e.g.,* a physiological fluid such as blood or gastric juice) or an area (*e.g.,* by topical application on to the surface of the eye, or by direct injection into the interior of the eye) including such ions. The gel does not have to be preformed prior to application to the desired site. Generally, self-assembly is expected to occur upon contacting the SAP with such a solution in any manner.

A wide variety of ions, including anions and cations (whether divalent, monovalent, or trivalent), can be used. For example, one can promote a phase transition by exposure to monovalent cations such as $Li^+$, $Na^+$, $K^+$, $Cs^+$, and $Ca^+$. The concentration of such ions required to induce or enhance self-assembly is typically at least 5 nM to 5 mM. Lower concentrations also facilitate assembly, although at a reduced rate. When desired, SAP can be delivered with a hydrophobic material (*e.g.* pharmaceutically-acceptable oil) in a concentration that permits self-assembly, but at a reduced rate. When SAP are mixed with a hydrophobic agent such as an oil or lipid the assembly of the material forms different structures. In some cases when another material is added, the material will assemble into various other three-dimensional structures that may be suitable for loading of a therapeutic agent. The hydrophilic portion of the molecule will assemble in such a way as to minimize hydrophobic-hydrophilic interaction, thereby creating a barrier between the two environments. Several experiments have shown that the SAP aligns on the surface of the oil with the hydrophobic portion of the molecule orienting toward the surface and the hydrophilic portion of the molecule facing away from the oil, or will form toroidal-like structures with the hydrophobic material contained inside. This type of behavior enables the encapsulation of therapeutics, prophylactic, or diagnostic molecules of interest for delivery in the body.

The composition may contain a salt scavenger to drive assembly to a preferred configuration. For example, circular dichroism ("CD") experiments indicate that the assembly dynamics can be controlled using salt scavengers or salt enhancement to increase the formation of β-sheets, α-helices, or more random configurations. The compositions may optionally contain an indicator showing the configuration of the assembly (*e.g.*, α-helix, β-sheet, lattice, *etc.*).

Alternatively, some of the described materials do not require ions to self-assemble but may self-assemble due to interactions with solvent, hydrophobic interactions, side chain interactions, and hydrogen bonding.

The materials can be formed within regularly or irregularly-shaped molds, which may include the surface of the eye, or a cavity on the surface of the eye, or a portion of the eye (*e.g.,* a tear in the cornea, or de-epithelialized section of the cornea) or which may be an inert material such as plastic or glass. The structures or scaffolds can be made to conform to a predetermined shape or to have a predetermined volume. To form a structure with a predetermined shape or volume (*e.g.,* a desired geometry or dimension, including thin sheets or films), an aqueous solution of the material is placed in a pre-shaped casting mold, and the materials are induced to self-assemble by the addition of a plurality of ions. Alternately, the ions may be added to the solution shortly before placing the solution into the mold, provided that care is taken to place the solution into the mold before substantial assembly occurs. Where the mold is a tissue (*e.g.*, within the eye or surrounding the eye, whether *in situ* or not), the addition of an ionic solution may not be necessary. The resulting material characteristics, the time required for assembly, and the dimensions of the macroscopic structure that forms are governed by the concentration and amount of solution that is applied, the concentration of ions used to induce assembly of the structure, and the dimensions of the casting apparatus. The assembled material can achieve a gel-like or substantially solid form at room temperature, and heat may be applied to facilitate the molding (*e.g.,* one can heat a solution used in the molding process (*e.g.,* a precursor-containing solution) to a temperature ranging up to about body temperature (approximately 37°C)). Once the assembled material has reached the desired degree of firmness, it can be removed from the mold and used for a described purpose. Alternatively, the materials described may be used to anchor host tissue to a tissue matrix or scaffold. For example, the described materials can be used as a "glue" to anchor host tissue that is to be regenerated to a tissue matrix or scaffold to ensure that the matrix or scaffold stays in place in the local environment to which it is injected or implanted. Tissue matrices and scaffolds are well known in the art and can be prepared from synthetic, semi-synthetic, and/or natural materials.

Materials that assemble and/or undergo a phase transition (*e.g.*, a transition from a liquid state to a semi-solid, gel, *etc.*) when they come in contact with bodily fluids (*e.g.,* the tear film) or an ionic solution are useful in providing an SAP structure at the surface or, or within one or more cavities of the eye. The SAP structure is effective to treat and prevent one or more symptoms of an eye disease. It may be that the SAP reduce or prevent inflammation, reduce or prevent the formation of adhesions between diseased tissue and surrounding tissues, or induce and enhance the restoration of a damaged corneal epithelium.

Self-assembly or phase transition is triggered by components found in a subject's body (*e.g.,* ions) or by physiological pH and is assisted by physiological temperatures. Self-assembly or phase transition can begin when the compositions are exposed to or brought into contact with a subject's body (*e.g.,* at the surface of the eye) and may be facilitated by the local application of heat to the area where the composition has been (or will be) deposited. Based on studies to date, self-assembly occurs rapidly upon contact with bodily fluids without the application of additional heat. The time required for effective assembly and/or phase transition can occur in 60 seconds or less (*e.g.,* in 50, 40, 30, 20, or 10 seconds or less) following contact with a subject's tissue, or to conditions similar to those found within the body. For example, solutions containing SAP can form a self-assembled fluid-impermeable structure upon contact with physiological fluids within times as short as 10 seconds following application. In some circumstances, such as when conditions are sub-optimal (*i.e.,* non-physiological), or when the concentration of self-assembling precursors is low, self-assembly or phase transition may take longer to achieve, for example, up to a minute, 5 minutes, 10 minutes, 30 minutes, an hour, or longer.

The compositions can form structures that are substantially rigid (*e.g.,* solid or nearly solid) or that assume a definite shape and volume (*e.g.,* structures that conform to the shape and volume of the location to which a liquid composition was administered, whether *in vivo* or *ex vivo*). The solidified SAP may be somewhat deformable or compressible after assembly or phase transition, but it will not substantially flow from one area to another, as compositions at a different point along the liquid to solid continuum may do, which may be due, at least in part, to their ability to undergo phase transitions. As a result, the compositions can also be used to prevent the movement of a bodily substance in a subject in need thereof. Self-assembly can be achieved *in vivo* or *ex vivo* by exposure to conditions within a certain range of physiological values (*e.g.,* conditions consistent with the tears at the surface of the eye) or non-physiological conditions. "Non-physiological conditions" refers to conditions within the body or at a particular site that deviate from normal physiological conditions at that site. Such conditions may result from trauma, surgery, injury, infection, or a disease, disorder, or condition. The SAP should self-assemble under such conditions. While liquid formulations are readily dispensed, the compositions administered may also be in a gel form that may become stiffer upon contact with the surface of the subject's eye.

Regardless of the precise nature of the SAPs, upon exposure to conditions such as those described, the SAP can form membranous two- or three-dimensional structures including a stable macroscopic porous matrix having ordered or non-ordered interwoven nanofibers (*e.g.,* fibers approximately 5-20 nm in diameter, with a pore size of about 50-100 nm in a linear dimension). Three-dimensional macroscopic matrices can have dimensions large enough to be visible under low magnification (*e.g.,* about 10-fold or less), and the membranous structures can be visible to the naked eye. Although three-dimensional, the structures can be exceedingly thin, including a limited number of layers of molecules (*e.g.,* 2, 3, or more layers of molecules). Typically, each dimension of a given structure will be at least 10 $\mu$m in size (*e.g.,* two dimensions of at least 100-1000 $\mu$m in size *(e.g.,* 1-10 mm, 10-100 mm, or more)). The relevant dimensions may be expressed as length, width, depth, breadth, height, radius, diameter, or circumference in the case of structures that have a substantially regular shape (*e.g.,* where the structure is a sphere, cylinder, cube, or the like) or an approximation of any of the foregoing where the structures do not have a regular shape.

The SAP can form a hydrated material when contacted with water under conditions such as those described (*e.g.,* in the presence of a sufficient concentration (*e.g.,* physiological concentrations) of ions (*e.g.,* monovalent cations)). These may have a high water content (*e.g.,* approximately 95% or more (*e.g.,* approximately 96%, 97%, 98%, 99% or more)), and the compositions can be hydrated but not substantially self-assembled. A given value may be "approximate" in recognition of the fact that measurements can vary depending, for example, on the circumstances under which they are made and the skill of the person taking the measurement. Generally, a first value is approximately equal to a second when the first falls within 10% of the second (whether greater than or less than) unless it is otherwise clear from the context that a value is not approximate or where, for example, such value would exceed 100% of a possible value.

The properties and mechanical strength of the structures or scaffolds can be controlled as required through manipulation of the components therein. For example, the stiffness of an assembled gel can be increased by increasing the concentration of SAP therein, as discussed above. Alternatively, it may be desirable for different parts of the SAP formulation to have different mechanical properties. For example, it may be advantageous to alter the stability or density of the SAP formulation by manipulating the amino acid sequence. This may be desirable when the SAP formulations are used to fill a void, such that the edges of the material self-assemble to attach to the tissue site while the rest of the SAP formulation flows out into the void. In another example, the density of the material may be adjusted or sufficiently raised such that it maintains enhanced resting contact with a healing retina, thereby allowing the patient greater freedom of head movement of the head. The sequences, characteristics, and properties of the SAP formulations and the structures formed by them upon self-assembly are discussed further below.

**E. Additional Agents**

The compositions of SAP can include other agents, such as therapeutic, prophylactic or diagnostic agents. The additional agents are typically non-self-assembling. These can be a biomolecule which is a molecule such as a peptide, proteoglycan, lipid, carbohydrate, or a small molecule. Like small molecules, biomolecules can be naturally occurring or may be artificial (*i.e.,* they may be molecules that have not been found in nature). For example, a protein having a sequence that has not been found in nature (*e.g.,* one that does not occur in a publicly available database of sequences) or that has a known sequence modified in an unnatural way by a human hand (*e.g.,* a sequence modified by altering a post-translational process such as glycosylation) is an artificial biomolecule. Nucleic acid molecules encoding such proteins (*e.g.,* an oligonucleotide, optionally contained within an expression vector) are also biomolecules and can be incorporated into the compositions described. For example, a composition can include a plurality of SAP and cells that express, or that are engineered to express, a protein biomolecule (by virtue of containing a nucleic acid sequence that encodes the protein biomolecule).

Many different therapeutic, prophylactic or diagnostic agents can be incorporated into the formulation. One or more therapeutic, diagnostic and/or prophylactic agents can be administered simultaneously with the SAP in the same formulation, administered simultaneously in separate formulations, or sequentially. Alternatively, the agent(s) can be covalently or non-covalently coupled to the SAP, either directly or via an intermediate molecule.

In some embodiments, compositions of SAP include one or more non-self-assembling therapeutic agents. For example, the additional agents can include one or more classes of therapeutic agents for the treatment of ocular diseases, such as anti-inflammatory agents, vasoactive agents, anti-infective agents, anesthetics, growth factors, vitamins, nutrients, and/or cells. Additional therapeutic agents can be selected according to the disease that is to be treated, and the route of administration. In some embodiments, additional therapeutic agents are suitable for topical application to the eye. In other embodiments, additional therapeutic agents are suitable for parenteral application to the eye.

In some embodiments, the compositions include one or more ophthalmic drugs, including but not limited to anti-glaucoma agents, anti-angiogenesis agents, anti-infective agents, anti-inflammatory agents, analgesics, anesthetics, growth factors, immunosuppressant agents, anti-allergic agents, anti-oxidants, cytokines, and combinations thereof.

In some embodiments, the ophthalmic drug is present in its neutral form, or in the form of a pharmaceutically acceptable salt. In some cases, it may be desirable to prepare a formulation containing a salt of an agent due to one or more of the salt's advantageous physical properties, such as enhanced stability or a desirable solubility or dissolution profile. Generally, pharmaceutically acceptable salts can be prepared by reaction of the free acid or base forms of an agent with a stoichiometric amount of the appropriate base or acid in water or in an organic solvent, or in a mixture of the two; generally, non-aqueous media like ether, ethyl acetate, ethanol, isopropanol, or acetonitrile are preferred. Pharmaceutically acceptable salts include salts of an agent derived from inorganic acids, organic acids, alkali metal salts, and alkaline earth metal salts as well as salts formed by reaction of the drug with a suitable organic ligand (*e.g.,* quaternary ammonium salts). Lists of suitable salts are found, for example, in Remington's Pharmaceutical Sciences, 20th ed., Lippincott Williams & Wilkins, Baltimore, MD, 2000, p. 704. Examples of ophthalmic drugs sometimes administered in the form of a pharmaceutically acceptable salt include timolol maleate, brimonidine tartrate, and sodium diclofenac.

In some embodiments, compositions of SAP include one or more anti-glaucoma agents for local administration to the eye or surrounding tissues. Representative anti-glaucoma agents include prostaglandin analogs (such as travoprost, bimatoprost, and latanoprost), beta-adrenergic receptor antagonists (such as timolol, betaxolol, levobetaxolol, and carteolol), alpha-2 adrenergic receptor agonists (such as brimonidine and apraclonidine), carbonic anhydrase inhibitors (such as brinzolamide, acetazolamide, and dorzolamide), miotic or parasympathomimetic drugs (such as pilocarpine and ecothiopate), serotonergic agents, muscarinic agents, dopaminergic agents, and adrenergic agents (such as apraclonidine and brimonidine).

In some embodiments, compositions of SAP include one or more anti-angiogenesis agents for local administration to the eye or surrounding tissues.

Representative anti-angiogenesis agents include, but are not limited to, antibodies to vascular endothelial growth factor (VEGF) such as bevacizumab (AVASTIN®) and rhuFab V2 (ranibizumab, LUCENTIS®), and other anti-VEGF compounds including aflibercept (EYLEA®); MACUGEN® (pegaptanib sodium, anti-VEGF aptamer or EYE001) (Eyetech Pharmaceuticals); pigment epithelium derived factor(s) (PEDF); COX-2 inhibitors such as celecoxib (CELEBREXO) and rofecoxib (VIOXX®); interferon alpha; interleukin-12 (IL-12); thalidomide (THALOMID®) and derivatives thereof such as lenalidomide (REVLIMID®); squalamine; endostatin; angiostatin; ribozyme inhibitors such as ANGIOZYME® (Sirna Therapeutics); multifunctional antiangiogenic agents such as NEOVASTAT® (AE-941) (AEterna Laboratories, Quebec City, Canada); receptor tyrosine kinase (RTK) inhibitors such as sunitinib (SUTENT®); tyrosine kinase inhibitors such as sorafenib (Nexavar®) and erlotinib (Tarceva®); antibodies to the epidermal grown factor receptor such as panitumumab (VECTIBIX®) and cetuximab (ERBITUX®), as well as other anti-angiogenesis agents known in the art.

In some embodiments, compositions of SAP include one or more vasoconstrictor agents for local administration to the eye or surrounding tissues.

Representative vasoconstrictors include epinephrine and phenylephrine. Vasoconstrictors such as phenylephrine can be included to prolong the effect of local anesthesia (*e.g.,* 0.1-0.5% phenylephrine). Analgesic agents other than a local anesthetic agent, such as steroids, non-steroidal anti-inflammatory agents like indomethacin, platelet activating factor (PAF) inhibitors such as lexipafant, CV 3988, and/or PAF receptor inhibitors such as SRI 63-441.

In some embodiments, compositions of SAP include one or more local anesthetics for local administration to the eye or surrounding tissues. A local anesthetic is a substance that causes reversible local anesthesia and has the effect of loss of the sensation of pain. The SAP composition may include an anesthetic agent in an amount of, *e.g.,* about 0.1%, about 0.2%, about 0.3%, about 0.4%, about 0.5%, about 0.6%, about 0.7%, about 0.8% about 0.9%, about 1 .0%, about 2.0%, about 3.0%, about 4.0%, about 5.0%, about 6.0%, about 7.0%, about 8.0%, about 9.0%, or about 10% by weight of the total composition. The concentration of local anesthetics in the compositions can be therapeutically effective meaning the concentration is adequate to provide a therapeutic benefit without inflicting harm to the patient.

In some embodiments, compositions of SAP include one or more anti-infective or antimicrobial agents (*e.g.,* an antibiotic, antibacterial, antiviral, or antifungal agent) for local administration to the eye or surrounding tissues.

Other therapeutic agents can be included in the compositions, such as growth factors to accelerate one or more aspects of the recovery from, or prevention of a disease or disorder (*e.g.,* angiogenesis, cell migration, process extension, and cell proliferation). The growth factor or another agent can be a chemotactic substance, which has the ability, *in vivo* or in cell culture, to recruit cells to a site at which the substance is present. The cells recruited may have the potential to contribute to the formation of new tissue or to augment and reinfoRCES existing, damaged tissue (*e.g.,* by contributing structurally and/or functionally to the tissue (*e.g.,* by providing growth factors or contributing to a desirable immune response)).

In some embodiments, the compositions include one or more anti-inflammatory agents include steroid and non-steroid drugs. Suitable steroids agents include glucocorticoids, progestins, mineralocorticoids, and corticosteroids. Small molecule steroidal anti-inflammatories include prednisone, dexamethasone, cortisone, loteprednol, triamcinolone acetonide, fluocinolone acetonide, fluorometholone, and fluticasone. Exemplary immune-modulating drugs include cyclosporine, tacrolimus and rapamycin. In some embodiments, anti-inflammatory agents are biologic drugs that block the action of one or more immune cell types such as T cells, or block proteins in the immune system, such as tumor necrosis factor-alpha (TNF-alpha), interleukin 17-A, interleukins 12 and 23.

The compositions can include a coloring agent. Suitable coloring agents include commercially available food colorings, natural and synthetic dyes, and fluorescent molecules.

In some embodiments, the compositions include cells. Where cells are delivered to the eyes of a patient (*e.g.,* to treat or prevent one or more diseases of the eye), autologous cells can be used.

## F. Excipients, Carriers, and Devices

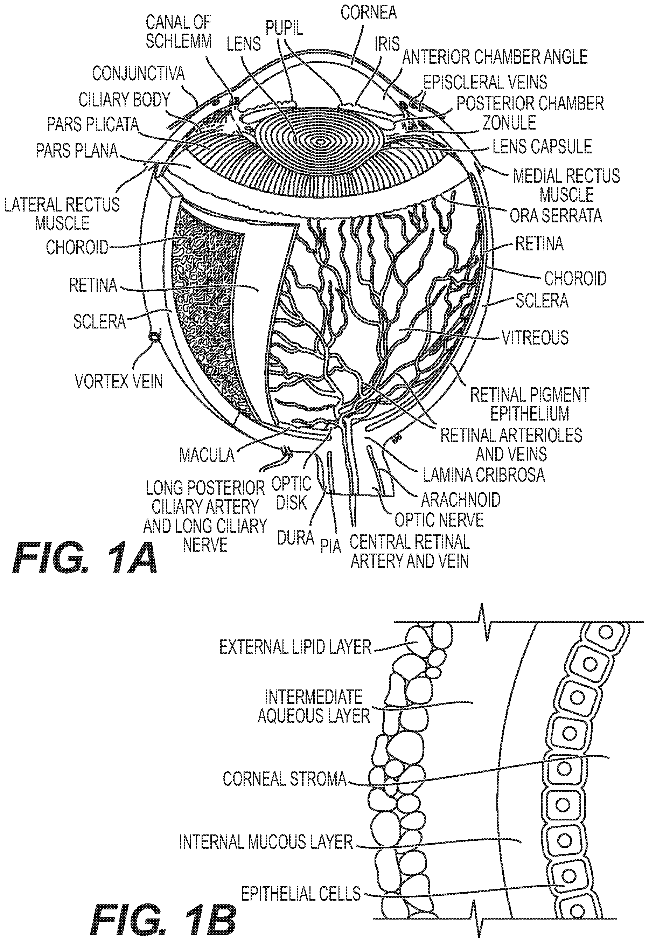

Compositions of SAP can include excipients suitable for administration onto or into the eye. For example, compositions of SAP can be formulated into a composition suitable for topical administration onto the surface of the eye, or for injection into one or more of the cavities within the eye (see, for example, the schematic depiction of the eye in Figure 1A). Formulations for application to the cornea are typically a liquid solution or suspension. These may be injected into the eye, the tissue surrounding the eye, or into a compartment of the eye, or topically applied to the eye or cornea. Topical administration can include application directly to exposed tissue, vasculature or to tissues or prostheses, for example, during surgery, or by direct administration to the skin.

In the preferred embodiment, the formulation is a liquid or reconstitutable powder, applied topically. The formulations can include a pharmaceutically acceptable carrier or are provided as part of a medical device or coating.

In some forms, the formulation is provided as a dry or lyophilized powder which can be administered directly as a powder which hydrates at the site of application.

Alternatively, the formulation is suspended or dissolved in a solvent, most preferably aqueous, and applied as a spray, paint, or injection. The formulation can also by administered in a hydrogel such as chitin, collagen, alginate, or a synthetic polymer. Any formulation suitable for application to the eye (*e.g.,* a liquid, which can be applied as a spray or a powder) can be used. In another embodiment, the formulation is provided as a coating on a device, for example a contact lens or adhesive bandage, which may be dissolved in an aqueous solution and dried on the device or mixed with a polymeric carrier and applied to the device. In yet another embodiment, the formulation is provided in a bandage, foam or matrix, in which the peptides may be dispersed or absorbed.

The formulation can also be in the form of sutures, tape, or adhesive. In some embodiments, for example, where the formulation is administered to the eye of a patient that has a disease or disorder relating to a prior injury to the eye, the SAP are formulated either alone, or together with other agents (*e.g.,* with anesthetics, anti-inflammatories, growth factors, anti-infectives, *etc.*), in the form of a foam, matrix or bandage, for example to reduce or stop suppuration, bleeding or loss of other bodily fluids, as required.

Suitable excipients can be selected based upon the desired assembly-state of the self-assembling precursor materials. For example, when the SAP are delivered as a solution, a suitable excipient may contain a concentration of ions below the threshold required to initiate assembly. Representative excipients include solvents, diluents, pH modifying agents, preservatives, antioxidants, suspending agents, wetting agents, viscosity modifiers, tonicity agents, stabilizing agents, and combinations thereof. A preferred excipient is water.

Solutions, suspensions, or emulsions for ocular or intraocular administration may be buffered with an effective amount of buffer necessary to maintain a pH suitable for ocular administration. Suitable buffers are well known by those skilled in the art and some examples of useful buffers are acetate, borate, carbonate, citrate, and phosphate buffers. Solutions, suspensions, or emulsions for ocular administration may also contain one or more tonicity agents to adjust the isotonic range of the formulation. Suitable tonicity agents are well known in the art and include, for example, glycerin, mannitol, sorbitol, sodium chloride, and other electrolytes.

In some instances, the formulation is distributed or packaged in a liquid form. Alternatively, formulations for ocular administration can be packed as a solid, obtained, for example by lyophilization of a suitable liquid formulation. The solid can be reconstituted with an appropriate carrier or diluent prior to administration or application onto the eye.

Typically, when the composition is dispensed in a multi-dose container that is to be used over a longer period of time, such as 24 hours, a preservative must be added to ensure microbiologic safety over the period of use.

Numerous ophthalmological excipients are known in the art and available. They may contain suitable additives, such as preservatives, antioxidants, and stabilizing agents.

In some embodiments, compositions of SAP are formulated as an ophthalmic solution for administration directly into the surface of the eye as an eye drop. Eye drops can contain SAP in any form suitable for administration onto the surface of the eye, such as solutions, emulsions, suspensions and ointments.

In some embodiments, compositions of SAP are formulated as an ophthalmic emulsion. Ophthalmic emulsions are generally dispersions of oily droplets in an aqueous phase. There should be no evidence of breaking or coalescence.

In some embodiments, compositions of SAP are formulated as an ophthalmic suspension. Ophthalmic suspensions contain solid particles dispersed in a liquid vehicle; they must be homogeneous when shaken gently and remain sufficiently dispersed to enable the correct dose to be removed from the container. Sediment that may occur should disperse readily when the container is shaken, and the size of the dispersed particles should be controlled. The SAP precursors, or self-assembled SAP and any other suspended material must be reduced to a particle size small enough to prevent irritation and damage to the cornea.

In some embodiments, compositions of SAP are formulated as an ophthalmic ointment. Ophthalmic ointments are sterile, homogeneous, semi-solid preparations intended for application to the conjunctiva or the eyelids. They are usually prepared from non-aqueous bases, *e.g.,* soft paraffin (VASELINE®), liquid paraffin, and wool fat.

Ideally, the pH of ophthalmic drops should be equivalent to that of tear fluid, which is 7.4. However, the decision to add a buffering agent should be based on stability considerations, as well as the conditions required to maintain the SAP in the desired state of assembly. The pH selected should be optimized for both stability of the SAP and physiological tolerance. For example, any buffer system must not cause precipitation or deterioration of the SAPs. The influence on the lachrymal flow should also be taken into account. Although solutions with the same pH as lacrimal fluid (7.4) are ideal, the outer surfaces of the eye tolerate a larger range, 3.5 to 8.5. The normal useful range to prevent corneal damage is 6.5 to 8.5. The final pH of the solution is often a compromise, because many ophthalmic drugs have limited solubility and stability at the desired pH of 7.4. Exemplary pH values for buffers formulated with excipients for topical administration include pH 5.0, 5.5, 6.0, 6.5, 7.0, 7.5, 8.0 and 8.5.

Ophthalmic solutions are ordinarily buffered at the pH of maximum stability of the drug(s) they contain. The buffers are included to minimize any change in pH during the storage life of the composition; this can result from absorbed carbon dioxide from the air or from hydroxyl ions from a glass container. Changes in pH can affect the solubility and stability of drugs; consequently, it is important to minimize fluctuations in pH. Therefore, in some embodiments, buffers or pH adjusting agents or vehicles are added to adjust and stabilize the pH at a desired level.

The buffer system should be sufficient to maintain the pH throughout the expected life of the product, but with a low buffer capacity so that when the ophthalmic solution is instilled into the eye, the buffer system of the tears will rapidly bring the pH of the solution back to that of the tears. In some embodiments, low concentrations of buffer salts (*i.e.,* less than 20 mM, for example, 10 mM, or 5 mM) are used to prepare buffers of low buffer capacity that do not induce or otherwise influence the self-assembly of the SAP or self-assembling peptidomimetics.

Ophthalmic drops are considered isotonic when the tonicity is equal to that of a 0.9% solution of sodium chloride. The eye can usually tolerate solutions equivalent to 0.5-1.8% of sodium chloride. There are times when hypertonic ophthalmic solutions are necessary therapeutically, or when the addition of an auxiliary agent required for reasons of stability supersedes the need for isotonicity. A hypotonic ophthalmic solution will require the addition of a substance (tonicity adjusting agent) to attain the proper tonicity range.

The most widely used ophthalmic buffer solutions are boric acid vehicle and Sorensen's modified phosphate buffer. The boric acid vehicle is a 1.9% solution of boric acid in purified water or preferably sterile water. It is isotonic with tears. It has a pH of approximately 5 and is useful when extemporaneously compounding ophthalmic solutions of drugs that are most stable at acid pH. This vehicle does not possess large buffering capacity, but it is sufficient to stabilize pH for the short expiratory periods used for compounded solutions, without overwhelming the natural buffers in lacrimal fluid. The second most commonly used buffer solution is the Sorensen's modified phosphate buffer and is used for drugs needing pH values between the range of 6.5-8.0. This buffer uses two stock solutions, one acidic containing $NaH_2PO_4$, and one basic containing $Na_2HPO_4$. Preferred ophthalmological excipients for topical administration are biocompatible, non-toxic and do not induce inflammation.

For application by the ophthalmic mucous membrane route, compositions may be formulated as eye drops or eye ointments. Typically, the eye drops are in the form of an aqueous solution, for example, including one or more excipients suitable for administration into the eye. Most formulations applied topically as drops are administered as volumes of 0.03 and 0.07 ml/drop, with a maximum typical volume of about 100 microliters. The formulations may also be provided in two units, one lyophilized and one a diluent for re-suspension of the lyophilized agent(s).

In some forms, compositions of SAP are formulated to include a pharmaceutically acceptable excipient for parenteral administration to the eye. For example, injectable solutions can be prepared by incorporating the SAP in the required amount in the appropriate solvent or dispersion medium with one or more pharmaceutically acceptable excipients, as required. Generally, dispersions are prepared by incorporating the various compositions into a sterile vehicle which contains the basic dispersion medium and the required other ingredients. In the case of powders for the preparation of injectable solutions, the preferred methods of preparation are vacuum-drying and freeze-drying techniques which yield a powder of the SAP plus any additional desired ingredient from a previously prepared solution thereof.

In some forms, pharmaceutical formulations for administration by injection are an aqueous solution or suspension of the SAPs. In preferred embodiments, formulations for injection into the eye include less than 20 mM ions, for example, between 20 mM and 0.01 mM ions, inclusive. In a particular embodiment, formulations of SAP for injection into the eye include a solution of the SAP in water. In other embodiments, the solutions of the SAP include one or more polymer conjugates. Exemplary solvents include, for example, water, Ringer's solution, phosphate buffered saline (PBS), and isotonic sodium chloride solution. The formulation may also be a sterile solution, suspension, or emulsion in a nontoxic, acceptable diluent or solvent such as 1,3-butanediol.

Solutions, suspensions, or emulsions for injection or instillation into the eye may be combined with an effective amount of buffer necessary to maintain a pH suitable for ocular administration. Suitable buffers are well known in the art, Examples include acetate, borate, carbonate, citrate, and phosphate buffers.

Solutions, suspensions, or emulsions for injection or instillation administration may contain at least one tonicity agents to adjust the isotonic range of the formulation. Suitable tonicity agents are well known in the art. Examples include glycerin, mannitol, sorbitol, sodium chloride, and electrolytes.

Formulations for intravitreal injection can be formulated to a desired volume. For example, the volume suitable for intravitreal injection is typically between about 0.03 and 0.1 ml. Preferred excipients for intravitreal injection are biocompatible, non-toxic and do not induce inflammation.

Solutions, suspensions, or emulsions for ocular administration may contain one or more preservatives to prevent bacterial contamination of the ophthalmic preparations. Suitable preservatives known in the art include polyhexamethylene biguanide (PHMB), benzalkonium chloride (BAK), stabilized oxychloro complexes (otherwise known as Purite®), phenylmercuric acetate, chlorobutanol, sorbic acid, chlorhexidine, benzyl alcohol, parabens, thimerosal, and mixtures thereof.

Solutions, suspensions, or emulsions for ocular administration may also contain one or more excipients known in the art, such as dispersing agents, wetting agents, and suspending agents.

The compositions of SAP can include additional organic and/or inorganic materials, for example, to provide a structure or physical support for the SAP. In some embodiments, the additional materials provide structural support to the compositions, such as materials that provide a scaffold. Scaffold materials can be selected to provide physical strength, elasticity, porosity, solubility, volume and bulk, as required by the application. In certain embodiments, the scaffold material has mechanical and/or biological properties similar to that of the extracellular matrix (ECM).

Scaffold materials can include natural or synthetic polymers, including natural polymers such as polypeptides and proteins, may create a scaffold onto which SAP, therapeutic agents, cells or other agents are attached or associated. In some embodiments, the described compositions include proteins, such as ECM proteins. Exemplary natural scaffold materials include alginate, fibrinogen, hyaluronic acid, starch, chitosan, silk, gelatin, dextran, elastin, collagen, and combinations thereof. In some embodiments, compositions of SAP include scaffold materials that are synthetic polymers. Exemplary synthetic polymers include poly(L-lactic acid co-$\varepsilon$-caprolactone) (PLCL), polyhydroxy acids such as poly(DL-lactic acid) (PDLA) and poly(lactic-co- glycolic acid) (PLGA), poly(ethylene oxide) (PEO); poly(vinyl alcohol) (PVA,; poly (methyl methacrylate) (PMMA), poly(ethylene-co-vinyl acetate) (PEVA), polystyrene; polyurethane; and mixtures thereof. In preferred embodiments the scaffold materials are biocompatible. In preferred embodiments the scaffold materials do not induce an immune response.

SAP structures can biodegrade at a time following application that is consistent with the time required for treatment, for example, the amount of time required for restoration or regeneration of the corneal epithelium, such as one day, one week, one month or more than one month following application.

Contact lenses including SAP can be used daily, overnight or long-term. Contact lenses including SAP can be formulated for use in therapeutic or cosmetic applications, according to the needs of the intended recipient. In some embodiments, SAP are applied to commercially available contact lenses, such as commercially-available cosmetic or therapeutic contact lenses. Application can occur before or after assembly of the SAP has occurred, and can be carried out by any suitable means known in the art for application, such as spraying, coating, painting, *etc.* Contact lenses including SAP can include one or more therapeutic, diagnostic or prophylactic agents.

When the contact lens does not contain an additional scaffold or support structure, additional agents can be mixed into the SAP prior to assembly to form an SAP structure having non-self-assembling agents incorporated or entrapped therein, or bound directly to the SAP prior to assembly. In some embodiments, additional agents are applied to one or more surfaces of an SAP structure, which is subsequently applied to the eye for use as a contact lens, or which is incorporated into one or more additional structures for use as a contact lens.

In some embodiments, contact lenses including SAP are optically clear. For example, the SAP and any support structures or materials are optically clear or transparent when desired. In other embodiments, SAP for use in contact lenses are formulated to include one or more coloring agents or dyes to, for example, prevent, reduce or otherwise alter the passage of light through the contact lens. For example, in certain embodiments, contact lenses including SAP are dyed or tinted to reduce or limit exposure of light to the eye. When support structures or surfaces are used, the support structure or surface can be colored or dyed to alter the amount or wavelength of light that reaches the eye. The SAP can include a coloring agent or dye in an amount sufficient to completely obscure the passage of light through the assembled structure.

Contact lenses can include a single SAP structure or can include two or more distinct layers of SAP structures, for example, including the same or different SAP and optionally including one or more additional agents.

Contact lenses can be packaged and stored in appropriate containers. The lenses can be stored within a suitable fluid, for example, contact lens solution. In some embodiments, the storage fluid or contact lens solution includes one or more SAPs. When contact lens solution includes one or more SAP, the ionic concentration of the solution is typically below 20 mM. The SAP can be added to the solution as required, for example, to maintain a constant concentration. In some embodiments, contact lenses are continually worn and immersed within a solution containing self-assembling precursor materials, for example, to maintain an SAP structure at the surface of the lens, for application to the surface of the eye.

Ocular wound bandages including SAP are described. Ocular wound bandages containing SAP can be formulated for use in therapeutic or cosmetic applications, according to the needs of the intended recipient. In some embodiments, SAP are applied to commercially available ocular wound bandages, such as commercially-available gauzes or eye patches. Application can occur before or after assembly of the SAP has occurred, and can be carried out by any suitable means known in the art for application, such as spraying, coating, painting, *etc.*

In certain embodiments, compositions of SAP are applied in the form of a solution or powder directly onto gauze or other non-peptide structures. For example, SAP and/or scaffold materials can be contacted with tissue around and within the eye, and held in place by a bandage or gauze, or as one component of an eye patch.

## G. Kits

The SAP can be assembled in kits, together with instructions for use. The kit may also include one or more of a syringe *(e.g.,* a barrel or bulb syringe) a dropper or dropper bottle, a needle, a pipette, gauze, sponges, or cotton, swabs, an eye bath, a bandage, a contact lens, an eye patch, a disinfectant, surgical thread, scissors, a scalpel, a sterile fluid, a spray canister, including those in which a liquid solution is sprayed through a simple hand pump, a sterile container, disposable gloves or an eye dropper.

Exemplary kits include SAP, self-assembling peptidomimetics, or combinations of SAP and peptidomimetics, excipients, suitable means for application, and instruction for use. In some embodiments, kits include measured dosages of one or more SAP and/or self-assembling peptidomimetics for application at distinct times. For example, a kit can include SAP having a different sequence, dose, or conjugated to a different or the same agent(s) for distinct applications into the same eye. Dosages, and application regimens can be determined according to the needs of the patient, for example, as specified by a physician or within the instructions.

Kits can include one or more means for administration into or onto the eye. Typically, the applicator will initiate or maintain contact between one or more SAP with one or more parts of the eye. In some embodiments, the applicator is pre-filled or loaded with the SAP. When SAP contained within the applicator, the amount and formulation of the SAP formulation to be dispensed can be fixed or varied, for example, by the patient or physician. Exemplary, applicators include an eye-dropper, a syringe, an eye bath, a spray, an air-applicator, a contact lens, an eye patch and a tube. In some embodiments, the SAP are provided in one or more containers, for example, in dried form *(e.g.,* lyophilized), or a solution, tablet, wafer, or gel. In some embodiments, the SAP are pre-packaged in a concentration stock powder or solution, with instructions for diluting to the desired concentration prior to application.

Kits including dried SAP can be packaged with a desiccant. In some embodiments, kits include one or more pharmaceutically acceptable excipients for administration into the eye. The excipient can be contained within a separate container or within an applicator. Therefore, in some embodiments, an applicator can include one or more SAPs, and one or more excipients within the same or different compartments. In some embodiments, one or more SAP is diluted or otherwise mixed with one or more excipients within the applicator prior to application. The amount and type of SAP to be administered can be pre-determined within the applicator or varied according to the desired effects.

In some embodiments, kits include an air applicator for the directed application of SAP in the form of a powder to the surface of the eye. The amount of SAP administered can be varied. The contacted surface area of the eye can be adjusted, for example, to a narrowly-defined area or the entire exposed portion of the eye. In other embodiments, kits include an apparatus to deliver a powder to the eye via a ballistic injection through the outer layers of the eye. In other embodiments, kits include an apparatus to deliver a solution or gel via a single or series of injections or infusion.

In some embodiments, kits include an applicator that will mix two or more different SAP together, to be dispensed and/or delivered together in one application or in a series of applications. For example, the device can contain several chambers, each of which contains an SAP which is specific for the eye. The composition can be dispensed directly onto the site of administration or can be mixed in a mixing chamber within the device prior to administration. In one embodiment, an applicator can be used to administer the same composition to the eye on several different occasions or different compositions to the eye at the same or different times.

### III. Methods of Making SAP and Compositions Thereof

Compositions of SAP can be prepared using any techniques known in the art. SAP are typically synthesized using standard procedures, so any technique in the art suitable to prepare synthetic peptides can be used. All of the described method steps can be performed in any suitable order unless otherwise indicated or otherwise clearly contradicted by context.

### A. Production of SAP

SAP can be chemically synthesized or purified from natural or recombinantly-produced sources, by methods well known in the art. For example, peptides can be synthesized using standard Fmoc chemistry.

Standard Fmoc (9-florenylmethoxycarbonyl) derivatives include Fmoc-Asp(OtBu)-OH, Fmoc-Arg(Pbf)-OH, and Fmoc-Ala-OH. Couplings are mediated with DIC (diisopropylcarbodiimide)/6-Cl-HOBT (6-chloro-1-hydroxybenzotriazole). In some embodiments, the last four residues of the peptide require one or more recoupling procedures. In particular, the final Fmoc-Arg(Pbf)-OH coupling can require recoupling. For example, a second or third recoupling can be carried out to complete the peptide using stronger activation chemistry such as DIC/HOAT (1-hydroxy-7-azabenzotriazole) or HATU (1-[bis(dimethylamino)methylene]-1H-1,2,3-triazolo[4,5-b]pyridinium 3-oxid hexafluorophosphate)/NMM (N-methylmorpholine).

Acidolytic cleavage of the peptide can be carried out with the use of carbocation scavengers (thioanisole, anisole and $H_2O$). Optimization can be achieved by varying the ratio of the components of the cleavage mixture. An exemplary cleavage mixture ratio is 90:2.5:2.5:5 (TFA-thioanisole-anisole-$H_2O$). The reaction can be carried out for 4 hours at room temperature.

In some embodiments, the removal of residual impurities is carried out by wash steps. For example, trifluoroacetic acid (TFA) and organic impurities can be eliminated by precipitation and repeated washes with cold diethyl ether and methyl t-butyl ether (MTBE).

Peptides produced using the disclosed methods can be purified using high pressure liquid chromatography (HPLC). Suitable solvents for dissolving the peptides include neat TFA. In some embodiments, 8 mL TFA/g peptide is sufficient to fully dissolve peptides following precipitation. For example, TFA can be diluted into $H_2O$. Typically, the peptides remain soluble at TFA concentrations of 0.5% to 8% and can be loaded onto reverse phase (RP)-HPLC columns for salt exchange. Exemplary salt exchange methods use 3-4 column volumes of acidic buffer to wash away the TFA counter ion due to its stronger acidity coefficient. Buffers suitable for use in washing away the TFA counter ion include 0.1% HCl in $H_2O$.

Following removal of TFA, peptides can be eluted with a step gradient. Exemplary elution buffers include 30% acetonitrile (MeCN) vs. 0.1% HCl in $H_2O$. For acetate exchange, peptides can be loaded from the same diluted TFA solution, washed with 3-4 column volumes of 1% acetic acid (AcOH) in $H_2O$, followed by 2 column volumes of 0.1 M NHaOAc in $H_2O$, pH 4.4. In some embodiments, the column is washed again with 3-4 column volumes of 1% AcOH in $H_2O$.

Peptides can be eluted from the columns using a step gradient of 30% MeCN vs. 1% AcOH in $H_2O$. In some embodiments, the elution of peptides can be enhanced by acetate exchange. Exemplary buffers for acetate exchange include 0.1 M NHtOAc in $H_2O$, pH 4.4.

Analytical HPLC can be carried out to assess the purity and homogeneity of peptides. An exemplary HPLC column for use in analytical HPLC is a PHENOMENEX® JUPITER® column. In some embodiments, analytical HPLC is carried out using a column and buffer that are heated to a temperature s greater than 25 °C, for example 25-75 °C. In a particular embodiment analytical HPLC is carried out at temperatures of about 65 °C. A step gradient can be used to separate the peptide composition. In some embodiments, the gradient is from 1%-40% MeCN vs 0.05% TFA in $H_2O$. The change in gradient can be achieved over 20 min using a flow rate of 1 ml/min. Peptides can be detected using UV detection at 215 nm.

In some embodiments, methods of making the described compositions for administration to the eye include the step of sterilization. Where compositions are required to be sterilized or otherwise processed for the removal of undesirable contaminants and/or micro-organisms, filtration is a preferred method. Filtration can be achieved using any system or procedures known in the art. In some embodiments, filtration removes contaminants or prevents the growth or presence of microorganisms. Exemplary microorganisms and contaminants that can be removed include bacteria, cells, protozoa, viruses, fungi, and combinations thereof. In some embodiments, the step of filtration is carried out to remove aggregated or oligomerized proteins. For example, solutions of self-assembling precursor peptides or peptidomimetics can be filtered to remove assembled peptide structures or oligomers on the basis of size.

**B. Fabrication of Compositions for Treating Eye Disease**

When the SAP are used to form a gelled or solid structure, for example, to be applied to the eye as part of a backing on a contact lens, the SAP formulations can be produced using one more techniques. Examples include templating onto the surface; injection molding of a formulation of SAP in a solvent; stamping of a dry powder or frozen formulation of SAP in a solvent; direct application of a slurry formulation containing SAP onto a stencil or patterned surface, such as a bandage, adhesive bandage or a composite structure formed by a combination of these methods.

In some embodiments, SAP formulations are dried or dehydrated to remove a solvent. Any methods known in the art can be used for the dehydration of compositions including SAP.

The term "dried" or "lyophilized" is used to describe the product of a process for the removal of the majority of the solvent from a material in solution, for example, by methods for dehydration, vacuum sublimation or "lyophilization". These methods typically remove a major portion of solvent, such as water, from the material, but can result in a residual amount of solvent within the "dry" product. For example, a lyophilized powder may include up to 10% w/w of water, for example, 5%, 3%, 2%, 1% or 0.5% w/w of water.

Any composition or formula of SAP can include up to 25% by mass of derivative molecules and/or degradation products. Exemplary derivative molecules and/or degradation products include, but are not limited to, amino acid substitutions, amino acid deletions, oligomers including dimer, trimmers, tetramers or higher-order oligomers, aggregates and impurities. In some embodiments, formulations of SAP include less than or equal to 20% derivative molecules and/or degradation products by mass, such as 15%, 10% or less than 10%, such as 5%, 1% or 0%.

**IV. Methods of Use**

**A. Routes of Administration and Dosages**

SAP can be used for the treatment and prevention of eye diseases. Methods of using SAP and compositions thereof for the treatment and prevention of eye diseases typically include administering the SAP directly to the eye, either topically or by injection or by instillation.

Dosage units containing an amount of SAP to provide pain relief in the eye or ocular cavity are also provided. Dosage units can be prepared in an amount effective to prevent, reduce or inhibit sensory function at the surface of the eye in an amount sufficient to reduce pain associated with one or more diseases, disorders, injuries and related symptoms that affect the eye.

SAP used in the compositions to treat eye diseases can be assembled prior to or at the time of application of the composition to the eye, either by contacting the composition with an ionic solution or allowing the composition to contact a bodily fluid.

In preferred embodiments, SAP are administered once, twice, three times or more than three times a day directly to the eyes of the individual in need thereof. The frequency will vary depending on the severity of symptoms. The formulation may be applied as a drop in the form of an emulsion or suspension, lotion, ointment, cream, gel, salve or powder and sustained or slow release, as well as eyelid lotion. It may also be used as an eye wash or rinse to irrigate the eye. The formulation may also be applied in a sprayable form.

In preferred embodiments, the dosage administered to the eye does not result in toxicity or inflammation within or at the surface of the eye. For example, multiple applications per day for an extended period of time do not result in damage or toxicity to the cornea.

Exemplary conditions that can be treated include DED, RCES, ocular pain or irritation associated with a contact lens or ocular prosthesis. Methods for treating or preventing pain associated with eye surgery and disorders or diseases of the eye are also provided. An exemplary prosthetic or medical device that can be coated, treated or otherwise associated with SAP is an intraocular lens. Replacement lenses that are implanted inside the eye to replace the eye's natural lens when it is removed during cataract surgery are known in the art. In some embodiments, coating, covering or otherwise associating an intraocular lens with SAP prior to, during or immediately following cataract surgery enhances the outcome of cataract surgery. For example, in some embodiments, implanting an intraocular lens coated with SAP into a subject reduces or prevents posterior capsule opacity (PCO) and/or intraocular lens dislocation relative to implanting an untreated control intraocular lens. In some embodiments, the formulation is used in the implantation of complicated retinal grafts (photoreceptors and pigment epithelium) or in Pterygia/pterygium surgery, including use of autologous conjunctival autografting.

Methods of administering SAP into or onto the eye to control (e.g., prevent) or treat diseases, disorders, injuries and related symptoms that affect the eye in subjects are described. In some embodiments, SAP can be administered into or onto the eye either prophylactically or in response to disruption of the tear film or reduced tear production in a subject. For example, in some embodiments, the compositions are administered to prevent, treat, or reduce the symptoms of a disease, disorder, injury or related. The compositions can be used prior to or at the time of or after eye surgery or to restore and augment one or more layers of the surface of injured, damaged or diseased cornea in a subject in need thereof. Damage to the cornea can result from traumatic injury, or surgical incisions, or chemical or heat burns, or as a result of an infectious agent, such as a viral, fungal, bacterial or protozoan pathogen, from local or systemic diseases and disorders, such as glaucoma or diabetes, or from chemical imbalances, contact lens use, disease or aging leading to disruption of the tear film. Disorders may arise due to traumatic injury, surgery, burns, viral, fungal, bacterial or protozoan infection, acquired or congenital diseases and disorders of the eye or ocular cavity.

SAP can be used to repair, replace or augment one or more of the biological structures of the surface of the eye, for example, by providing an optically clear coating across the surface of the eye. In some embodiments, the methods treat and augment one or more layers within the tear film at the surface of the cornea, including the external lipid layer, the aqueous layer, the mucus layer, the epithelial layer, and the corneal stroma.

The SAP are administered in an amount sufficient to coat or cover the diseased or damage portion of the cornea. In some embodiments, the amount of SAP administered is sufficient to cover the entire exposed surface of the cornea. For example, SAP are administered as a solution in a volume sufficient to form a coating of up to 1 mm in thickness over the entire exposed surface of the cornea. The SAP self-assemble upon contacting the ocular surface to form a visually clear structure that covers the surface of the eye. The SAP structure can be of uniform thickness sufficient to augment and/or reproduce the outer surface layer(s) of the natural cornea and/or recreate the biological functions of the outer surface layer(s) of the cornea. In other embodiments, the SAP are administered to the eye to reduce or mediate one or more inflammatory responses associated with a disease or damage to one or more structures of the eye.

In some embodiments, the methods can reduce, prevent or otherwise control the biological functions of one or more cell types in or surrounding the eye. An exemplary cell type is a corneal keratinocyte cell. For example, in some embodiments, SAP are administered to the eye in an amount sufficient to prevent or reduce the deleterious effects of corneal scarring and opacity associated with uncontrolled activation of corneal keratinocytes in response to corneal damage. Therefore, methods for reducing the activation and/or proliferation of corneal keratinocytes by administering SAP to the eye are also provided. In some embodiments, the methods reduce or prevent the onset of scarring and fibrosis following damage to one or more of the structures of the eye, for example, associated with disease, trauma, or surgery. Exemplary surgical procedures of the eye include corneal transplant surgery, cataract removal/lens replacement surgery, pterygium removal surgery, conjunctival surgery, incisional glaucoma surgery, refractive surgery such as LASIK or PRK procedures, and other invasive eye procedures. In some embodiments, the methods reduce or prevent the onset of scarring and opacity following damage to the corneal surface, for example, associated with corneal surgery. Therefore, methods of prophylactically treating a site of disease or damage of the eye surface to maintain visual acuity or prevent or reduce the rate of deterioration of visual acuity are also provided. In some embodiments, SAP are administered to one or more structures of the eye to prevent the loss of vision, or to restore or enhance the vision of a subject in need thereof.

SAP can be administered via any means which is effective to deliver an effective amount of the SAP to the eye. For example, compositions can be administered by topical, intra-corneal, intravitreal, intracameral, periocular, punctual, subconjunctival, sub-tenon, subchoroidal, suprachoroidal and subretinal routes. Exemplary ocular compartments into which SAP can be administered include the vitreous chamber, subretinal space, subchoroidal space, episclera, conjunctiva, sclera, anterior chamber, and/or the cornea and compartments therein (e.g., subepithelial, intrastromal, endothelial).

The SAP can be applied to the outer surface of the eye, tissues contacting or surrounding the eye, or into one or more inner compartment to be treated. Exemplary tissues that can be contacted with the SAP include the lids, lacrimal glands, lacrimal ducts, conjunctiva, sclera, cornea, aqueous humor, iris, ciliary body, trabecular meshwork, lens, vitreous humor, retina, choroid, optic nerve and adnexa.

The compositions can be administered as a liquid or powder containing substantially non-assembled SAP (*i.e.,* SAP that has yet to undergo a phase transition from the liquid-to-solid state), or they can be administered in the form of a solid (*i.e., as a substantially assembled gel*). In other embodiments, the SAP can be administered to the eye as an emulsion, for example, as a mixture of solid particles including substantially-assembled peptides, and non-assembled precursor peptides.

In some embodiments, formulations of SAP are delivered directly to one or more of intraocular cavities within the eye using a syringe or other means for puncturing the outer surface of the eye and delivering the SAP. An exemplary procedure for administering formulations to the eye is intravitreal injection. Intravitreal injection is an injection into the vitreous, which is the jelly-like substance inside the eye. It is performed to place formulations inside the eye, such as near the retina. The formulations of SAP can be injected using a syringe, or other suitable deliver means.

In some embodiments, formulations of SAP are delivered directly to the surface of the eye using mechanical delivery means, *i.e.,* dropping, spraying, bathing of the eyes in a solution, or a combination of these. Most formulations applied topically as drops are administered as volumes of 0.03 and 0.07 ml/drop, with a maximum typical volume of about 100 microliters/drop.

In some embodiments, a powder of SAP is applied to the surface of the eye using an air applicator that can be adjusted for directed application to a narrow area or the entire exposed portion of the eye. In other embodiments, the powder can be delivered to the eye via a ballistic injection through the outer layers of the eye. In another embodiment, an applicator can mix several SAP together to be delivered in one or a series of applications or in a staged delivery system via which components or combinations thereof are delivered in a specific order to achieve a desired structure based on the sequence of delivery. The sequence of delivery can result in different structures by varying the amount and the form of delivery. In one example, the delivery sequence can begin with a powder, which is then followed by a liquid, which is in turn followed by a powder to result in a triple layered structure that can conform to specific shapes based on the area and volume covered.

The SAP can be assembled before, during or after application to the eyes. For example, the SAPcan be synthesized and the finished formulation exposed to an ionic solution to induce gel formation. The gelled structure can be stored until use. The gelled structure can be dehydrated prior to storage. The structure can be gelled in a mold to form a particular shape, for example, to form a layer of on the surface of a soft contact lens. In other embodiments, the SAP precursors are synthesized and stored in a substantially non-assembled form. The non-assembled SAP precursors can be dried prior to storage. Immediately prior to use, the dehydrated or dried non-assembled SAP can be exposed to an ionic solution to initiate assembly. The SAP can be gelled in a mold to form a particular shape. In still other embodiments, the gelled structure is applied or implanted in an unassembled form and the peptides assemble upon contact with a bodily fluid, such as the tear film. This can be useful, for instance, to allow SAP to assemble and foncform to the shape of the application site.

In some embodiments, methods include administering formulations of SAP including one or more therapeutic, prophylactic, and/or diagnostic agents, as discussed above. When the SAP are applied in the form of a gelled structure, the agent can be impregnated into an SAP structure and/or coated on the surface of the structure. In other embodiments, the methods include administering an agent that is covalently coupled to one or more of the SAPs. For example, in some embodiments, the formulations of SAP include a pH-adjusting agent which is released at the site of administration to alter the pH at the site of administration.

Since the SAP form an optically clear SAP structure, the methods can include creating a self-assembled barrier-structure that acts as a molecular "exclusion zone" at a desired location within the eye, for example, to prevent undesirable cellular proliferation. Typically, the formation of a self-assembled barrier that prevents passage of bodily fluids through the structure requires an SAP concentration of greater than about 1% w/v, for example, and amount between 1% and 4% w/v, inclusive. In an exemplary embodiment, the methods provide a self-assembled barrier-structure over the foveal region of the retina to exclude the growth of blood vessels in this area. Typically, the methods preserve normal vision throughout the healing process. The methods can be used in place of, or in addition to existing therapeutic techniques, such as laser removal of the blood vessels that have grown over the fovea. The methods result in an enhanced outcome relative to existing methods and protect the foveal region of the retina while preserving normal vison.

In another embodiment, SAP can be used to fill the vitreous cavity in lieu of or in combination with sterile saline (salt water) or with a vitreous substitute such as a gas bubble or silicone oil for posterior eye procedures, such as to lessen traction on the retina, to close retinal tissue breaks and minimize bleeding in retinal repair, or prevent proliferative vitreoretinopathy (PVR) by providing a barrier to stabilize the structure and minimize retinal pigment epithelial cell abnormal migration and proliferation. Further, adequate density of SAP could be valuable to lessen constraints on head movement and position for the recovering post-operative surgical patient.

In some embodiments, the SAP applied at the surface of the cornea can pass into and across the cornea to enter the inner compartments of the eye. For example, Cy5.5 conjugated to a RADARADARADARADA peptide (Cy5.5(RADA)$_4$CONH$_2$; SEQ ID NO: 434) administered onto the healthy corneal surface was shown to localize within the retina. In contrast, the Cy5.5 dye alone was not transported across the cornea. Specifically, a solution of Cy5.5 conjugated to RADA (Cy5.5(RADA)COOH; SEQ ID NO: 442), Cy5.5 conjugated to RADARADA (Cy5.5(RADA)$_2$COOH; SEQ ID NO: 444), and Cy5.5 conjugated to (RADA)$_4$ (Cy5.5(RADA)$_4$CONH$_2$; SEQ ID NO: 434) administered as eye drops was shown to localize to the retina. It may be that the size and/or sequence of the SAP is sufficient to enable passive or active uptake of non-assembled peptide monomers by transport processes at the surface of the cornea. The size of the RADA-16 peptide is approximately 10 nm.

Therefore, SAP bound to one or more agents can transport bound agents through the corneal surface without causing damage or inflammation of the cornea or the ocular compartments. The methods direct SAP across the cornea without the need for injections, penetration enhancers or surgical implements that pierce the cornea and risk potential infection in the eye.

In some embodiments, administration of the SAP to the corneal surface is carried out in two or more consecutive applications, to produce two or more distinct layers of SAP structures at the surface of the cornea.

The surface of the eye contains multiple stacked layers, including the oil (top) layer; the aqueous (second) layer; the mucous (third) layer, and the epithelial (base) layer (see Figure 1B). The outer three layers form the tear film, which constantly changes, at least in part due to blinking. The structure and function of each layer can vary, depending on the disease state of the eye. For example, in high pressure glaucoma, typically, tear duct outflow from the tear ducts is reduced, and the corneal surface structure changes. The reduction of outflow can lessen the overall volume of liquid available to the eye surface, and it can cause elongation, or "stretching" of the conformation of collagen fibers present within the cornea. The difference in the surface area of the collagen can vary by as much as 15%, opening pores within the tissue, thereby enhancing the risk of infection and causing pain or discomfort in the eyes. Each blink of the eyelid (opening and closing) displaces approximately 80% of the fluid content of the tear film, eye drop, or other fluid placed on the eye. To counteract this loss, SAP administered to the eye needs to be able to interact with a low degree of mobility to adhere to the eye. Therefore, in some embodiments, administration of SAP onto the corneal surface provides a first SAP structure or "layer" that is directly in contact with the surface of the cornea. In some embodiments, the SAP provided in the first administration includes one or more tissue-specific motifs, for example, to bind to the corneal surface. An exemplary tissue-specific motif is the sequence MSCRAMM (SEQ ID NO: 141).

A second administration of the SAP onto the corneal surface provides a second SAP structure or "layer" that is directly in contact with the first layer. The second or other middle layer(s) can have a combination of hydrophilic material to enable fluid flow and maintain the correct orientation of the third or outer layer. Therefore, the second can interact with the first layer and be an intermediary to anchor the third, or further layer(s).

A third administration of the SAP onto the corneal surface provides a third SAP structure or "layer" that is directly in contact with the second layer. In some embodiments the third layer is sensed when the eyelid moves over the eye. In some embodiments, the third layer can form a fluid-resistant barrier to reduce the evaporation due to the orientation of the hydrophobic layer to the air. The SAP for use in each of the layers can be designed according to the needs of the subject, for example, to reduce evaporation, to reduce or prevent bacterial, viral or fungal infection at the surface of the cornea, prevent dust and debris from entering the tear film, *etc.* Therefore, each of the layers can be designed to provide relatively greater or reduced fluid flow between the layers. In some embodiments, one or more of the layers is a barrier to fluid movement. In other embodiments, one or more of the layers directs the movement of fluids out of the eye or the penetration of gases into the eye.

To form layers at the surface of the eye, the SAP can be administered to the corneal surface in consecutive applications, followed by a time interval sufficient to allow assembly of the SAP. An exemplary time interval is between about 30 seconds and 10 minutes, for example, 1, 2, 3, 4, 5 or 6 minutes between applications. Different or equivalent administration forms, for example, powders and solutions can be used for successive applications, according to the needs of the patient and the desired properties of the resulting structures. For example, application of a powder may provide an SAP structure having greater interaction with the ocular surface than does a solution.

In some embodiments, SAP is administered to the eye to produce a depot in or around the eye. In an exemplary embodiment, the SAP are introduced within a slow-release formulation into the eyelid, for example, when implanted within a medical device. In order to counteract fluid loss due to blinking, for example, SAP can be slowly and continuously released across the surface of the eye following each blink.

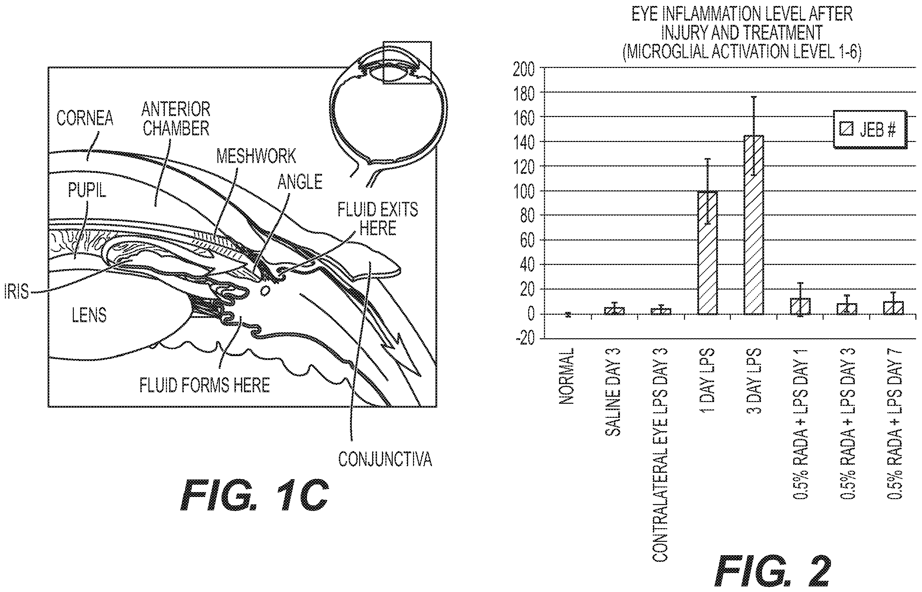

In an exemplary embodiment, SAP are applied to the eye with, or as part of, such as a coating on, a medical device, for example, as a solution for slow-release from the medical device. In an exemplary embodiment, a medical device for dispensing the SAP is embedded within one or more structures proximal to or within the eye of the subject. In some embodiments, a medical device for measured or continuous release of SAP is embedded within tear duct or within the eyelid of a subject for the treatment of DED, following or during, for example, reconstructive surgery. The constant application of a low-viscosity solution of SAP enables deposition and spreading of the tear film, maintains the overall makeup of the tear film to treat and prevent DED, and provide a visibly clear artificial cover across the eye. Therefore, a medical device slowly releasing the SAP from the eyelid or tear duct serves as a reservoir. A representation depicting the relative orientations of the tear duct and the cornea is provided in Figure 1C.

In further embodiments, the methods include introducing to the surface of the eye, SAP containing a backing or support layers that provides support and/or protection, such as a soft contact lens. The backing or support layer may be biodegradable or non-biodegradable and can be composed of any material that is biocompatible for those embodiments, wherein the backing layer is also applied/implanted into the eye. In other embodiments, the methods include the step of removing the backing layer prior to application of the SAPs. The backing layer can be adhesive or non-adhesive. The backing layer can have associated with it one or more therapeutic, prophylactic, and/or diagnostic agents as discussed above. In some embodiments, the SAP are administered onto the surface of the eye in the form of a contact lens, or as a coating or layer associated with a contact lens or other implant that is placed directly onto the surface of the eye. Typically, the SAP contact the surface of the eye upon administration of the lens. The contact lens can be held in place for the desired amount of time, and replaced or repeatedly applied as necessary, for example, until the desired treatment or prevention has been achieved. Therefore, in some embodiments, administration of SAP can occur via their controlled release from a contact lens. For example, the SAP would migrate into the interface of the contact lens and the cornea where the SAP will then start to self-assemble or cause a non-adherent surface to be created between the contact lens. This would allow for lens removal while protecting the corneal surface, for example, as the SAP wicks into the interface between the contact lens and the cornea. Material wicked into the interface subsequently may be able to penetrate the tissue and deliver a bound cargo molecule to the interior of the eye or remain in the interface between the lens and the tissue.

## B. Diseases and Disorders to be Treated

SAP and compositions thereof, optionally including one or more additional therapeutic agents, can be used to treat or prevent any eye diseases or disorders according to the methods.

Symptoms of exemplary diseases, disorders, injuries and related symptoms that affect the eye that can be treated include keratitis, conjunctivitis, DED and RCES, allergic conjunctivitis, exposure keratopathy, amoebic keratitis, fungal keratitis, bacterial keratitis, viral keratitis, onchocercal keratitis, bacterial keratoconjunctivitis, viral keratoconjunctivitis, corneal dystrophic diseases, Fuchs' endothelial dystrophy, Stevens-Johnson syndrome, corneal neovascularization diseases, post-corneal transplant rejection prophylaxis and treatment, autoimmune uveitis, infectious uveitis, anterior uveitis, posterior uveitis (including toxoplasmosis), pan-uveitis, an inflammatory disease of the vitreous or retina, endophthalmitis prophylaxis and treatment, macular edema, macular degeneration, age related macular degeneration, corneal ectasia, keratoconus proliferative and non-proliferative diabetic retinopathy, hypertensive retinopathy, proliferative vitreoretinopathy, an autoimmune disease of the retina, primary and metastatic intraocular melanoma, other intraocular metastatic tumors, open angle glaucoma, closed angle glaucoma, pigmentary glaucoma and combinations thereof. In some embodiments, the methods are effective to treat and prevent one or more symptoms of an acute eye disease or disorder. In some embodiments, the methods are effective to treat and prevent one or more symptoms of a chronic eye disease. In other embodiments, the methods are effective to treat and prevent one or more symptoms or disorders of the eye resulting from a traumatic or surgical injury. Therefore, the methods can be used to treat and prevent conditions of the surface of the eye or one or more of the interior compartments of the eye resulting from chemical burns, heat burns, surgery, injury, or other damage resulting to exposure to one or more environmental or chemical agents.

## Treatment in Conjunction with Vitrectomy

For example, SAP may be used whether or not in conjunction with vitrectomy to address prophylactically or therapeutically to other ophthalmologic conditions, such as retinal detachment and proliferative vitreoretinopathy (PVR). Vitrectomy may be performed for a range of eye applications including to repair or remove a vitreous opacity, reduction of abnormal traction on the retina, for retinal surgery, for certain vitreoretinal diagnostic procedures, and to place a separate device or drug.

Retinal detachment occurs when subretinal fluid accumulates in the potential space between the neurosensory retina and the underlying retinal pigment epithelium (RPE). Depending on the mechanism of subretinal fluid accumulation, retinal detachments traditionally have been classified into rhegmatogenous, tractional, and exudative. The most common type is a rhegmatogenous retinal detachment, which occurs when a tear in the retina leads to fluid accumulation with a separation of the neurosensory retina from the underlying retinal pigment epithelium.

SAP can be used to fill the vitreous cavity in lieu of or in combination with sterile saline (salt water) or with a vitreous substitute such as a gas bubble or silicone oil for posterior eye procedures, such as to lessen traction on the retina, to close retinal tissue breaks and minimize bleeding in retinal repair, or prevent proliferative vitreoretinopathy (PVR) by providing a barrier to stabilize the structure and minimize retinal pigment epithelial cell abnormal migration and proliferation. Adequate density of SAP can be used to lessen constraints on head movement and position for the recovering post-operative surgical patient.

Other relevant conditions and procedures include, removal of vitreous opacities, separation of the vitreous from the retina, membrane peeling to lessen retinal traction, and placement of gas bubble. Barrier properties may prevent neovascularization in diabetic retinopathy.

**Prophylactic Treatment**

The methods can be used for prophylactic treatment, for example, to prevent the onset or development of a disease or disorder of the eye. In some embodiments, the methods include treating an individual who has not been diagnosed with one or more diseases or disorders of the eye, but who has been identified as being at risk of developing or acquiring one or more diseases, disorders or injuries of the eye. Exemplary subjects at risk of developing or acquiring one or more diseases, disorders or injuries of the eye include subjects with metabolic disease, at risk of developing infectious diseases, who are immunocompromised, or individuals exposed to trauma, such as car wrecks, sports head injuries, and explosive devices. In some embodiments, the methods are used to treat or prevent disorders in diabetic subjects. In other embodiments, the methods are used to treat or prevent disorders in patients prior to, during or after one or more investigative or surgical procedures of the eye.

**Ocular Inflammation**

In some embodiments, SAP and compositions thereof are used to treat or prevent inflammatory responses within or around the eye. Therefore, methods of administering SAP to one or more of the structures of the eye for treating and preventing ocular inflammation and/or intraocular inflammation are provided.

Suppression of unwanted inflammation resulting from disease and/or injury can be of high clinical importance in many environments. For example, early and effective treatment of traumatic eye injuries may be critical to vision preservation. Self-assembling peptides and related peptidomimetics (SAPs), a class of materials that can form a biocompatible barrier of intertwined nanofibers on contact with a charged surface such as a wound, appear to provide a solution for the significant unmet needs in corneal and other ocular structure healing after acute eye injury in military settings.

Care protocols for ophthalmic injuries and diseases indicate that the most important medical actions for an ocular injury are evaluation of the extent of the injury, evacuation of the subject for more specialized treatment, and immediate protection of the eye from further damage.

In some embodiments, SAPs immediately help to repair and/or mitigate further structural damage with a barrier that limits or prevents inflammation, infection and contamination, and creates a microenvironment to enable restoration of the cornea, external and internal globe structures, surrounding socket, optic nerve and related tissue.

Examination of an injured eye may be hampered by hemorrhage, tissue damage or pain. SAP used shortly after injury during initial field-based examination can stop bleeding and allow better visualization of the injury while relieving pain, protecting ocular structures and stabilizing the wound. In some embodiments, SAP is applied immediately following trauma.

**Dry Eye Disease (DED)**

In some embodiments, SAP and compositions thereof are used to treat or prevent symptoms of DED, also known as keratoconjunctivitis sicca (Lemp, et al., Report of the International Dry Eye Workshop (DEWS) Ocul Surf 5: 65-204 (2007)), which is a multifactorial disease of the tears and ocular surface that may cause discomfort, visual disturbance, tear film instability and damage to the ocular surface. It is typically accompanied by increased osmolarity of the tear film and inflammation at the ocular surface. DED is associated with a range of symptoms and is often further described according to its underlying cause, examples of which include autoimmune, environmental, age-related, procedure-related (Lasik, etc.), and viewing related (reading, computer, movie, television, and driving) factors. In addition, adhesions may form between the palpebral conjunctiva of the eyelids and the corneal epithelium in DED patients, causing recurrent pain and further damage to the corneal epithelium.

Treatment, which typically includes frequent applications per day of artificial tears and non-prescription eye drops to mitigate irritation and lubricate the eyes, usually provides only minimal and limited relief without modifying the disease course. DED is common, afflicting tens of millions of people globally.

In some embodiments, compositions of SAP are effective to treat and prevent one or more symptoms of DED by reducing the evaporation of tears or tear film, preventing the formation of adhesions, providing a continuous surface to maintain the tear film, enhancing or inducing healing of damaged corneal tissue, reducing inflammation of the cornea, reducing irritation and pain, or combinations of these. In some embodiments, SAP are effective for the treatment and prevention of DED associated with Sjogren's syndrome.

**Drug-induced Eye Conditions**

The formulations are also suitable for use in the management of eye problems that arise as a side effect of using one or more systemic drugs. The SAP formulations are used prior, during or after taking one or more systemic drugs. In some embodiments, SAP can be administered into or onto the eye prophylactically, to prevent disruption of the tear film or tear production in a subject identified at risk of disruption of the tear film or tear production. Therefore, methods of administering SAP into or onto the eye for treating and preventing eye diseases, disorders, injuries and related symptoms that affect the eye in subjects who have taken or are prescribed to take one or more therapeutic agents associated with disruption of the tear film or tear production are provided. Exemplary drugs that can cause ocular side effects include corticosteroids, antihistamines, antipsychotic medications, anti-malarial medications, blood pressure medications, herbal medicines, erectile dysfunction drugs, anticholinergics, immuno-suppressants, antibiotics, antiarrhythmic agents, and anti-cancer drugs/treatment. Some specific examples are bisphosphonate, amiodarone, tamsulosin, topiramate, ethambutol, minocycline, cyclosporine and tacrolimus.

Corticosteroids used for many conditions, such as asthma, allergies, arthritis and dermatitis, can cause swelling in the back of the eye or retina and may lead to cataracts. Antihistamines, used for conditions such as allergies, can raise certain patients' risk for glaucoma. Antipsychotic medications, such as THORAZINE® and MELLARIL® can be toxic to the retina. Anti-malarial medications, such as PLAQUENIL® (hydroxychloroquine), used to treat malaria, lupus and rheumatoid arthritis, is a known retinal toxin, and the effects are irreversible. FOSAMAX®, a bisphosphonate prescribed for post-menopausal women to prevent calcium bone loss, can cause orbital inflammation, uveitis and scleritis. Cyclosporine and Tacrolimus, commonly used in patients who have undergone organ or bone marrow transplants, can cause posterior reversible encephalopathy syndrome. These patients present with bilateral vision loss. Minocycline is a tetracycline derivative commonly used to treat acne. Minocycline can cause increased intracranial pressure and papilledema, which can cause permanent vision loss if not reversed. Ethambutol is widely used to treat mycobacterial disease, including tuberculosis; if not taken at safe doses, it is an optic nerve toxin. Topiramate (Topamax) is used to treat epilepsy and migraine headaches, and it is used off-label for weight loss. It can cause angle-closure glaucoma soon after starting treatment. Tamsulosin (Flomax), which is used to treat prostate enlargement and improve urinary flow in men. The well-known syndrome, intraoperative floppy iris syndrome, is often associated with men who were on medicine to relax their prostate. Anticholinergics *e.g.,* dicyclomine (BENTYL®), and other drugs with anticholinergic effects, are administered to patients who have stomach conditions that require stomach relaxers and to patients with Parkinson's disease. Young patients taking these drugs will develop difficulty with accommodation of the eyes. Erectile dysfunction drugs, *e.g.,* sildenafil citrate (VIAGRA®) and tadalafil (CIALIS®) are often prescribed for men with erectile dysfunction. Some ocular side effects are blue vision, and ischemic optic neuropathy. Blood pressure medications can cause glaucoma.

In some embodiments, the formulations and methods are used for treating, alleviating, and/or preventing one or more ocular symptoms that arise as a side effect from taking a systemic drug.

In some embodiments, the formulations and methods are used for treating, alleviating, and/or preventing one or more ocular symptoms in patients with ocular graft versus host disease. Ocular Graft Versus Host Disease (GVHD) occurs in patients who have undergone allogenic hematological stem cell transplantation. It can occur in patients who have acute or chronic GVHD, though it is more common in patients with the chronic form. Approximately 40-90% of patients with chronic GVHD will develop ocular symptoms.

**Recurrent Corneal Erosion Syndrome (RCES)**

SAP and compositions thereof are used to treat or prevent symptoms of RDEC. RCES is a common clinical disorder characterized by a disturbance at the level of the corneal epithelial basement membrane, resulting in defective adhesions and recurrent breakdowns of the epithelium. It may arise spontaneously or from anterior basement membrane dystrophy *(e.g.,* Cogan dystrophy or map dot fingerprint dystrophy) or from other problems, such as adhesions between the palpebral conjunctiva of the eyelids and the corneal epithelium. Pain associated with RCE, whether due to trauma or to anterior basement membrane dystrophy, results from abnormalities in the epithelial basement membrane.

Corneal erosions are common chronic ocular disorders and can worsen with time, especially if neglected. RCES may occur either secondary to corneal injury or spontaneously. When RCES arises spontaneously, a predisposing factor, such as corneal dystrophy, diabetes, or infection, may be the underlying cause. Management of RCES is usually aimed at regenerating or repairing the epithelial basement membrane to restore the adhesion between the epithelium and the anterior stroma. However, current limited options for effectively controlling the development and progression of RCES typically include application of lubricating ointment to prevent surface aggravation and antimicrobials to prevent and reduce infection of the damaged mucosal tissue. Treatment can be prolonged and arduous, often leading to poor patient compliance poor therapeutic outcomes.

Although often unsuccessful, RCES treatment may include punctal occlusion, in which a plug is inserted into the tear duct, or direct application of a bandage soft contact lens. Patients with refractory RCES may undergo surgical interventions, such as Anterior Stromal Micropuncture (ASM), phototherapeutic keratectomy and debridement of the corneal epithelium, however, the attendant risks include scarring, glare, and blurred vision, and they often fail.

Epithelial basement membrane dystrophy is usually bilateral and characterized by various patterns of dots, parallel lines that mimic fingerprints, and patterns that resemble maps, which appear in the epithelium. Individual microcysts may be oval, oblong, or comma-shaped and rarely appear alone but usually are associated with map and fingerprint patterns. On the other hand, the map and fingerprint patterns appear without dots or individual microcysts. Map and fingerprint alterations of the corneal epithelium are not rare and can be found in asymptomatic individuals without prior history of trauma or ocular disease. Epithelial changes are more common than previously recognized and are frequently present in conditions involving corneal edema *(e.g.,* such as near a healing cataract surgery incision) or in the center of the cornea associated with Fuchs corneal dystrophy.

The epithelial healing process begins when basal epithelial cells undergo mitosis, producing new cells that occupy fresh wounds. Basal cells adhere the epithelium to the stroma in two ways: they secrete the basement membrane, and they contain hemidesmosomes, which behave as linchpins that protrude through the posterior surface of basal cells and into the stroma; each is held in place by an anchoring fibril. Any disruption to basal cell production makes the eye more prone to recurrent erosion.

RCES occurs because of a defect in the epithelial basement membrane and in hemidesmosomes formation, resulting in epithelial loss, microcysts, and bullae. RCES occurring after corneal injury or insult typically results from improper or inadequate healing of the basement membrane, typically either because the basal epithelial cells fail to produce proper basement membrane complexes to attach to the Bowman layer and stroma or because of faulty basement membrane adherence.

Prognosis for RCES due to trauma is better than RCES that arises the spontaneously. The disease process underlying spontaneous RCES may be epithelial basement membrane corneal dystrophy. Electron microscope studies of tissue during RCES episodes show separation of the anchoring system at the level of the epithelial cell membrane or below the level of the anchoring plaques. Normal and degenerate polymorphonuclear leucocytes (PMNs) are found within and between the epithelial cells and within the anchoring layer. The degenerate PMNs may secrete metalloproteinases that cleave the Bowman layer below the anchoring system.

In some embodiments, compositions of SAP are effective to treat and prevent one or more symptoms of RCES by reducing the evaporation of tears or tear film, preventing the formation of adhesions, providing a continuous surface to maintain the tear film, enhancing or inducing healing of the damaged corneal tissue, reducing inflammation of the cornea, reducing irritation and pain, or combinations of these. In a particular embodiment, compositions of SAP are effective to treat and prevent one or more symptoms of RCES by enhancing or inducing the regeneration or repair of the epithelial basement membrane to restore the adhesion between the epithelium and the anterior stroma. This enhancement can be achieved by forming a barrier to inflammatory cells and markers, and limiting access of inflammatory factors, such as microbial or environmental contaminants, to the damaged corneal surface.

**Fuchs' dystrophy**

In some embodiments, formulations of SAP are used for treatment or prevention of one or more of the symptoms associated with Fuchs' Dystrophy.

Fuchs' Dystrophy (also known as Fuchs' corneal endothelial dystrophy or FCED) is a slowly progressing corneal dystrophy that usually affects both eyes. FCED results in corneal clouding, loss of corneal sensation and the formation of epithelial bullae. Multiple stages of Fuchs' endothelial dystrophy are recognized, typically evolving gradually over a period of about 25 years.

The first stage is the onset of cornea guttata, usually in the fourth decade of life. Subjective symptoms rarely occur until the fifth or sixth decade. During the asymptomatic phase, endothelial guttata and pigment dusting can be seen by slit lamp examination of the central corneal endothelium and by specular reflection. The guttata excrescences can become more numerous and confluent so that individual guttata are lost completely in the beaten-metal appearance of the endothelial surface. The central cornea is involved first, and, as the disease progresses, it spreads toward the periphery.

In the second phase of the disease, blurred vision, glare, and halos around lights develop because of incipient corneal edema in the stroma and epithelium. Epithelial edema can be seen as small droplets (bedewing) on retroillumination with the slit lamp. Epithelial microcysts coalesce to form bullae, which produce varying amounts of pain when they burst; hence, the name bullous keratopathy. Striae form in the Descemet membrane as the cornea thickens posteriorly due to stromal swelling. The arc of the Descemet membrane from limbus to limbus is shortened, causing wrinkles in the Descemet membrane called striae. The microcystic epithelial vesicles may break, causing foreign body sensations and severe pain with more extensive corneal epithelial disruption.

In the third stage, RCES, microbial ulceration, and persistent pain may occur. Corneal sensitivity usually is reduced.

**Eye Infections**

The formulations are suitable for use in the management of eye infections. Therefore, in some embodiments, SAP and compositions thereof are used to treat or prevent eye infections from bacteria, fungi, viruses, and protozoa. Eye infections can occur in different parts of one or both eyes and can be associated with conjunctivitis, stye, inflammation pain, etc.

In some embodiments, the formulations are for prophylactic purposes to prevent onset of an infection. For example, people recently exposed to a person with an eye infection, e.g., conjunctivitis, can use SAP for prophylactic purposes. In some embodiments, the formulations are used to reduce or alleviate one or more symptoms from an eye infection.

In some embodiments, SAP and compositions thereof are used to treat or prevent eye keratitis, *i.e.,* inflammations of the cornea, by applying topically or by injection the compositions containing one or more SAP into the corneal stroma. Conditions treated by the methods include, but are not limited to, amoebic keratitis, fungal keratitis, bacterial keratitis, viral keratitis, and onchorcercal keratitis.

In some embodiments, SAP and compositions thereof are used to treat or prevent amoebic keratitis. Amoebic keratitis can be caused by acanthamoeba and can be treated by administering to the corneal stroma of a subject in need of treatment thereof the compositions, optionally containing one or more anti-amoebic agents. Any anti-amoebic agent known in the art can be combined with the compositions. Representative, anti-amoebic agents include, but are not limited to, polyhexamethylene biguanide (PHMB), propamidine isethionate, miconazole nitrate, neomycin, chlorhexidine digluconate, polymyxin B, clotrimazole, and combinations thereof.

In some embodiments, SAP and compositions thereof are used to treat or prevent fungal keratitis. Fungal keratitis can be caused by, for example, aspergillus fumigates, fusarium, and/or yeasts, such as candida, and can be treated by administering to the corneal stroma of a subject in need of treatment thereof the compositions, optionally containing one or more antifungal agents. Any antifungal agent known in the art can be combined with the compositions. Representative, antifungal agents include natamycin, nystatin, amphotericin B, chlorhexidine, fluorinated pyrimidines, such as flucytosine, azoles, such as imidazoles and triazoles, including ketoconazole, miconazole, itraconazole, fluconazole, econazole, and clotrimazole, and combinations thereof.

In certain embodiments, SAP and compositions thereof are used to treat or prevent bacterial keratitis. Bacterial keratitis can be caused, for example, by streptococcus, pseudomonas, Enterobacteriaceae (including Klebsiella, Enterobacter, Serratia, and Proteus), and staphylococcus species. Such infection can be treated by administering to the corneal stroma of a subject in need of treatment thereof the compositions, optionally containing one or more anti-bacterial agents. Further, up to 20% of cases of fungal keratitis (particularly candidiasis) can be complicated by bacterial co-infection. Thus, in some embodiments, the methods include combination therapies involving administering to the corneal stroma of a subject in need of treatment thereof the compositions, optionally containing one or more antifungal agents and one or more anti-bacterial agents.

In certain embodiments, SAP and compositions thereof are used to treat or prevent viral keratitis. Viral keratitis can, for example, be caused by a herpes simplex virus and can be treated by administering to the corneal stroma of a subject in need of treatment thereof the compositions, optionally containing one or more anti-viral agents, such as, but not limited to, acyclovir. Any anti-viral agent known in the art can be combined with the compositions.

In some embodiments, SAP and compositions thereof are used to treat or prevent keratitis associated with infection by a nematode, such as onchoceral keratitis. Onchoceral keratitis, also referred to as "River Blindness," is caused by the nematode Onchocerca volvulus and can be treated by administering to the corneal stroma of a subject in need of treatment thereof the compositions, optionally containing one or more anti-parasitic agents, such as, but not limited to ivermectin. Any anti-parasitic agent known in the art can be combined with the compositions.

**C. Effective Amounts and Controls**

SAP can be administered to the eye therapeutically to achieve a therapeutic benefit, or prophylactically to achieve a prophylactic benefit, or to achieve both a therapeutic and prophylactic benefit. Therapeutic benefit means treating the underlying disorder including eradication or amelioration of one or more of the symptoms associated with the underlying disorder such that the patient reports an improvement in feeling or condition, notwithstanding that the patient can still be afflicted with the underlying disorder. For example, administration of a composition to a patient suffering from a condition provides therapeutic benefit when the patient reports a decrease in the severity or duration of the symptoms associated with the condition. Therapeutic benefit also includes halting or slowing the progression of the disease, regardless of whether improvement is realized by the patient.

In preferred embodiments, SAP are retained at the site of administration (e.g., at the surface of the cornea) for periods of time sufficient to yield a therapeutic effect. For example, when administered topically onto the cornea, compositions of SAP assemble at the surface of the cornea to form an SAP structure that resists clearance by tearing, eye movement and diffusion at the site of the administration. In preferred embodiments, the amount of time that the SAP remains in contact with the surface of the cornea is sufficient to prevent or reduce corneal inflammation and edema. In certain embodiments, the ability to resist clearance from the site of administration and prevent or reduce corneal inflammation is associated with parameters such as the concentration and homogeneity of SAP within the composition.

Typically, SAP are administered to the eye in an effective amount in order to achieve a clinically significant result. Effective dosages and concentrations are those that provide a therapeutic benefit and can be determined according to the desired therapeutic or prophylactic result. For example, the SAP can be effective to treat and prevent one or more symptoms of an ocular disease or disorder. Exemplary symptoms that can be treated, prevented reduced or otherwise moderated by SAP include, but are not limited to, pain, irritation, inflammation and swelling of the eye, redness or discoloration of the eye and/or surrounding tissues, dryness of the eye, discharge and/or watering of the eyes, impaired vision, blindness, and photosensitivity.

In one embodiment, the SAP are administered in an amount effective to reduce or prevent inflammation of the eye. In some embodiments, the SAP are administered in an amount effective to reduce or prevent inflammation of the cornea or surface of the eye. Reducing inflammation can include reducing or preventing tissue damage, inducing or enhancing healing, reducing or preventing scarring, or combinations of these.

In some embodiments, the SAP are in an amount effective to provide a fluid impermeable barrier at the site of administration. The barrier is effective to prevent the movement of bodily fluids and contaminants through the structure. When SAP provide a barrier at the surface of the eye, the presence of the barrier can be effective to treat diseases associated with recurrent infection or contamination of a damaged or missing corneal tissue. For example, in some embodiments, the presence of a self-assembled barrier provides a continuous surface that prevents the formation of adhesions between the damaged corneal epithelium and other tissues. In other embodiments, the presence of the barrier mitigates inflammatory processes associated with eye diseases, such as chronic eye diseases *(e.g.,* RCES).

In some embodiments, the SAP are in an amount effective to provide a fluid permeable structure at the site of administration. In some embodiments, the presence of a fluid permeable structure that acts as a membrane to enable passage of one or more fluids into and through the structure provides a suitable scaffold for the replacement, repair or augmentation of a diseased or damaged tissue on the surface of the eye or within the eye.

For guidance, one can consult texts such as Goodman and Gilman's The Pharmacological Basis of Therapeutics, 10th Ed., and Katzung, Basic and Clinical Pharmacology.

Inflammation and intraocular pressure (IOP) can be measured before and after administration of the compositions by methods known in the art. For example, a Reichert Tono-Pen contact tonometer can be used to assess the IOP, and changes in IOP. A subject, for example, a human patient, can be evaluated on several post-administration days, for example at 1, 7, 14 and 30 days post-administration, using the same evaluation procedure on each day. One or more measurements, for example about five, can be obtained for each subject at each time point. When measuring IOP according to methods known in the art, for example the method above, IOP is shown to be reduced by, for example, at least about 10%, or at least about 30%, or at least about 50% over what is observed when a control composition, for example a composition that does not include SAP, is administered to the patient. To measure inflammation, slit-lamp bio-microscopy can be performed to examine structures of the eye, for example, the cornea, for signs of inflammation.

The examination can involve observation of criteria such as the presence of cells, flare and fibrin. The subjects can be evaluated on several post-injection days, for example at 1, 7, 14 and 30 days post-injection using the same evaluation procedure on each day. After general and/or local anesthesia is achieved, each subject can be examined for gross abnormalities. The exams can be performed by the same trained ophthalmologist, and the ophthalmologist can be blinded to the assignment of the treatment and control subjects.

Quantification of inflammation of an eye compartment such as the cornea can be performed using a modified version of the Standard Uveitis Nomenclature clinical grading scheme. When measuring inflammation according to methods known in the art, for example the method above, inflammation is shown to be reduced by, for example, at least about 10%, or at least about 30%, or at least about 50% over what is observed when an equivalent control composition, e.g., lacking SAPs, is administered to the patient. When comparing an IOP or inflammation measurement obtained after administration of SAP versus an IOP or inflammation measurement obtained for a control composition, the measurements compared can, and frequently should, be taken at similar time points post administration. For example, an IOP measurement after administration of SAP taken 7 days post-administration is generally compared with an IOP measurement for a control composition taken 7 days post-administration.

All references cited herein are incorporated by reference in their entirety. The present description will be further understood by reference to the following non-limiting examples.

**Example 1: SAP Exhibit Therapeutic Effects in the Eye**

The anti-inflammatory effect of SAP was examined using the lipopolysaccharide ocular inflammation model, and measurement of microglial cell activation was assessed as an indicator of inflammation. Morphological changes in macrophage cells, such as microglial cells, can be used as a marker of the presence and extent of inflammation. Methods for identifying and scoring variations in microglial cell morphology in response to inflammatory stimuli are described in the art (see, for example, Jonas, et al., PLoS ONE 7(2): e30763 (2012)).

## Methods and Materials

### Animals

Adult white Sprague-Dawley rats weighing between 200 and 240 grams were housed in a 12 hour light / 12 hour night cycle with free access to food and water. The animals were randomly divided into study or control groups.

### Intravitreal Injections

Animals were anesthetized by an intraperitoneal injection of xylazine 2% (5 mg / kg body weight) and ketamine 10% (100 mg/ kg body weight). Topical anesthesia was additionally provided by repeated administration of proparacaine (0.5%) drops onto the cornea. A thorough disinfection of the ocular surface, the lid margins and the periocular skin was carried out with iodine 5% solution.

Using a glass pipette pulled to the size of a 30 gauge needle and attached to a syringe, 2 $\mu$l of sterile, pyrogen-free saline containing lipopolysaccharides from *Salmonella typhimurium* (catalog no. L-7261; Sigma-Aldrich, St. Louis, MO) was intravitreally injected.

In a preliminary experiment, the optimal lipopolysaccharide (LPS) concentration and survival time was assessed in a group of animals. Concentrations of 1 $\mu$g or 2 $\mu$g LPS / $\mu$L were administered, and the animals sacrificed after a post injection survival time of 8, 12, 24, 72 hours, 1 week, or 2 weeks, respectively. This study showed that the optimal concentration was 2 $\mu$g LPS / $\mu$L in 2 $\mu$l of carrier solution, and that a survival time of 66 to 68 hours was the optimal time for measurement and quantification of retinal inflammation. Retinal inflammation and retinal destruction with tissue lysis was also detected starting at about 24 hours post injection. The retinal lysis led to an accumulation of retinal ganglion cells in the pre-retinal vitreous.

For the primary experiment, the study included 40 Sprague-Dawley rats. All intravitreal injection volumes were of 2 $\mu$l, to reduce the effects of increased ocular pressure. All intravitreal injections of lipopolysaccharides (LPS) volumes were 4 $\mu$g at a concentration of 2 $\mu$g /$\mu$L.

The control groups (n=20) received either an intravitreal saline injection (n=5), lipopolysaccharide (LPS) alone (n=15), or no injection as a normal control group (n=5). Animals in the SAP study group (n=15) also received the self-assembling peptide (amino acid sequence RADARADARADARADA; "RADA-16") in concentrations of 0.5% of SAP/2 $\mu$L.

At one, three and seven days after the injections, the animals were sacrificed, and the globes were immunohistochemically stained and histo-morphometrically examined. The contralateral eye from the LPS group was also examined.

### Immuno-histochemical Staining

The eyes were embedded with Optimal Cutting Temperature compound (Tissue-Tek® O.C.T., Ted Pella, Inc. Redding, CA) after the lenses were removed. The globes were sectioned and placed on subbed slides. After being washed in 0.01M phosphate buffered saline for 10 minutes, the sections (15$\mu$m) were immersed in a blocking solution containing 0.3% triton, 2.5% bovine serum albumin (BSA; Proliant Co, Ankeny, IA, USA) and 2% goat serum for 30 minutes. The sections were incubated in a primary antibody diluted solution overnight at 4°C, containing rabbit anti-IBA1 (Ionized calcium Binding Adaptor molecule 1; 1:500; Wako Pure Chemical Industries, Ltd. Osaka, Japan) and mouse anti rat CD 68 (ED1) (1:1000; AbD Serotech, D-40470 Dusseldorf, Germany). The sections were then washed in 0.01M phosphate buffered saline (10 minutes, 3 times), and incubated with secondary antibody diluted solution at room temperature for 2 hours (Goat-anti-rabbit 568, Goat-anti-mouse Alexa 488, (Invitrogen, Carlsbad, California, 1:400)). The slides were then washed in 0.01M phosphate buffered saline (10 minutes, 3 times) and cover slipped with fluorescein mounting medium with DAPI (4',6-diamidin-2'-phenylindol- dihydrochloride; Dako Ltd, Glostrup, Denmark). All images were taken under a 20X objective with a Carl Zeiss Flour microscope (Carl Zeiss Inc. Oberkochen, Germany) fitted with a spot camera. All illumination levels were fixed during image acquisition.

To determine a potential interaction between the LPS and RADA-16, a SDS PAGE gel electrophoresis (sodium dodecylsulfate polyacrylamide gel electrophoresis) was performed in a 12.5% gel, running 4.5 h with urea in stacking and separating, 5% SDS and 5% urea in loading buffer.

*Assessment of Intraocular Inflammation*

The amount of intraocular inflammation was assessed by quantification of immunohistochemically labeled activated retinal microglial cells, which function as the immune mediators of the central nervous system.

The images of the retinas were quantified using the program Image J from the National Institute of Health. Continuous sections of the retina, choroid and pre-retinal vitreous were outlined, the number of activated was determined, and the density of activated pixels was calculated. These measurements were performed in a masked manner.

Six categories of inflammation have been described in the microglia system. Each retinal section was examined, and each microglia was categorized based on activation level (type 1-6) according to morphological characteristics. In addition, both the microglia marker ionized calcium-binding adapter molecule 1 (IBA-1) and the macrophage marker ED-1 immuno-histochemical reactivities were used as indicators of the shift from type 4 to 5 activation level. Three blind assessors were used to count and categorize the microglial activation level. A weighted calculation based on the morphological classification of activated microglia cells (the "JEB" score, as determined according to the methods of Jonas, et al., PLoS ONE 7(2): e30763 (2012)) was derived and applied to the activation category level to provide an inflammation score that could indicate anti- or pro-inflammatory effects, and the results were averaged across the three assessors (see Table 4). Normalization was done by dividing the number of microglia over the volume of the sections. A potential direct interaction between the lipopolysaccharides (LPS) and the SAP material was examined by gel electrophoresis.

## Results

The density of activated retinal microglial cells was significantly lower in animals in the SAP group compared to LPS controls. The numbers of activated microglia within the tissue sections from each group assessed are indicated in Table 4 below.

**Table 4:** Results across animals treated with LPS, LPS+AC5 (RADA), Saline, and untreated controls.

| TOTALS FOR COMPARISON | JEB # | TOTAL AREA WEIGHT | N | SD | SD |
|---|---|---|---|---|---|
| NORMAL | -0.513244048 | -3.190105616 | 5 | 1.227546 | 9.255464 |
| SALINE DAY 3 | 5.309575517 | 30.81892728 | 5 | 4.217035 | 25.37896 |
| CONTRALATERAL EYE LPS DAY 3 | 3.583019943 | 15.15861947 | 10 | 3.927901 | 23.80311 |
| 1 DAY LPS | 99.33963341 | 787.1193889 | 5 | 26.36615 | 236.5959 |
| 3 DAY LPS | 145.0817385 | 818.3299501 | 10 | 31.73665 | 116.6731 |
| 0.5% RADA +LPS DAY 1 | 11.63263348 | 74,99464859 | 5 | 13.08403 | 26.60439 |
| 0.5% RADA + LPS DAY 3 | 8.502149471 | 55.91265281 | 5 | 6.457764 | 45.92137 |
| 0.5% RADA + LPS DAY 7 | 9.158481692 | 52.65745506 | 5 | 8.855492 | 47.74718 |

The amount of inflammation in the experimental combination injections with both SAP and LPS showed no significant difference from normal or saline injected controls (0.5% SAP + LPS [Day 1, $75.0 \pm 26.6$; Day 3, $55.9 \pm 45.9$; Day 7, $52.7 \pm 47.7$], saline injection [Day 3, $30.8 \pm 25.4$], normal control [$-3.2 \pm 9.3$]). In the LPS-only group, there was a significant increase in inflammation (Day 1, $787.1 \pm 236.6$; Day 3, $818.3 \pm 116.7$). The level of inflammation was significantly ($P<0.001$) lower in the SAP group than in the LPS-only control group. The contralateral eye of the LPS-only group showed evidence of a numerical, but not significant increase in the inflammation score (Day 3, $15.16 \pm 23$), compared to the normal non-injected eye. Electrophoresis revealed no interaction between the lipopolysaccharides and SAP.

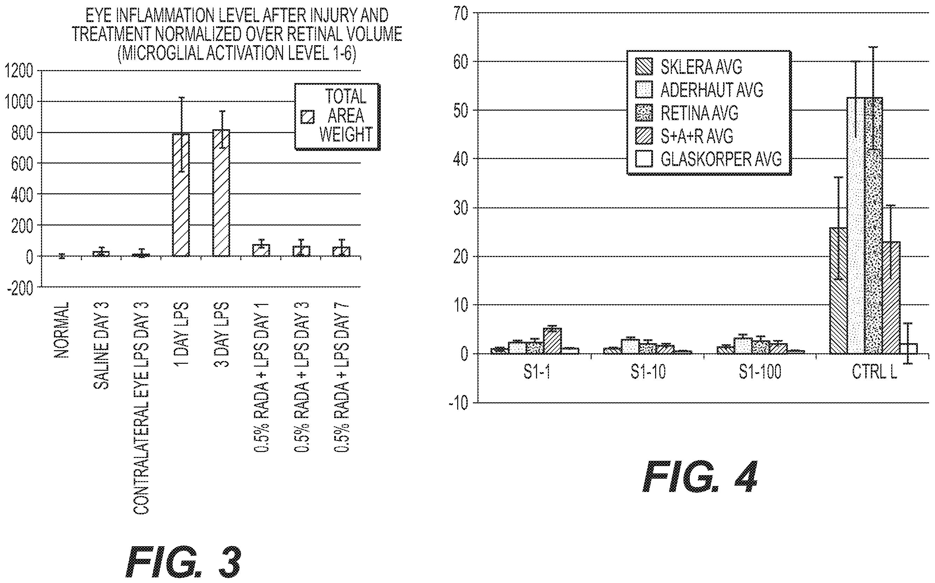

Differences in the density (mean $\pm$ standard deviation) of activated retinal microglial cells in the LPS control and RADA study group are depicted as histograms in Figures 2 and 3.

In this experimental intraocular inflammation model of injected LPS, the additional intraocular application of SAP along with LPS was associated with a marked reduction in signs of retinal inflammation. The density of activated retinal microglial cells was significantly lower in the eyes of the study animals with the combined application of lipopolysaccharides and SAP than in the eyes of the LPS-only control group.

**Example 2: (RADA)$_2$ and EARA based SAPs Exhibit Therapeutic Effects in the Eye**

**Methods and Materials**

*Animals*

Adult white Sprague-Dawley rats with a weight of between 200 and 240 grams were housed in a 12 hours light / 12 hours night cycle with free access to food and water. The animals were randomly divided into a study or control groups.

*Treatment and analysis*

Intravitreal injections, immunohistochemical staining and assessment of intraocular inflammation were generally performed as described in Example 1.

1 μl sterile lipopolysaccharide (LPS; from *S. typhimurium;* catalog no. L-7261; Sigma-Aldrich, St. Louis, MO) either alone or in combination with (RADA)$_2$ (SEQ ID NO: 88) solutions at 0.1%, 1.0%, and 10.0% were administered via direct intravitreal injections. The animals were sacrificed 24 hours after treatment. The experiments were repeated at least 5 times.

In some experiments, different SAP, including S2 (H$_2$N(EARA)COOH; SEQ ID NO: 92), S3 (H$_2$N(RARA)CONH$_2$; SEQ ID NO: 413), and S5 (H$_2$N(EARA)$_2$CONH$_2$; SEQ ID NO: 414), were evaluated as described above for (RADA)$_2$ (SEQ ID NO: 88).

Finally, a conjugate of an EARA based SAP and a tissue-specific motif (MSCRAMM; SEQ ID NO: 141) was also evaluated using this lipopolysaccharide ocular inflammation model. Experiments were performed as described above for (RADA)$_2$ (SEQ ID NO: 88). Briefly, 1 μl sterile lipopolysaccharide (LPS; from *S. typhimurium;* catalog no. L-7261; Sigma-Aldrich, St. Louis, MO) either alone or in combination with MSCRAMM(EARA)$_4$ (SEQ ID NO: 432) solutions at 1.0%, and 3.0% were administered via direct intravitreal injections. The animals were sacrificed 24 hours after treatment. The experiments were repeated three times for each SAP conjugate concentration (that is, n=3 for 1%; n=3 for 3%).

**Results**

As shown in Figure 4, at all concentrations of (RADA)$_2$ (SEQ ID NO: 88), the combinatorial application of (RADA)$_2$ along with LPS was associated with a marked reduction in signs of retinal inflammation compared to control. The density of activated retinal microglial cells was significantly lower in the eyes of the experimental group (combination injection with both (RADA)$_2$ and LPS) compared to the controls (injections of LPS alone).

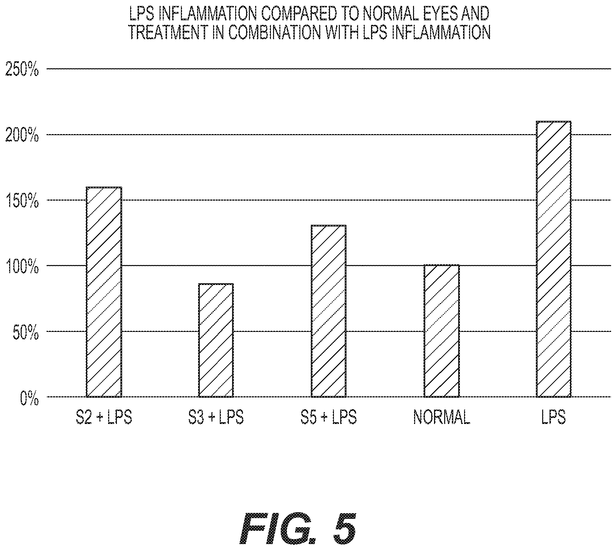

Furthermore, the combinatorial application of EARA (SEQ ID NO: 92), H$_2$N(RARA)CONH$_2$ (SEQ ID NO: 413), or H$_2$N(EARA)$_2$CONH$_2$ (SEQ ID NO: 414) along with LPS was associated with a marked reduction in signs of retinal inflammation compared to control (Figure 5). In particular, S3 (H$_2$N(RARA)CONH$_2$; SEQ ID NO: 413) reduced LPS induced inflammation to normal levels (Figure 5).

Application of the MSCRAMM(EARA)$_4$ (SEQ ID NO: 432) conjugate was also associated with a reduction in signs of retinal inflammation compared to control (LPS only). There appeared to be no inflammation and no evidence of ED1 reactivity in MSCRAMM(EARA)$_4$ treated animals. Any activation of microglia for MSCRAMM(EARA)$_4$ treated animals was a 4 or below (JEB score), while animals treated with LPS alone exhibited microglial activation of 5 and above.

The data demonstrate that compositions of SAP, and conjugates thereof (e.g., (RADA)$_2$, SEQ ID NO: 88; EARA, SEQ ID NO: 92; H$_2$N(RARA)CONH$_2$, SEQ ID NO: 413; H$_2$N(EARA)$_2$CONH$_2$, SEQ ID NO: 414; MSCRAMM(EARA)$_4$, SEQ ID NO: 432) can be used to treat eye diseases (*e.g.*, reducing associated symptoms, such as retinal inflammation).

**Example 3: Delivery of SAP To The Retina via Application of Eye Drops**

The fate of SAP upon application to the eye was investigated. Specifically, the ability of SAP to reach the retina via application of eye drop solutions of the SAP to the cornea was evaluated using an SAP-Cy5 conjugate.

**Methods**

*Animals*

Adult white Sprague-Dawley rats weighing between 200 and 240 grams were housed in a 12 hour light / 12 hour night cycle with free access to food and water. The animals were randomly divided into experimental or control groups.

*Conjugation of Cy5.5 to RADA*

Conjugates of Cy5.5 and RADA (SEQ ID NO: 57), Cy5.5 and RADARADA ((RADA)$_2$; SEQ ID NO: 88), and Cy5.5 and $H_2N(RADA)_4CONH_2$ (SEQ ID NO: 433) were prepared according to a modified protocol of Chen X., et al., Cancer Res., 64(21): 8009-8014 (2004).

In one experiment, RADA-4-Cy5.5 conjugate was prepared as follows: 0.5 mg of $H_2N(RADA)_4CONH_2$ was dissolved in 50 $\mu$L of DMSO to a final concentration of 0.5% in DMSO. The solution was mixed with 20 $\mu$L of diethyl isopropyl amine (DIPEA) to adjust the pH to 12. A solution of Cy5.5 dyes (1.0 mg in 20 $\mu$L of DMSO) was added at room temperature for conjugation and the mixture was periodically vortexed over 2 hours.

**Table 5. Components for Cy5.5(RADA)$_4$ conjugation**

| Sample | Formula | Weight (mg) | Final Volume (mL) | Final Conc ($\mu$g/$\mu$L) | Final Conc (mM) | Mole ratio |
|---|---|---|---|---|---|---|
| SEQ ID NO: 433 | $H_2N$ $(RADA)_4CONH_2$ | 0.5 | 0.1 | 5 | 2.994 | 1 |
| Cy5.5 | | 1.0 | 0.1 | 10 | 8.425 | 2.8 |

In some experiments, the RADA-4 solution was prepared with water as the solvent, for example: to a solution of RADA-4 (1.8mg of $H_2N(RADA)_4CONH_2$ (SEQ ID NO: 433) in 500 $\mu$L of Milli-Q water), 1 $\mu$L of Diisopropyl ethylene amine (DIPEA) was added to obtain pH 8 from 4.

To prepare a working Cy5.5 stock, 165 $\mu$L of Milli-Q water was added to 165 $\mu$L Cy5.5 to make a 6.59 $\times10^{-7}$ M solution. The final conjugate was prepared by mixing equal volumes (165 $\mu$L) of the buffered (pH 8) (RADA)$_4$ solution and Cy5.5 solution at room temperature for 2 hours.

**Table 6. Components for Cy5.5(RADA)$_4$ conjugation**

| Sample | Formula | Weight (mg) | Milli-Q Vol. ($\mu$L) | Final Conc. ($\mu$g/$\mu$L) | Mole (x10$^{-7}$) | Mole ratio |
|---|---|---|---|---|---|---|
| SEQ ID NO: 433 | $H_2N(RADA)_4$ $CONH_2$ | 1.5 | 500 | 3 | 8.98 | 1.3 |
| Cy5.5 | | 1.0 | 500 | 2 | 13.17 | 1.0 |

The Cy5.5 SAP conjugates listed in Table 7 were also prepared and evaluated as described above for the RADA-based conjugates.

**Table 7. Cy5.5 SAP conjugates**

| | |
|---|---|
| Cy5.5(RADA)$_4$CONH$_2$ | SEQ ID NO: 434 |
| Cy5.5(EARA)CONH$_2$ | SEQ ID NO: 435 |
| Cy5.5(EARA)COOH | SEQ ID NO: 436 |
| Cy5.5(RARA)CONH$_2$ | SEQ ID NO: 437 |
| Cy5.5(RARA)COOH | SEQ ID NO: 438 |
| Cy5.5(EARA)$_2$CONH$_2$ | SEQ ID NO: 439 |
| Cy5.5(EARA)$_2$COOH | SEQ ID NO: 440 |
| Cy5.5(RARA)$_2$CONH$_2$ | SEQ ID NO: 441 |
| Cy5.5(RARA)$_2$COOH | SEQ ID NO: 140 |
| Cy5.5(RADA)COOH | SEQ ID NO: 442 |
| Cy5.5(RADA)CONH$_2$ | SEQ ID NO: 443 |
| Cy5.5(RADA)$_2$COOH | SEQ ID NO: 444 |
| Cy5.5(RADA)$_2$CONH$_2$ | SEQ ID NO: 445 |

*Treatment*

Eye drops were applied to both eyes of rats. The experimental group of rats were administered 2 $\mu$L of

Cy5.5-(RADA)$_4$ conjugate via drops, while control rats were administered Cy5.5 only via drops. The experiments were repeated using Cy5.5 conjugated to RADA (Cy5.5(RADA)COOH; SEQ ID NO: 442) and Cy5.5 conjugated to (RADA)$_2$ (Cy5.5(RADA)$_2$COOH; SEQ ID NO: 444).

The rats were sacrificed 24 hours or 3 days post treatment. The eye balls of the rats were harvested and were then fixed in 4% paraformaldehyde (PFA) overnight. Excessive PFA was washed with 0.1M phosphate buffer (0.1MPB) 3 times (30 minutes each). The eyeballs were then dehydrated with 30% sucrose solution (30g sucrose in 100 mL of 0.1 MPB) overnight, while replenishing the sucrose solution once for at least 6 hours. As necessary, the shape of the eyeballs was reformed with O.C.T. (Optimal Cutting Temperature compound; TISSUE-TEK® O.C.T.; CA) by injecting through the optic nerve. Each eye was embedded entirely in a mount with O.C.T. in liquid nitrogen to form frozen blocks. Blocks containing the eye samples were then sectioned at 20 microns and were placed on charged slides and allowed to dry. The slides were then rinsed to remove excess OCT and cover-slipped. The fate of the Cy5.5-RADA conjugate was investigated by tracing the color of the Cy5.5 in the eye. Visualization was performed with light microscopy and photographs were taken with a SPOT camera affixed to the Zeiss microscope.

## Results

At both 24 hour and 3 day time points, pink coloration was observed throughout the outer retina of mice treated with the Cy5.5-(RADA)$_4$ conjugate. However, no pink coloration was observed in any part of the retina or optic nerve head of control rats (i.e., receiving Cy5.5 only) at either time point. This data demonstrates that upon application as eye drops, the SAP can penetrate the cornea and migrate to the retina.

SAP can be used for treatment of inflammation from diseases in the retina or other parts of the eye, such as but not limited to, diabetic retinopathy, retinitis pigmentosa, uveitis, inflammatory eye diseases, autoimmune eye diseases, Reiter's syndrome, psoriasis, rheumatoid arthritis, Sjogren's Syndrome, optic neuritis, and sarcoidosis. In such applications, the compositions could be applied as drops. It is contemplated that the compositions containing SAP could be used for treatment and/or repair of damaged tissue, e.g., retina.

**Claims**

1. A dosage unit of a formulation for use in treating ocular and/or intraocular inflammation in a subject in need thereof, wherein the formulation is suitable for ophthalmological administration and comprises self-assembling peptides or self-assembling peptidomimetics, and the formulation is administered onto or into the eye of the subject in an amount effective for the treatment of ocular and/or intraocular inflammation;

   wherein the self-assembling peptides or self-assembling peptidomimetics have a sequence of amino acid residues conforming to one or more of Formulas I-XII:

$$((Xaa^{neu}\text{-}Xaa^+)_x(Xaa^{neu}\text{-}Xaa^-)_y)_n \qquad (I);$$

$$((Xaa^{neu}\text{-}Xaa^-)_x(Xaa^{neu}\text{-}Xaa^+)_y)_n \qquad (II);$$

$$((Xaa^+\text{-}Xaa^{neu})_x(Xaa^-\text{-}Xaa^{neu})_y)_n \qquad (III);$$

$$((Xaa^-\text{-}Xaa^{neu})_x(Xaa^+\text{-}Xaa^{neu})_y)_n \qquad (IV);$$

$$Xaa^{neu} ((Xaa^{neu}\text{-}Xaa^+)_x(Xaa^{neu}\text{-}Xaa^-)_y)_n \qquad (V)$$

$$Xaa^{neu} ((Xaa^{neu}\text{-}Xaa^-)_x(Xaa^{neu}\text{-}Xaa^+)_y)_n \qquad (VI)$$

$$((Xaa^+\text{-}Xaa^{neu})_x(Xaa^-\text{-}Xaa^{neu})_y)_n Xaa^{neu} \qquad (VII)$$

$$((Xaa^-\text{-}Xaa^{neu})_x(Xaa^+\text{-}Xaa^{neu})_y)_n\ Xaa^{neu} \qquad (VIII)$$

$$((Xaa^{neu}\text{-}Xaa^+)_x(Xaa^{neu}\text{-}Xaa^-)_y)_n\ Xaa^{neu} \qquad (IX)$$

$$((Xaa^{neu}\text{-}Xaa^-)_x(Xaa^{neu}\text{-}Xaa^+)_y)_n\ Xaa^{neu} \qquad (X)$$

$$Xaa^{neu}\ ((Xaa^+\text{-}Xaa^{neu})_x(Xaa^-\text{-}Xaa^{neu})_y)_n \qquad (XI)$$

$$Xaa^{neu}\ ((Xaa^-\text{-}Xaa^{neu})_x(Xaa^+\text{-}Xaa^{neu})_y)_n \qquad (XII)$$

wherein $Xaa^{neu}$ represents an amino acid residue having a neutral charge; $Xaa^+$ represents an amino acid residue having a positive charge; $Xaa^-$ represents an amino acid residue having a negative charge; x and y are integers having a value of 1, 2, 3, or 4, independently; and n is an integer having a value of 1-5;
wherein between about 70% and 100% of the self-assembling peptides or self-assembling peptidomimetics are of the same size and have the same amino acid sequence, and
wherein the dosage unit is effective to prevent or mitigate ocular and/or intraocular inflammation when administered onto and/or into the eye of the subject.

2. The dosage unit for use according to claim 1, further comprising a pH-adjusting agent.

3. The dosage unit for use according to claim 1 or 2, wherein:

(a) the concentration of self-assembling peptides or self-assembling peptidomimetics in the formulation is between 0.1% w/v and 6% w/v, inclusive, preferably between 0.1% w/v and 4% w/v, inclusive;
(b) the concentration of ions in the formulation is between 5 nM and less than 5 mM;
(c) the self-assembling peptides or self-assembling peptidomimetics have from 8 to 16 residues, 12 to 16 residues, or 16 to 20 residues, preferably 16 residues; and/or
(d) the self-assembling peptides or self-assembling peptidomimetics include two or more repeating units of a sequence selected from the group consisting of RADA (SEQ ID NO:57) and EAKA (SEQ ID NO:77).

4. The dosage unit for use according to any one of claims 1 to 3, comprising a pharmaceutically acceptable excipient for administration into or onto the eye.

5. The dosage unit for use according to any one of claims 1 to 4, wherein the dosage unit is a device comprising a backing material or support structure.

6. The dosage unit for use according to any one of claims 1 to 5, further comprising one more therapeutic agents, prophylactic agents, diagnostic agents, or combinations thereof.

7. The dosage unit for use according to any one of claims 1 to 6, wherein the dosage unit is:

(a) in a form selected from the group consisting of powders, liquids, emulsions, gels, nanoparticles, and microparticles; or
(b) a device selected from the group consisting of ocular implants, intraocular lens, contact lens, coatings on a medical device, eye patches, and woven or non-woven fiber wound coverings.

8. The dosage unit for use according to any one of claims 1 to 7, wherein the formulation is dried, dehydrated or vacuum packaged.

9. The dosage unit for use according to any one of claims 1 to 8, wherein the formulation is formulated for administration into the eyes in the form of eye drops.

10. The dosage unit for use according to any one of claims 1-9 in a kit with means for administration.

11. A contact lens comprising self-assembling peptides or self-assembling peptidomimetics for use in treating ocular and/or intraocular inflammation associated with one or more diseases, injuries, or disorders of the eye,

wherein the self-assembling peptides or self-assembling peptidomimetics have a sequence of amino acid residues conforming to one or more of Formulas I-XII:

$$((Xaa^{neu}\text{-}Xaa^+)_x(Xaa^{neu}\text{-}Xaa^-)_y)_n \qquad (I);$$

$$((Xaa^{neu}\text{-}Xaa^-)_x(Xaa^{neu}\text{-}Xaa^+)_y)_n \qquad (II);$$

$$((Xaa^+\text{-}Xaa^{neu})_x(Xaa^-\text{-}Xaa^{neu})_y)_n \qquad (III);$$

$$((Xaa^-\text{-}Xaa^{neu})_x(Xaa^+\text{-}Xaa^{neu})_y)_n \qquad (IV);$$

$$Xaa^{neu} ((Xaa^{neu}\text{-}Xaa^+)_x(Xaa^{neu}\text{-}Xaa^-)_y)_n \qquad (V)$$

$$Xaa^{neu} ((Xaa^{neu}\text{-}Xaa^-)_x(Xaa^{neu}\text{-}Xaa^+)_y)_n \qquad (VI)$$

$$((Xaa^+\text{-}Xaa^{neu})_x(Xaa^-\text{-}Xaa^{neu})_y)_n Xaa^{neu} \qquad (VII)$$

$$((Xaa^-\text{-}Xaa^{neu})_x(Xaa^+\text{-}Xaa^{neu})_y)_n Xaa^{neu} \qquad (VIII)$$

$$((Xaa^{neu}\text{-}Xaa^+)_x(Xaa^{neu}\text{-}Xaa^-)_y)_n Xaa^{neu} \qquad (IX)$$

$$((Xaa^{neu}\text{-}Xaa^-)_x(Xaa^{neu}\text{-}Xaa^+)_y)_n Xaa^{neu} \qquad (X)$$

$$Xaa^{neu} ((Xaa^+\text{-}Xaa^{neu})_x(Xaa^-\text{-}Xaa^{neu})_y)_n \qquad (XI)$$

$$Xaa^{neu} ((Xaa^-\text{-}Xaa^{neu})_x(Xaa^+\text{-}Xaa^{neu})_y)_n \qquad (XII)$$

wherein each $Xaa^{neu}$ represents an amino acid residue having a neutral charge; Xaa+ represents an amino acid residue having a positive charge; Xaa-represents an amino acid residue having a negative charge; x and y are integers having a value of 1, 2, 3, or 4, independently; and n is an integer having a value of 1-5; optionally further comprising one or more therapeutic agents, prophylactic agents, diagnostic agents, or combinations thereof.

12. The dosage unit for use according to any one of claims 1-10, or the contact lens for use according to claim 11, wherein the subject:

(a) has or is at risk of developing a disease or disorder selected from the group consisting of diabetes, glaucoma, corneal ectasia, dry eye, recurring corneal erosion, Fuchs' dystrophy, keratitis, conjunctivitis, ocular herpes and Sjogren's syndrome;
(b) is undergoing or has undergone a vitrectomy; or
(c) has or is at risk of retinal detachment;

optionally wherein the self-assembling peptides or self-assembling peptidomimetics are self-assembled at the time of or after administration.

**13.** The dosage unit for use according to claim 9, wherein the self-assembling peptides or self-assembling peptidomimetics are assembled immediately prior to administration, optionally wherein the peptides are assembled by contacting the self-assembling peptides or self-assembling peptidomimetics with a solution of cations.

**14.** The dosage unit for use according to any one of claims 1-10, or the contact lens for use according to claim 11, wherein the use comprises:

   (a) administering the dosage unit topically to the eye or a compartment or structure associated therewith, optionally wherein the dosage is applied as eye drops, an ointment, gel, or emulsion;
   (b) implanting or inserting the contact lens into the eye or a compartment or structure associated therewith; or
   (c) administering the dosage unit by intra-corneal, intravitreal, intracameral, periocular, punctual, subconjunctival, sub-tenon, subchoroidal, suprachoroidal or subretinal routes.

**15.** The dosage unit for use according to any one of claims 1-10, or the contact lens for use according to claim 11, wherein the inflammation is associated with a disease, disorder or injury.

**Patentansprüche**

**1.** Dosierungseinheit einer Formulierung zur Verwendung bei der Behandlung von Augen- und/oder intraokularen Entzündungen bei einem Patienten, der dies benötigt, wobei die Formulierung zur ophthalmologischen Verabreichung geeignet ist und selbstassemblierende Peptide oder selbstassemblierende Peptidmimetika umfasst und die Formulierung auf oder in das Auge des Patienten in einer Menge verabreicht wird, die zur Behandlung von Augen- und/oder intraokularen Entzündungen wirksam ist;

   wobei die selbstassemblierenden Peptide oder selbstassemblierenden Peptidmimetika eine Sequenz von Aminosäureresten aufweisen, die einer oder mehreren der Formeln I bis XII entsprechen:

$$((Xaa^{neu}\text{-}Xaa^+)_x\ (Xaa^{neu}\text{-}Xaa^-)_y)_n \qquad (I);$$

$$((Xaa^{neu}\text{-}Xaa^-)_x\ (Xaa^{neu}\text{-}Xaa^+)_y)_n \qquad (II);$$

$$((Xaa^+\text{-}Xaa^{neu})_x\ (Xaa^-\text{-}Xaa^{neu})_y)_n \qquad (III);$$

$$((Xaa^-\text{-}Xaa^{neu})_x\ (Xaa^+\text{-}Xaa^{neu})_y)_n \qquad (IV);$$

$$Xaa^{neu}\ ((Xaa^{neu}\text{-}Xaa^+)_x\ (Xaa^{neu}\text{-}Xaa^-)_y)_n \qquad (V)$$

$$Xaa^{neu}\ ((Xaa^{neu}\text{-}Xaa^-)_x\ (Xaa^{neu}\text{-}Xaa^+)_y)_n \qquad (VI)$$

$$((Xaa^+\text{-}Xaa^{neu})_x\ (Xaa^-\text{-}Xaa^{neu})_y)_n\ Xaa^{neu} \qquad (VII)$$

$$((Xaa^-\text{-}Xaa^{neu})_x\ (Xaa^+\text{-}Xaa^{neu})_y)_n\ Xaa^{neu} \qquad (VIII)$$

$$((Xaa^{neu}\text{-}Xaa^+)_x\ (Xaa^{neu}\text{-}Xaa^-)_y)_n\ Xaa^{neu} \qquad (IX)$$

$$( (Xaa^{neu}\text{-}Xaa^-)_x (Xaa^{neu}\text{-}Xaa^+)_y)_n \ Xaa^{neu} \qquad (X)$$

$$Xaa^{neu} ( (Xaa^+\text{-}Xaa^{neu})_x (Xaa^-\text{-}Xaa^{neu})_y)_n \qquad (XI)$$

$$Xaa^{neu} ( (Xaa^-\text{-}Xaa^{neu})_x (Xaa^+\text{-}Xaa^{neu})_y)_n \qquad (XII)$$

wobei $Xaa^{neu}$ einen Aminosäurerest mit einer neutralen Ladung darstellt; $Xaa^+$ einen Aminosäurerest mit einer positiven Ladung darstellt; $Xaa^-$ einen Aminosäurerest mit einer negativen Ladung darstellt; x und y unabhängig voneinander ganze Zahlen mit einem Wert von 1, 2, 3 oder 4 sind; und n eine ganze Zahl mit einem Wert von 1-5 ist;

wobei zwischen etwa 70 % und 100 % der selbstassemblierenden Peptide oder selbstassemblierenden Peptidmimetika die gleiche Größe haben und die gleiche Aminosäuresequenz haben, und

wobei die Dosierungseinheit wirksam ist, um Augen- und/oder intraokulare Entzündungen zu verhindern oder zu lindern, wenn sie auf und/oder in das Auge des Patienten verabreicht wird.

2. Dosierungseinheit zur Verwendung nach Anspruch 1, ferner umfassend ein Mittel zur pH-Wert-Einstellung.

3. Dosierungseinheit zur Verwendung nach Anspruch 1 oder 2, wobei:

(a) die Konzentration selbstassemblierender Peptide oder selbstassemblierender Peptidmimetika in der Formulierung zwischen 0,1 % (Gew./Vol.) und 6 % (Gew./Vol.) einschließlich, vorzugsweise zwischen 0,1 % (Gew./Vol.) und 4 % (Gew./Vol.) einschließlich liegt;
(b) die Ionenkonzentration in der Formulierung zwischen 5 nM und weniger als 5 mM liegt;
(c) die selbstassemblierenden Peptide oder selbstassemblierenden Peptidmimetika 8 bis 16 Reste, 12 bis 16 Reste oder 16 bis 20 Reste, vorzugsweise 16 Reste aufweisen; und/oder
(d) die selbstassemblierenden Peptide oder selbstassemblierenden Peptidmimetika zwei oder mehr Wiederholungseinheiten einer Sequenz umfassen, ausgewählt aus der Gruppe bestehend aus RADA (SEQ ID NO:57) und EAKA (SEQ ID NO:77).

4. Dosierungseinheit zur Verwendung nach einem der Ansprüche 1 bis 3, umfassend einen pharmazeutisch verträglichen Hilfsstoff zur Verabreichung in oder auf das Auge.

5. Dosierungseinheit zur Verwendung nach einem der Ansprüche 1 bis 4, wobei die Dosierungseinheit eine Vorrichtung ist, die ein Trägermaterial oder eine Stützstruktur umfasst.

6. Dosierungseinheit zur Verwendung nach einem der Ansprüche 1 bis 5, ferner umfassend ein oder mehrere therapeutische Mittel, prophylaktische Mittel, diagnostische Mittel oder Kombinationen davon.

7. Dosierungseinheit zur Verwendung nach einem der Ansprüche 1 bis 6, wobei die Dosierungseinheit:

(a) in einer Form ist, ausgewählt aus der Gruppe bestehend aus Pulvern, Flüssigkeiten, Emulsionen, Gelen, Nanopartikeln und Mikropartikeln; oder
(b) ein Gerät ist, ausgewählt aus der Gruppe bestehend aus Augenimplantaten, Intraokularlinsen, Kontaktlinsen, Beschichtungen auf einem medizinischen Gerät, Augenklappen und Wundabdeckungen aus gewebten oder nicht gewebten Fasern.

8. Dosierungseinheit zur Verwendung nach einem der Ansprüche 1 bis 7, wobei die Formulierung getrocknet, dehydriert oder vakuumverpackt ist.

9. Dosierungseinheit zur Verwendung nach einem der Ansprüche 1 bis 8, wobei die Formulierung zur Verabreichung in die Augen in Form von Augentropfen formuliert ist.

10. Dosierungseinheit zur Verwendung nach einem der Ansprüche 1 bis 9 in einem Kit mit Mitteln zur Verabreichung.

11. Kontaktlinse, umfassend selbstassemblierende Peptide oder selbstassemblierende Peptidmimetika zur Verwendung bei der Behandlung von Augen- und/oder intraokularen Entzündungen, die mit einer oder mehreren Krankheiten, Verletzungen oder Störungen des Auges verbunden sind,

wobei die selbstassemblierenden Peptide oder selbstassemblierenden Peptidmimetika eine Sequenz von Aminosäureresten aufweisen, die einer oder mehreren der Formeln I bis XII entsprechen:

$$((Xaa^{neu}\text{-}Xaa^+)_x\ (Xaa^{neu}\text{-}Xaa^-)_y)_n \qquad (I);$$

$$((Xaa^{neu}\text{-}Xaa^-)_x\ (Xaa^{neu}\text{-}Xaa^+)_y)_n \qquad (II);$$

$$((Xaa^+\text{-}Xaa^{neu})_x\ (Xaa^-\text{-}Xaa^{neu})_y)_n \qquad (III);$$

$$((Xaa^-\text{-}Xaa^{neu})_x\ (Xaa^+\text{-}Xaa^{neu})_y)_n \qquad (IV);$$

$$Xaa^{neu}\ ((Xaa^{neu}\text{-}Xaa^+)_x\ (Xaa^{neu}\text{-}Xaa^-)_y)_n \qquad (V)$$

$$Xaa^{neu}\ ((Xaa^{neu}\text{-}Xaa^-)_x\ (Xaa^{neu}\text{-}Xaa^+)_y)_n \qquad (VI)$$

$$((Xaa^+\text{-}Xaa^{neu})_x\ (Xaa^-\text{-}Xaa^{neu})_y)_n\ Xaa^{neu} \qquad (VII)$$

$$((Xaa^-\text{-}Xaa^{neu})_x\ (Xaa^+\text{-}Xaa^{neu})_y)_n\ Xaa^{neu} \qquad (VIII)$$

$$((Xaa^{neu}\text{-}Xaa^+)_x\ (Xaa^{neu}\text{-}Xaa^-)_y)_n\ Xaa^{neu} \qquad (IX)$$

$$((Xaa^{neu}\text{-}Xaa^-)_x\ (Xaa^{neu}\text{-}Xaa^+)_y)_n\ Xaa^{neu} \qquad (X)$$

$$Xaa^{neu}\ ((Xaa^+\text{-}Xaa^{neu})_x\ (Xaa^-\text{-}Xaa^{neu})_y)_n \qquad (XI)$$

$$Xaa^{neu}\ ((Xaa^-\text{-}Xaa^{neu})_x\ (Xaa^+\text{-}Xaa^{neu})_y)_n \qquad (XII)$$

wobei jedes $Xaa^{neu}$ einen Aminosäurerest mit einer neutralen Ladung darstellt; $Xaa^+$ einen Aminosäurerest mit einer positiven Ladung darstellt; $Xaa^-$ einen Aminosäurerest mit einer negativen Ladung darstellt; x und y unabhängig voneinander ganze Zahlen mit einem Wert von 1, 2, 3 oder 4 sind; und n eine ganze Zahl mit einem Wert von 1-5 ist;
optional ferner umfassend ein oder mehrere therapeutische Mittel, prophylaktische Mittel, diagnostische Mittel oder Kombinationen davon.

**12.** Dosierungseinheit zur Verwendung nach einem der Ansprüche 1 bis 10 oder die Kontaktlinse zur Verwendung nach Anspruch 11, wobei der Patient:

(a) eine Krankheit oder Störung aus der Gruppe bestehend aus Diabetes, Glaukom, Hornhautektasie, trockenem Auge, wiederkehrender Hornhauterosion, Fuchs-Dystrophie, Keratitis, Konjunktivitis, Augenherpes und Sjögren-Syndrom hat oder das Risiko besteht, diese zu entwickeln;
(b) sich einer Vitrektomie unterzieht oder unterzogen wurde; oder
(c) eine Netzhautablösung hat oder dem Risiko einer Netzhautablösung ausgesetzt ist;

optional wobei die selbstassemblierenden Peptide oder selbstassemblierenden Peptidmimetika zum Zeitpunkt oder nach der Verabreichung selbstassembliert sind.

**13.** Dosierungseinheit zur Verwendung nach Anspruch 9, wobei die selbstassemblierenden Peptide oder selbstassemblierenden Peptidomimetika unmittelbar vor der Verabreichung zusammengesetzt werden, optional wobei die Peptide durch Kontaktieren der selbstassemblierenden Peptide oder selbstassemblierenden Peptidmimetika mit einer Kationenlösung zusammengesetzt werden.

**14.** Dosierungseinheit zur Verwendung nach einem der Ansprüche 1 bis 10 oder die Kontaktlinse zur Verwendung nach Anspruch 11, wobei die Verwendung Folgendes umfasst:

(a) topisches Verabreichen der Dosierungseinheit an das Auge oder ein damit verbundenes Kompartiment oder eine damit verbundene Struktur, wobei die Dosierung optional in Form von Augentropfen, einer Salbe, einem Gel oder einer Emulsion angewendet wird;
(b) Implantieren oder Einsetzen der Kontaktlinse in das Auge oder eine damit verbundene Kammer oder Struktur; oder
(c) Verabreichen der Dosierungseinheit auf intrakornealen, intravitrealen, intrakameralen, periokularen, punktuellen, subkonjunktivalen, subtenonalen, subchoroidalen, suprachoroidalen oder subretinalen Wegen.

**15.** Dosierungseinheit zur Verwendung nach einem der Ansprüche 1 bis 10 oder Kontaktlinse zur Verwendung nach Anspruch 11, wobei die Entzündung mit einer Krankheit, Störung oder Verletzung verbunden ist.

**Revendications**

**1.** Unité posologique d'une formulation destinée à être utilisée dans le traitement d'une inflammation oculaire et/ou intraoculaire chez un sujet en ayant besoin, dans laquelle la formulation est appropriée pour une administration ophtalmologique et comprend des peptides à auto-assemblage ou des peptidomimétiques à auto-assemblage, et la formulation est administrée sur ou dans l'œil du sujet en une quantité efficace pour le traitement d'une inflammation oculaire et/ou intraoculaire ;
dans laquelle les peptides à auto-assemblage ou les peptidomimétiques à auto-assemblage ont une séquence de résidus d'acide aminé se conformant à une ou plusieurs parmi les formules I à XII :

$$((Xaa^{neu}-Xaa^+)_x(Xaa^{neu}-Xaa^-)_y)_n \qquad (I) \; ;$$

$$((Xaa^{neu}-Xaa^-)_x(Xaa^{neu}-Xaa^+)_y)_n \qquad (II) \; ;$$

$$((Xaa^+-Xaa^{neu})_x(Xaa^--Xaa^{neu})_y)_n \qquad (III) \; ;$$

$$((Xaa^--Xaa^{neu})_x(Xaa^+-Xaa^{neu})_y)_n \qquad (IV) \; ;$$

$$Xaa^{neu} \; ((Xaa^{neu}-Xaa^+)_x(Xaa^{neu}-Xaa^-)_y)_n \qquad (V)$$

$$Xaa^{neu} \; ((Xaa^{neu}-Xaa^-)_x(Xaa^{neu}-Xaa^+)_y)_n \qquad (VI)$$

$$((Xaa^+-Xaa^{neu})_x(Xaa^--Xaa^{neu})_y)_n \; Xaa^{neu} \qquad (VII)$$

$$((Xaa^--Xaa^{neu})_x(Xaa^+-Xaa^{neu})_y)_n \; Xaa^{neu} \qquad (VIII)$$

$$((Xaa^{neu}-Xaa^+)_x (Xaa^{neu}-Xaa^-)_y)_n Xaa^{neu} \qquad (IX)$$

$$((Xaa^{neu}-Xaa^-)_x (Xaa^{neu}-Xaa^+)_y)_n Xaa^{neu} \qquad (X)$$

$$Xaa^{neu} ((Xaa^+-Xaa^{neu})_x (Xaa^--Xaa^{neu})_y)_n \qquad (XI)$$

$$Xaa^{neu} ((Xaa^--Xaa^{neu})_x (Xaa^+-Xaa^{neu})_y)_n \qquad (XII)$$

dans laquelle Xaa$^{neu}$ représente un résidu d'acide aminé ayant une charge neutre ; Xaa$^+$ représente un résidu d'acide aminé ayant une charge positive ; Xaa$^-$ représente un résidu d'acide aminé ayant une charge négative ; x et y sont des nombres entiers ayant une valeur de 1, 2, 3 ou 4, indépendamment ; et n est un nombre entier ayant une valeur de 1 à 5 ;

dans laquelle entre environ 70% et 100% des peptides à auto-assemblage ou des peptidomimétiques à auto-assemblage ont la même taille et ont la même séquence d'acides aminés, et

dans lequel l'unité posologique est efficace pour prévenir ou atténuer une inflammation oculaire et/ou intraoculaire lorsqu'elle est administrée sur et/ou dans l'œil du sujet.

**2.** Unité posologique destinée à être utilisée selon la revendication 1, comprenant en outre un agent d'ajustement du pH.

**3.** Unité posologique destinée à être utilisée selon la revendication 1 ou 2, dans laquelle :

(a) la concentration en peptides à auto-assemblage ou peptidomimétiques à auto-assemblage de la formulation est comprise entre 0,1% en p/v et 6% en p/v, bornes comprises, de préférence entre 0,1% en p/v et 4% en p/v, bornes comprises ;

(b) la concentration en ions de la formulation est comprise entre 5nM et moins de 5mM ;

(c) les peptides à auto-assemblage ou peptidomimétiques à auto-assemblage ont de 8 à 16 résidus, de 12 à 16 résidus, ou de 16 à 20 résidus, de préférence 16 résidus ; et/ou

(d) les peptides à auto-assemblage ou les peptidomimétiques à auto-assemblage comprennent deux ou plusieurs motifs récurrents d'une séquence choisie dans le groupe constitué par RADA (SEQ ID NO : 57) et EAKA (SEQ ID NO : 77).

**4.** Unité posologique destinée à être utilisée selon l'une quelconque des revendications 1 à 3, comprenant un excipient pharmaceutiquement acceptable pour une administration dans ou sur l'œil.

**5.** Unité posologique destinée à être utilisée selon l'une quelconque des revendications 1 à 4, dans laquelle l'unité posologique est un dispositif comprenant un matériau de support ou une structure de support.

**6.** Unité posologique destinée à être utilisée selon l'une quelconque des revendications 1 à 5, comprenant en outre un ou plusieurs agents thérapeutiques, agents prophylactiques, agents de diagnostic ou combinaisons de ceux-ci.

**7.** Unité posologique destinée à être utilisée selon l'une quelconque des revendications 1 à 6, dans laquelle l'unité posologique est :

(a) sous une forme choisie dans le groupe constitué par des poudres, des liquides, des émulsions, des gels, des nanoparticules et des microparticules ; ou

(b) un dispositif choisi dans le groupe constitué par des implants oculaires, des lentilles intraoculaires, des lentilles de contact, des revêtements sur un dispositif médical, des pansements oculaires et des pansements en fibres tissées ou non tissées.

**8.** Unité posologique destinée à être utilisée selon l'une quelconque des revendications 1 à 7, dans laquelle la formulation est séchée, déshydratée ou emballée sous vide.

**9.** Unité posologique destinée à être utilisée selon l'une quelconque des revendications 1 à 8, dans laquelle la formulation est formulée pour une administration dans les yeux sous la forme de gouttes ophtalmiques.

**10.** Unité posologique destinée à être utilisée selon l'une quelconque des revendications 1 à 9 dans un kit avec des moyens d'administration.

**11.** Lentille de contact comprenant des peptides à auto-assemblage ou des peptidomiétiques à auto-assemblage destinée à être utilisée dans le traitement d'une inflammation oculaire et/ou intraoculaire associée à une ou plusieurs maladies, lésions ou troubles de l'œil,

dans laquelle les peptides à auto-assemblage ou les peptidomimétiques à auto-assemblage ont une séquence de résidus d'acide aminé se conformant à une ou plusieurs parmi les formules I à XII :

$$((Xaa^{neu}-Xaa^+)_x(Xaa^{neu}-Xaa^-)_y)_n \quad (I) \, ;$$

$$((Xaa^{neu}-Xaa^-)_x(Xaa^{neu}-Xaa^+)_y)_n \quad (II) \, ;$$

$$((Xaa^+-Xaa^{neu})_x(Xaa^--Xaa^{neu})_y)_n \quad (III) \, ;$$

$$((Xaa^--Xaa^{neu})_x(Xaa^+-Xaa^{neu})_y)_n \quad (IV) \, ;$$

$$Xaa^{neu} \, ((Xaa^{neu}-Xaa^+)_x(Xaa^{neu}-Xaa^-)_y)_n \quad (V)$$

$$Xaa^{neu} \, ((Xaa^{neu}-Xaa^-)_x(Xaa^{neu}-Xaa^+)_y)_n \quad (VI)$$

$$((Xaa^+-Xaa^{neu})_x(Xaa^--Xaa^{neu})_y)_n \, Xaa^{neu} \quad (VII)$$

$$((Xaa^--Xaa^{neu})_x(Xaa^+-Xaa^{neu})_y)_n \, Xaa^{neu} \quad (VIII)$$

$$((Xaa^{neu}-Xaa^+)_x(Xaa^{neu}-Xaa^-)_y)_n \, Xaa^{neu} \quad (IX)$$

$$((Xaa^{neu}-Xaa^-)_x(Xaa^{neu}-Xaa^+)_y)_n \, Xaa^{neu} \quad (X)$$

$$Xaa^{neu} \, ((Xaa^+-Xaa^{neu})_x(Xaa^--Xaa^{neu})_y)_n \quad (XI)$$

$$Xaa^{neu} \, ((Xaa^--Xaa^{neu})_x(Xaa^+-Xaa^{neu})_y)_n \quad (XII)$$

dans laquelle chaque $Xaa^{neu}$ représente un résidu d'acide aminé ayant une charge neutre ; $Xaa^+$ représente un résidu d'acide aminé ayant une charge positive ; $Xaa^-$ représente un résidu d'acide aminé ayant une charge négative ; x et y sont des nombres entiers ayant une valeur de 1, 2, 3 ou 4, indépendamment ; et n est un nombre entier ayant une valeur de 1 à 5 ;
comprenant éventuellement en outre un ou plusieurs agents thérapeutiques, agents prophylactiques, agents de diagnostic ou combinaisons de ceux-ci.

**12.** Unité posologique destinée à être utilisée selon l'une quelconque des revendications 1 à 10, ou lentille de contact destinée à être utilisée selon la revendication 11, dans laquelle le sujet :

(a) a ou risque de développer une maladie ou un trouble choisi dans le groupe constitué par le diabète, le glaucome, l'ectasie cornéenne, la sécheresse oculaire, l'érosion cornéenne récurrente, la dystrophie de Fuchs, la kératite, la conjonctivite, l'herpès oculaire et le syndrome de Sjögren ;
(b) subit ou a subi une vitrectomie ; ou
(c) a ou est à risque de décollement de la rétine ;

éventuellement dans lequel les peptides à auto-assemblage ou les peptidomimétiques à auto-assemblage sont auto-assemblés au moment de ou après l'administration.

13. Unité posologique destinée à être utilisée selon la revendication 9, dans laquelle les peptides à auto-assemblage ou les peptidomimétiques à auto-assemblage sont assemblés immédiatement avant l'administration, éventuellement dans laquelle les peptides sont assemblés en mettant en contact les peptides à auto-assemblage ou les peptidomimétiques à auto-assemblage avec une solution de cations.

14. Unité posologique destinée à être utilisée selon l'une quelconque des revendications 1 à 10, ou lentille de contact destinée à être utilisée selon la revendication 11, dans laquelle l'utilisation comprend :

(a) l'administration topique de l'unité posologique à l'œil ou à un compartiment ou une structure associée à celui-ci, éventuellement dans laquelle la posologie est appliquée sous forme de gouttes oculaires, de pommade, de gel ou d'émulsion ;
(b) l'implantion ou l'insertion de la lentille de contact dans l'œil ou un compartiment ou une structure associée à celui-ci ; ou
(c) l'administration de l'unité posologique par voies intra-cornéenne, intravitréenne, intracamérulaire, périoculaire, ponctuelle, sous-conjonctivale, sous-tenonneuse, sous-choroïdienne, suprachoroïdienne ou sous-rétinienne.

15. Unité posologique destinée à être utilisée selon l'une quelconque des revendications 1 à 10, ou lentille de contact destinée à être utilisée selon la revendication 11, dans laquelle l'inflammation est associée à une maladie, un trouble ou une lésion.

CANAL OF SCHLEMM
PUPIL
CORNEA
LENS
IRIS
CONJUNCTIVA
ANTERIOR CHAMBER ANGLE
CILIARY BODY
EPISCLERAL VEINS
PARS PLICATA
POSTERIOR CHAMBER
PARS PLANA
ZONULE
LENS CAPSULE
MEDIAL RECTUS MUSCLE
LATERAL RECTUS MUSCLE
ORA SERRATA
CHOROID
RETINA
RETINA
CHOROID
SCLERA
SCLERA
VITREOUS
VORTEX VEIN
RETINAL PIGMENT EPITHELIUM
RETINAL ARTERIOLES AND VEINS
MACULA
LAMINA CRIBROSA
LONG POSTERIOR CILIARY ARTERY AND LONG CILIARY NERVE
OPTIC DISK
ARACHNOID
OPTIC NERVE
DURA PIA
CENTRAL RETINAL ARTERY AND VEIN

**FIG. 1A**

EXTERNAL LIPID LAYER
INTERMEDIATE AQUEOUS LAYER
CORNEAL STROMA
INTERNAL MUCOUS LAYER
EPITHELIAL CELLS

**FIG. 1B**

**FIG. 1C**

EYE INFLAMMATION LEVEL AFTER
INJURY AND TREATMENT
(MICROGLIAL ACTIVATION LEVEL 1-6)

☑ JEB #

NORMAL
SALINE DAY 3
CONTRALATERAL EYE LPS DAY 3
1 DAY LPS
3 DAY LPS
0.5% RADA + LPS DAY 1
0.5% RADA + LPS DAY 3
0.5% RADA + LPS DAY 7

**FIG. 2**

*FIG. 4*

*FIG. 3*

LPS INFLAMMATION COMPARED TO NORMAL EYES AND
TREATMENT IN COMBINATION WITH LPS INFLAMMATION

*FIG. 5*

## REFERENCES CITED IN THE DESCRIPTION

### Patent documents cited in the description

- US 62647184
- WO 2006116524 A
- WO 2015027203 A
- WO 2008113030 A
- US 2010272803 A
- US 5670483 A, Zhang
- US 5955343 A
- US 6548630 B
- US 6800481 B
- US 7098028 B
- US 9327010 B
- US 9364513 B, Zhang
- US 9162005 B
- US 9415084 B
- US 9339476 B, Ellis-Behnke
- WO 2007142757 A

### Non-patent literature cited in the description

- **YU et al.** *Cornea,* 2011, vol. 30 (4), 379-87
- **WADUTHANTRIL et al.** *PLoS ONE,* 2012, vol. 7 (6), e37711
- **LEMP et al.** *Report of the International Dry Eye Workshop (DEWS) Ocul Surf,* 2007, vol. 5, 65-204
- Working Together to Manage Diabetes: A guide for pharmacy, podiatry, Optometry, and dentistry. *What Eye Care Professionals Would Like Team Members to Know About Eye Health and Diabetes,* 2016, 59-67
- **YAGIHASHI et al.** *Journal of Diabetes Investigation,* 2011, vol. 2 (1), 18-32
- **LEMP.** *Am J Manag Care:,* 2008, vol. 14 (3), 88-101
- **HOLMES et al.** *Proc. Natl. Acad. Sci. USA,* 2000, vol. 97, 6728-6733
- **ZHANG et al.** *Proc. Natl. Acad. Sci. USA,* 1993, vol. 90, 3334-3338
- **ZHANG et al.** *Biomaterials,* 1995, vol. 16, 1385-1393
- **CAPLAN et al.** *Biomaterials,* 2002, vol. 23, 219-227
- **LEON et al.** *J. Biomater. Sci. Polym. Ed.,* 1998, vol. 9, 297-312
- **CAPLAN et al.** *Biomacromolecules,* 2000, vol. 1, 627-631
- **VAGNER J. et al.** *Curr. Opin. Chem. Biol.,* 2008, vol. 12 (3), 292-296
- **MOORE et al.** *Chem. Rev.,* 2001, vol. 101 (12), 3893-4012
- Remington's Pharmaceutical Sciences. Lippincott Williams & Wilkins, 2000, 704
- **GOODMAN ; GILMAN'S.** The Pharmacological Basis of Therapeutics
- **KATZUNG.** *Basic and Clinical Pharmacology*
- **JONAS et al.** *PLoS ONE,* 2012, vol. 7 (2), e30763
- **CHEN X. et al.** *Cancer Res.,* 2004, vol. 64 (21), 8009-8014